# EUROPEAN PATENT APPLICATION

(11) **EP 2 386 639 A2**
(43) Date of publication of application: **16.11.2011**
(21) Application number: 11166965.1
(22) Date of filing: 21.11.2006
(51) Int. Cl.: C12N 15/31, C12N 15/63, C12N 5/10, C12N 1/21, C07K 14/195, C07K 14/33, A61K 38/00, A61K 38/16, A61P 35/00

(54) **Modified pore-forming protein toxins and use thereof**

(30) Priority: 21.11.2005 US 738916 P
(62) Divisional of application: 06804758.8
(71) Applicant: Protox Therapeutics Incorporated, Vancouver, BC V6C 3E8 (CA)
(72) Inventor: Buckley, James, Thomas, Victoria, British Columbia V8S 4K3 (CA)
(74) Representative: Gowshall, Jonathan Vallance

(57) **Abstract**

The present invention provides modified pore-forming protein toxins (MPPTs), capable of being used to kill cancer cells. The MPPTs according to the present invention comprise a modification of the naturally occurring activation sequence comprising one or more general cleavage sites, each of which is cleavable by general activating agent, or plurality of specific cleavage sites, each of which is cleavable by a specific activating agent. Optional further modifications that allow specific targeting of these molecules are also described. These MPPts may be used to treat cancer.

SEQUENCE LISTING

<110> Protox Therapeutics Incorporated
      BUCKLEY, James Thomas

<120> Modified Pore-Forming Protein Toxins and
  Use Thereof

<130> 1436-105PCT

<140> N/A
<141> 2006-11-21

<150> 60/738,916
<151> 2005-11-21

<160> 61

<170> FastSEQ for Windows Version 4.0

<210> 1
<211> 1410
<212> DNA
<213> Aeromonas hydrophila

<220>
<221> CDS
<222> (1)...(1410)

<400> 1
gca gag ccc gtc tat cca gac cag ctt cgc ttg ttt tca ttg ggc caa     48
Ala Glu Pro Val Tyr Pro Asp Gln Leu Arg Leu Phe Ser Leu Gly Gln
 1               5                  10                  15

ggg gtc tgt ggc gac aag tat cgc ccc gtc aat cga gaa gaa gcc caa     96
Gly Val Cys Gly Asp Lys Tyr Arg Pro Val Asn Arg Glu Glu Ala Gln
            20                  25                  30

agc gtt aaa agc aat att gtc ggc atg atg ggg caa tgg caa ata agc     144
Ser Val Lys Ser Asn Ile Val Gly Met Met Gly Gln Trp Gln Ile Ser
        35                  40                  45

ggg ctg gcc aac ggc tgg gtc att atg ggg ccg ggt tat aac ggt gaa     192
Gly Leu Ala Asn Gly Trp Val Ile Met Gly Pro Gly Tyr Asn Gly Glu
    50                  55                  60

ata aaa cca ggg aca gcg tcc aat acc tgg tgt tat ccg acc aat cct     240
Ile Lys Pro Gly Thr Ala Ser Asn Thr Trp Cys Tyr Pro Thr Asn Pro
65                  70                  75                  80

gtt acc ggt gaa ata ccg aca ctg tct gcc ctg gat att cca gat ggt     288
Val Thr Gly Glu Ile Pro Thr Leu Ser Ala Leu Asp Ile Pro Asp Gly
                85                  90                  95

gac gaa gtc gat gtg cag tgg cga ctg gta cat gac agt gcg aat ttc     336
Asp Glu Val Asp Val Gln Trp Arg Leu Val His Asp Ser Ala Asn Phe
            100                 105                 110

atc aaa cca acc agc tat ctg gcc cat tac ctc ggt tat gcc tgg gtg     384
Ile Lys Pro Thr Ser Tyr Leu Ala His Tyr Leu Gly Tyr Ala Trp Val
        115                 120                 125

ggc ggc aat cac agc caa tat gtc ggc gaa gac atg gat gtg acc cgt     432
Gly Gly Asn His Ser Gln Tyr Val Gly Glu Asp Met Asp Val Thr Arg
    130                 135                 140

gat ggc gac ggc tgg gtg atc cgt ggc aac aat gac ggc ggc tgt gac 480
Asp Gly Asp Gly Trp Val Ile Arg Gly Asn Asn Asp Gly Gly Cys Asp
145                 150                 155                 160

ggc tat cgc tgt ggt gac aag acg gcc atc aag gtc agc aac ttc gcc 528
Gly Tyr Arg Cys Gly Asp Lys Thr Ala Ile Lys Val Ser Asn Phe Ala
                165                 170                 175

tat aac ctg gat ccc gac agc ttc aag cat ggc gat gtc acc cag tcc 576
Tyr Asn Leu Asp Pro Asp Ser Phe Lys His Gly Asp Val Thr Gln Ser
            180                 185                 190

gac cgc cag ctg gtc aag act gtg gtg ggc tgg gcg gtc aac gac agc 624
Asp Arg Gln Leu Val Lys Thr Val Val Gly Trp Ala Val Asn Asp Ser
        195                 200                 205

gac acc ccc caa tcc ggc tat gac gtc acc ctg cgc tac gac aca gcc 672
Asp Thr Pro Gln Ser Gly Tyr Asp Val Thr Leu Arg Tyr Asp Thr Ala
    210                 215                 220

acc aac tgg tcc aag acc aac acc tat ggc ctg agc gag aag gtg acc 720
Thr Asn Trp Ser Lys Thr Asn Thr Tyr Gly Leu Ser Glu Lys Val Thr
225                 230                 235                 240

acc aag aac aag ttc aag tgg cca ctg gtg ggg gaa acc caa ctc tcc 768
Thr Lys Asn Lys Phe Lys Trp Pro Leu Val Gly Glu Thr Gln Leu Ser
                245                 250                 255

atc gag att gct gcc aat cag tcc tgg gcg tcc cag aac ggg ggc tcg 816
Ile Glu Ile Ala Ala Asn Gln Ser Trp Ala Ser Gln Asn Gly Gly Ser
            260                 265                 270

acc acc acc tcc ctg tct cag tcc gtg cga ccg act gtg ccg gcc cgc 864
Thr Thr Thr Ser Leu Ser Gln Ser Val Arg Pro Thr Val Pro Ala Arg
        275                 280                 285

tcc aag atc ccg gtg aag ata gag ctc tac aag gcc gac atc tcc tat 912
Ser Lys Ile Pro Val Lys Ile Glu Leu Tyr Lys Ala Asp Ile Ser Tyr
    290                 295                 300

ccc tat gag ttc aag gcc gat gtc agc tat gac ctg acc ctg agc ggc 960
Pro Tyr Glu Phe Lys Ala Asp Val Ser Tyr Asp Leu Thr Leu Ser Gly
305                 310                 315                 320

ttc ctg cgc tgg ggc ggc aac gcc tgg tat acc cac ccg gac aac cgt 1008
Phe Leu Arg Trp Gly Gly Asn Ala Trp Tyr Thr His Pro Asp Asn Arg
                325                 330                 335

ccg aac tgg aac cac acc ttc gtc ata ggt ccg tac aag gac aag gcg 1056
Pro Asn Trp Asn His Thr Phe Val Ile Gly Pro Tyr Lys Asp Lys Ala
            340                 345                 350

agc agc att cgg tac cag tgg gac aag cgt tac atc ccg ggt gaa gtg 1104
Ser Ser Ile Arg Tyr Gln Trp Asp Lys Arg Tyr Ile Pro Gly Glu Val
        355                 360                 365

aag tgg tgg gac tgg aac tgg acc ata cag cag aac ggt ctg tct acc 1152
Lys Trp Trp Asp Trp Asn Trp Thr Ile Gln Gln Asn Gly Leu Ser Thr
    370                 375                 380

atg cag aac aac ctg gcc aga gtg ctg cgc ccg gtg cgg gcg ggg atc 1200
Met Gln Asn Asn Leu Ala Arg Val Leu Arg Pro Val Arg Ala Gly Ile
385                 390                 395                 400

acc ggt gat ttc agt gcc gag agc cag ttt gcc ggc aac ata gag atc 1248
Thr Gly Asp Phe Ser Ala Glu Ser Gln Phe Ala Gly Asn Ile Glu Ile
                405                 410                 415

ggt gct ccc gtg ccg ctc gcg gct gac agc aag gtg cgt cgt gct cgc 1296
Gly Ala Pro Val Pro Leu Ala Ala Asp Ser Lys Val Arg Arg Ala Arg
            420                 425                 430

agt gtg gac ggc gct ggt caa ggc ctg agg ctg gag atc ccg ctc gat 1344
Ser Val Asp Gly Ala Gly Gln Gly Leu Arg Leu Glu Ile Pro Leu Asp
        435                 440                 445

gcg caa gag ctc tcc ggg ctt ggc ttc aac aac gtc agc ctc agc gtg 1392
Ala Gln Glu Leu Ser Gly Leu Gly Phe Asn Asn Val Ser Leu Ser Val
    450                 455                 460

acc cct gct gcc aat caa 1410
Thr Pro Ala Ala Asn Gln
465                 470

<210> 2
<211> 470
<212> PRT
<213> Aeromonas hydrophila

<400> 2
Ala Glu Pro Val Tyr Pro Asp Gln Leu Arg Leu Phe Ser Leu Gly Gln
1               5                   10                  15
Gly Val Cys Gly Asp Lys Tyr Arg Pro Val Asn Arg Glu Glu Ala Gln
            20                  25                  30
Ser Val Lys Ser Asn Ile Val Gly Met Met Gly Gln Trp Gln Ile Ser
        35                  40                  45
Gly Leu Ala Asn Gly Trp Val Ile Met Gly Pro Gly Tyr Asn Gly Glu
    50                  55                  60
Ile Lys Pro Gly Thr Ala Ser Asn Thr Trp Cys Tyr Pro Thr Asn Pro
65                  70                  75                  80
Val Thr Gly Glu Ile Pro Thr Leu Ser Ala Leu Asp Ile Pro Asp Gly
                85                  90                  95
Asp Glu Val Asp Val Gln Trp Arg Leu Val His Asp Ser Ala Asn Phe
            100                 105                 110
Ile Lys Pro Thr Ser Tyr Leu Ala His Tyr Leu Gly Tyr Ala Trp Val
        115                 120                 125
Gly Gly Asn His Ser Gln Tyr Val Gly Glu Asp Met Asp Val Thr Arg
    130                 135                 140
Asp Gly Asp Gly Trp Val Ile Arg Gly Asn Asn Asp Gly Gly Cys Asp
145                 150                 155                 160
Gly Tyr Arg Cys Gly Asp Lys Thr Ala Ile Lys Val Ser Asn Phe Ala
                165                 170                 175
Tyr Asn Leu Asp Pro Asp Ser Phe Lys His Gly Asp Val Thr Gln Ser
            180                 185                 190
Asp Arg Gln Leu Val Lys Thr Val Val Gly Trp Ala Val Asn Asp Ser
        195                 200                 205
Asp Thr Pro Gln Ser Gly Tyr Asp Val Thr Leu Arg Tyr Asp Thr Ala
    210                 215                 220
Thr Asn Trp Ser Lys Thr Asn Thr Tyr Gly Leu Ser Glu Lys Val Thr
225                 230                 235                 240
Thr Lys Asn Lys Phe Lys Trp Pro Leu Val Gly Glu Thr Gln Leu Ser
                245                 250                 255
Ile Glu Ile Ala Ala Asn Gln Ser Trp Ala Ser Gln Asn Gly Gly Ser
            260                 265                 270
Thr Thr Thr Ser Leu Ser Gln Ser Val Arg Pro Thr Val Pro Ala Arg
        275                 280                 285
Ser Lys Ile Pro Val Lys Ile Glu Leu Tyr Lys Ala Asp Ile Ser Tyr
    290                 295                 300
Pro Tyr Glu Phe Lys Ala Asp Val Ser Tyr Asp Leu Thr Leu Ser Gly
305                 310                 315                 320
Phe Leu Arg Trp Gly Gly Asn Ala Trp Tyr Thr His Pro Asp Asn Arg
                325                 330                 335
Pro Asn Trp Asn His Thr Phe Val Ile Gly Pro Tyr Lys Asp Lys Ala

                    340                           345                           350
Ser Ser Ile Arg Tyr Gln Trp Asp Lys Arg Tyr Ile Pro Gly Glu Val
            355                           360                           365
Lys Trp Trp Asp Trp Asn Trp Thr Ile Gln Gln Asn Gly Leu Ser Thr
      370                           375                           380
Met Gln Asn Asn Leu Ala Arg Val Leu Arg Pro Val Arg Ala Gly Ile
385                           390                           395                           400
Thr Gly Asp Phe Ser Ala Glu Ser Gln Phe Ala Gly Asn Ile Glu Ile
                        405                           410                           415
Gly Ala Pro Val Pro Leu Ala Ala Asp Ser Lys Val Arg Arg Ala Arg
                  420                           425                           430
Ser Val Asp Gly Ala Gly Gln Gly Leu Arg Leu Glu Ile Pro Leu Asp
            435                           440                           445
Ala Gln Glu Leu Ser Gly Leu Gly Phe Asn Asn Val Ser Leu Ser Val
      450                           455                           460
Thr Pro Ala Ala Asn Gln
465                           470

<210> 3
<211> 1482
<212> DNA
<213> Aeromonas hydrophila

<400> 3
atgcaaaaaa taaaactaac tggcttgtca ttaatcatat ccggcctgct gatggcacag 60
gcgcaagcgg cagagcccgt ctatccagac cagcttcgct tgttttcatt gggccaaggg 120
gtctgtggcg acaagtatcg ccccgtcaat cgagaagaag cccaaagcgt taaaagcaat 180
attgtcggca tgatggggca atggcaaata agcgggctgg ccaacggctg ggtcattatg 240
gggccgggtt ataacggtga aataaaacca gggacagcgt ccaatacctg gtgttatccg 300
accaatcctg ttaccggtga aataccgaca ctgtctgccc tggatattcc agatggtgac 360
gaagtcgatg tgcagtggcg actggtacat gacagtgcga atttcatcaa accaaccagc 420
tatctggccc attacctcgg ttatgcctgg gtgggcggca atcacagcca atatgtcggc 480
gaagacatgg atgtgacccg tgatggcgac ggctgggtga tccgtggcaa caatgacggc 540
ggctgtgacg gctatcgctg tggtgacaag acggccatca aggtcagcaa cttcgcctat 600
aacctggatc ccgacagctt caagcatggc gatgtcaccc agtccgaccg ccagctggtc 660
aagactgtgg tgggctgggc ggtcaacgac agcgacaccc cccaatccgg ctatgacgtc 720
accctgcgct acgacacagc caccaactgg tccaagacca acacctatgg cctgagcgag 780
aaggtgacca ccaagaacaa gttcaagtgg ccactggtgg gggaaaccga actctccatc 840
gagattgctg ccaatcagtc ctgggcgtcc cagaacgggg gctcgaccac cacctccctg 900
tctcagtccg tgcgaccgac tgtgccggcc cgctccaaga tcccggtgaa gatagagctc 960
tacaaggccg acatctccta tccctatgag ttcaaggccg atgtcagcta tgacctgacc 1020
ctgagcggct tcctgcgctg gggcggcaac gcctggtata cccacccgga caaccgtccg 1080
aactggaacc acaccttcgt cataggtccg tacaaggaca aggcgagcag cattcggtac 1140
cagtgggaca agcgttacat cccgggtgaa gtgaagtggt gggactggaa ctggaccata 1200
cagcagaacg gtctgtctac catgcagaac aacctggcca gagtgctgcg cccggtgcgg 1260
gcggggatca ccggtgattt cagtgccgag agccagtttg ccggcaacat agagatcggt 1320
gctcccgtgc cgctcgcggc tgacagcaag gtgcgtcgtg ctcgcagtgt ggacggcgct 1380
ggtcaaggcc tgaggctgga gatcccgctc gatgcgcaag agctctccgg gcttggcttc 1440
aacaacgtca gcctcagcgt gacccctgct gccaatcaat aa                    1482

<210> 4
<211> 493
<212> PRT
<213> Aeromonas hydrophila

<400> 4
Met Gln Lys Ile Lys Leu Thr Gly Leu Ser Leu Ile Ile Ser Gly Leu
1                     5                           10                          15
Leu Met Ala Gln Ala Gln Ala Ala Glu Pro Val Tyr Pro Asp Gln Leu
                  20                          25                          30
Arg Leu Phe Ser Leu Gly Gln Gly Val Cys Gly Asp Lys Tyr Arg Pro
            35                          40                          45
Val Asn Arg Glu Glu Ala Gln Ser Val Lys Ser Asn Ile Val Gly Met
      50                          55                          60
Met Gly Gln Trp Gln Ile Ser Gly Leu Ala Asn Gly Trp Val Ile Met
65                          70                          75                          80

Gly Pro Gly Tyr Asn Gly Glu Ile Lys Pro Gly Thr Ala Ser Asn Thr
                85                  90                  95
Trp Cys Tyr Pro Thr Asn Pro Val Thr Gly Glu Ile Pro Thr Leu Ser
            100                 105                 110
Ala Leu Asp Ile Pro Asp Gly Asp Glu Val Asp Val Gln Trp Arg Leu
        115                 120                 125
Val His Asp Ser Ala Asn Phe Ile Lys Pro Thr Ser Tyr Leu Ala His
    130                 135                 140
Tyr Leu Gly Tyr Ala Trp Val Gly Gly Asn His Ser Gln Tyr Val Gly
145                 150                 155                 160
Glu Asp Met Asp Val Thr Arg Asp Gly Asp Gly Trp Val Ile Arg Gly
                165                 170                 175
Asn Asn Asp Gly Gly Cys Asp Gly Tyr Arg Cys Gly Asp Lys Thr Ala
            180                 185                 190
Ile Lys Val Ser Asn Phe Ala Tyr Asn Leu Asp Pro Asp Ser Phe Lys
        195                 200                 205
His Gly Asp Val Thr Gln Ser Asp Arg Gln Leu Val Lys Thr Val Val
    210                 215                 220
Gly Trp Ala Val Asn Asp Ser Asp Thr Pro Gln Ser Gly Tyr Asp Val
225                 230                 235                 240
Thr Leu Arg Tyr Asp Thr Ala Thr Asn Trp Ser Lys Thr Asn Thr Tyr
                245                 250                 255
Gly Leu Ser Glu Lys Val Thr Thr Lys Asn Lys Phe Lys Trp Pro Leu
            260                 265                 270
Val Gly Glu Thr Glu Leu Ser Ile Glu Ile Ala Ala Asn Gln Ser Trp
        275                 280                 285
Ala Ser Gln Asn Gly Gly Ser Thr Thr Thr Ser Leu Ser Gln Ser Val
    290                 295                 300
Arg Pro Thr Val Pro Ala Arg Ser Lys Ile Pro Val Lys Ile Glu Leu
305                 310                 315                 320
Tyr Lys Ala Asp Ile Ser Tyr Pro Tyr Glu Phe Lys Ala Asp Val Ser
                325                 330                 335
Tyr Asp Leu Thr Leu Ser Gly Phe Leu Arg Trp Gly Gly Asn Ala Trp
            340                 345                 350
Tyr Thr His Pro Asp Asn Arg Pro Asn Trp Asn His Thr Phe Val Ile
        355                 360                 365
Gly Pro Tyr Lys Asp Lys Ala Ser Ser Ile Arg Tyr Gln Trp Asp Lys
    370                 375                 380
Arg Tyr Ile Pro Gly Glu Val Lys Trp Trp Asp Trp Asn Trp Thr Ile
385                 390                 395                 400
Gln Gln Asn Gly Leu Ser Thr Met Gln Asn Asn Leu Ala Arg Val Leu
                405                 410                 415
Arg Pro Val Arg Ala Gly Ile Thr Gly Asp Phe Ser Ala Glu Ser Gln
            420                 425                 430
Phe Ala Gly Asn Ile Glu Ile Gly Ala Pro Val Pro Leu Ala Ala Asp
        435                 440                 445
Ser Lys Val Arg Arg Ala Arg Ser Val Asp Gly Ala Gly Gln Gly Leu
    450                 455                 460
Arg Leu Glu Ile Pro Leu Asp Ala Gln Glu Leu Ser Gly Leu Gly Phe
465                 470                 475                 480
Asn Asn Val Ser Leu Ser Val Thr Pro Ala Ala Asn Gln
                485                 490

<210> 5
<211> 1541
<212> DNA
<213> Clostridium septicum

<400> 5
tgttaataat atgttaatat tttgataaca tttattatat aataaattat ttattttaaa 60
attaaaggga gggatattta tgtcaaaaaa atcttttgct aaaaaagtaa tttgtacatc 120
tatgattgca attcagtgtg cggcagtagt accacatgta caagcttatg cacttacaaa 180
tcttgaagag gggggatatg caaatcataa taatgcttct tcaattaaaa tatttggata 240
tgaagacaat gaagatttaa aagctaaaat tattcaagat ccagagttta taagaaattg 300
ggcaaatgta gctcattcat taggatttgg atggtgcggt ggaacggcta atccaaacgt 360
tggacaaggt tttgaattta aaagagaagt tggggcaggt ggaaaagtat cttatttatt 420

atctgctaga tacaatccaa atgatcctta tgcaagtgga tatcgtgcaa aagatagact 480
ttctatgaaa atatcaaatg ttagatttgt tattgataat gattctataa aattaggtac 540
acctaaagtg aaaaaattag cacctttaaa ctctgctagt tttgatttaa aaatgaaagt 600
aaaactgagt ctaaattatc aaaaacattt aattatacaa cttctaaaac agtttctaaa 660
acagataact ttaaatttgg agaaaaaata ggagtaaaaa catcatttaa agtaggtctt 720
gaagctatag ctgacagtaa agttgagaca agctttgaat ttaatgcaga acaaggttgg 780
tcaaatacaa atagtactac tgaaactaaa caagaaagta ctacatatac tgcaacagtt 840
tctccacaaa ctaaaaagag attattccta gatgtgttag gatcacaaat tgatattcct 900
tatgaaggaa aaatatatat ggaatacgac atagaattaa tgggattttt aagatataca 960
ggaaatgctc gtgaagatca tactgaagat agaccaacag ttaaacttaa atttggtaaa 1020
aacggtatga gtgctgagga acatcttaaa gatttatata gtcataagaa tattaatgga 1080
tattcagaat gggattggaa atgggtagat gagaaatttg gttatttatt taaaaattca 1140
tacgatgctc ttactagtag aaaattagga ggaataataa aaggctcatt tactaacatt 1200
aatggaacaa aaatagtaat tagagaaggt aaagaaattc cacttcctga taagaagaga 1260
agaggaaaac gttcagtaga ttctttagat gctagattac aaaatgaagg tattagaata 1320
gaaaatattg aaacacaaga tgttccagga tttagactaa atagcataac atacaatgat 1380
aaaaaattga tattaattaa taatatataa ttataattta ttaaaatatg cttctctata 1440
ctttatatta atatttaaag tataaaaact aacaaaatct cacttagtag gtagaattgt 1500
ataaaaacaa atctacctac tattttttta ttatttagtc g 1541

<210> 6
<211> 443
<212> PRT
<213> Clostridium septicum

<400> 6
Met Ser Lys Lys Ser Phe Ala Lys Lys Val Ile Cys Thr Ser Met Ile
1               5                   10                  15
Ala Ile Gln Cys Ala Ala Val Val Pro His Val Gln Ala Tyr Ala Leu
            20                  25                  30
Thr Asn Leu Glu Glu Gly Gly Tyr Ala Asn His Asn Asn Ala Ser Ser
        35                  40                  45
Ile Lys Ile Phe Gly Tyr Glu Asp Asn Glu Asp Leu Lys Ala Lys Ile
    50                  55                  60
Ile Gln Asp Pro Glu Phe Ile Arg Asn Trp Ala Asn Val Ala His Ser
65                  70                  75                  80
Leu Gly Phe Gly Trp Cys Gly Gly Thr Ala Asn Pro Asn Val Gly Gln
                85                  90                  95
Gly Phe Glu Phe Lys Arg Glu Val Gly Ala Gly Gly Lys Val Ser Tyr
            100                 105                 110
Leu Leu Ser Ala Arg Tyr Asn Pro Asn Asp Pro Tyr Ala Ser Gly Tyr
        115                 120                 125
Arg Ala Lys Asp Arg Leu Ser Met Lys Ile Ser Asn Val Arg Phe Val
    130                 135                 140
Ile Asp Asn Asp Ser Ile Lys Leu Gly Thr Pro Lys Val Lys Lys Leu
145                 150                 155                 160
Ala Pro Leu Asn Ser Ala Ser Phe Asp Leu Ile Asn Glu Ser Lys Thr
                165                 170                 175
Glu Ser Lys Leu Ser Lys Thr Phe Asn Tyr Thr Thr Ser Lys Thr Val
            180                 185                 190
Ser Lys Thr Asp Asn Phe Lys Phe Gly Glu Lys Ile Gly Val Lys Thr
        195                 200                 205
Ser Phe Lys Val Gly Leu Glu Ala Ile Ala Asp Ser Lys Val Glu Thr
    210                 215                 220
Ser Phe Glu Phe Asn Ala Glu Gln Gly Trp Ser Asn Thr Asn Ser Thr
225                 230                 235                 240
Thr Glu Thr Lys Gln Glu Ser Thr Thr Tyr Thr Ala Thr Val Ser Pro
                245                 250                 255
Gln Thr Lys Lys Arg Leu Phe Leu Asp Val Leu Gly Ser Gln Ile Asp
            260                 265                 270
Ile Pro Tyr Glu Gly Lys Ile Tyr Met Glu Tyr Asp Ile Glu Leu Met
        275                 280                 285
Gly Phe Leu Arg Tyr Thr Gly Asn Ala Arg Glu Asp His Thr Glu Asp
    290                 295                 300
Arg Pro Thr Val Lys Leu Lys Phe Gly Lys Asn Gly Met Ser Ala Glu
305                 310                 315                 320
Glu His Leu Lys Asp Leu Tyr Ser His Lys Asn Ile Asn Gly Tyr Ser

                  325                 330                 335
Glu Trp Asp Trp Lys Trp Val Asp Glu Lys Phe Gly Tyr Leu Phe Lys
            340                 345                 350
Asn Ser Tyr Asp Ala Leu Thr Ser Arg Lys Leu Gly Gly Ile Ile Lys
        355                 360                 365
Gly Ser Phe Thr Asn Ile Asn Gly Thr Lys Ile Val Ile Arg Glu Gly
    370                 375                 380
Lys Glu Ile Pro Leu Pro Asp Lys Lys Arg Arg Gly Lys Arg Ser Val
385                 390                 395                 400
Asp Ser Leu Asp Ala Arg Leu Gln Asn Glu Gly Ile Arg Ile Glu Asn
                405                 410                 415
Ile Glu Thr Gln Asp Val Pro Gly Phe Arg Leu Asn Ser Ile Thr Tyr
            420                 425                 430
Asn Asp Lys Lys Leu Ile Leu Ile Asn Asn Ile
        435                 440

<210> 7
<211> 1332
<212> DNA
<213> Clostridium septicum

<400> 7
   atgtcaaaaa aatcttttgc taaaaaagta atttgtacat ctatgattgc aattcagtgt 60
   gcggcagtag taccacatgt acaagcttat gcacttacaa atcttgaaga ggggggatat 120
   gcaaatcata ataatgcttc ttcaattaaa atatttggat atgaagacaa tgaagattta 180
   aaagctaaaa ttattcaaga tccagagttt ataagaaatt gggcaaatgt agctcattca 240
   ttaggatttg gatggtgcgg tggaacggct aatccaaacg ttggacaagg ttttgaattt 300
   aaaagagaag ttggggcagg tggaaaagta tcttatttat tatctgctag atacaatcca 360
   aatgatcctt atgcaagtgg atatcgtgca aaagatagac tttctatgaa aatatcaaat 420
   gttagatttg ttattgataa tgattctata aaattaggta cacctaaagt gaaaaaatta 480
   gcacctttaa actctgctag ttttgattta ataaatgaaa gtaaaactga gtctaaatta 540
   tcaaaaacat ttaattatac aacttctaaa acagtttcta aaacagataa ctttaaattt 600
   ggagaaaaaa taggagtaaa aacatcattt aaagtaggtc ttgaagctat agctgacagt 660
   aaagttgaga caagctttga atttaatgca gaacaaggtt ggtcaaatac aaatagtact 720
   actgaaacta aacaagaaag tactacatat actgcaacag tttctccaca aactaaaaag 780
   agattattcc tagatgtgtt aggatcacaa attgatattc cttatgaagg aaaaatatat 840
   atggaatacg acatagaatt aatgggattt ttaagatata caggaaatgc tcgtgaagat 900
   catactgaag atagaccaac agttaaactt aaatttggta aaaacggtat gagtgctgag 960
   gaacatctta aagatttata tagtcataag aatattaatg gatattcaga atgggattgg 1020
   aaatgggtag atgagaaatt tggttattta tttaaaaatt catacgatgc tcttactagt 1080
   agaaaattag gaggaataat aaaaggctca tttactaaca ttaatggaac aaaaatagta 1140
   attagagaag gtaaagaaat tccacttcct gataagaaga gaagaggaaa acgttcagta 1200
   gattctttag atgctagatt acaaaatgaa ggtattagaa tagaaaatat tgaaacacaa 1260
   gatgttccag gatttagact aaatagcata acatacaatg ataaaaaatt gatattaatt 1320
   aataatatat aa                                                     1332

<210> 8
<211> 443
<212> PRT
<213> Clostridium septicum

<400> 8
Met Ser Lys Lys Ser Phe Ala Lys Lys Val Ile Cys Thr Ser Met Ile
1               5                   10                  15
Ala Ile Gln Cys Ala Ala Val Val Pro His Val Gln Ala Tyr Ala Leu
            20                  25                  30
Thr Asn Leu Glu Glu Gly Gly Tyr Ala Asn His Asn Asn Ala Ser Ser
        35                  40                  45
Ile Lys Ile Phe Gly Tyr Glu Asp Asn Glu Asp Leu Lys Ala Lys Ile
    50                  55                  60
Ile Gln Asp Pro Glu Phe Ile Arg Asn Trp Ala Asn Val Ala His Ser
65                  70                  75                  80
Leu Gly Phe Gly Trp Cys Gly Gly Thr Ala Asn Pro Asn Val Gly Gln
                85                  90                  95
Gly Phe Glu Phe Lys Arg Glu Val Gly Ala Gly Gly Lys Val Ser Tyr
            100                 105                 110

Leu Leu Ser Ala Arg Tyr Asn Pro Asn Asp Pro Tyr Ala Ser Gly Tyr
            115                 120                 125
Arg Ala Lys Asp Arg Leu Ser Met Lys Ile Ser Asn Val Arg Phe Val
    130                 135                 140
Ile Asp Asn Asp Ser Ile Lys Leu Gly Thr Pro Lys Val Lys Lys Leu
145                 150                 155                 160
Ala Pro Leu Asn Ser Ala Ser Phe Asp Leu Ile Asn Glu Ser Lys Thr
                165                 170                 175
Glu Ser Lys Leu Ser Lys Thr Phe Asn Tyr Thr Thr Ser Lys Thr Val
            180                 185                 190
Ser Lys Thr Asp Asn Phe Lys Phe Gly Glu Lys Ile Gly Val Lys Thr
        195                 200                 205
Ser Phe Lys Val Gly Leu Glu Ala Ile Ala Asp Ser Lys Val Glu Thr
    210                 215                 220
Ser Phe Glu Phe Asn Ala Glu Gln Gly Trp Ser Asn Thr Asn Ser Thr
225                 230                 235                 240
Thr Glu Thr Lys Gln Glu Ser Thr Thr Tyr Thr Ala Thr Val Ser Pro
                245                 250                 255
Gln Thr Lys Lys Arg Leu Phe Leu Asp Val Leu Gly Ser Gln Ile Asp
            260                 265                 270
Ile Pro Tyr Glu Gly Lys Ile Tyr Met Glu Tyr Asp Ile Glu Leu Met
        275                 280                 285
Gly Phe Leu Arg Tyr Thr Gly Asn Ala Arg Glu Asp His Thr Glu Asp
    290                 295                 300
Arg Pro Thr Val Lys Leu Lys Phe Gly Lys Asn Gly Met Ser Ala Glu
305                 310                 315                 320
Glu His Leu Lys Asp Leu Tyr Ser His Lys Asn Ile Asn Gly Tyr Ser
                325                 330                 335
Glu Trp Asp Trp Lys Trp Val Asp Glu Lys Phe Gly Tyr Leu Phe Lys
            340                 345                 350
Asn Ser Tyr Asp Ala Leu Thr Ser Arg Lys Leu Gly Gly Ile Ile Lys
        355                 360                 365
Gly Ser Phe Thr Asn Ile Asn Gly Thr Lys Ile Val Ile Arg Glu Gly
    370                 375                 380
Lys Glu Ile Pro Leu Pro Asp Lys Lys Arg Arg Gly Lys Arg Ser Val
385                 390                 395                 400
Asp Ser Leu Asp Ala Arg Leu Gln Asn Glu Gly Ile Arg Ile Glu Asn
                405                 410                 415
Ile Glu Thr Gln Asp Val Pro Gly Phe Arg Leu Asn Ser Ile Thr Tyr
            420                 425                 430
Asn Asp Lys Lys Leu Ile Leu Ile Asn Asn Ile
        435                 440


<210> 9
<211> 116
<212> PRT
<213> Artificial Sequence

<220>
<223> AFAI antibody fragment

<400> 9
Val Phe Asp Val Gln Leu Gln Ala Ser Gly Gly Gly Val Val Gln Pro
1               5                   10                  15
Gly Gly Ser Leu Arg Leu Ser Cys Ala Ala His Asp Pro Ile Phe Asp
            20                  25                  30
Lys Asn Leu Met Gly Trp Gly Arg Gln Arg Pro Gly Lys Gln Arg Glu
        35                  40                  45
Tyr Val Ala Thr Ile Ser Gly Asn Gly Gly Thr Asn Tyr Ala Ser Ser
    50                  55                  60
Val Glu Gly Arg Phe Thr Ile Ser Arg Asp Asn Ala Lys Lys Thr Val
65                  70                  75                  80
Tyr Leu Gln Met Asn Asp Leu Lys Pro Glu Asp Thr Ala Val Tyr Tyr
                85                  90                  95
Cys Asn Ser Ala Phe Ala Ile Trp Gly Gln Gly Ile Gln Val Thr Val
            100                 105                 110

Ser Ser Val His
        115

<210> 10
<211> 27
<212> DNA
<213> Artificial Sequence

<220>
<223> Primer

<400> 10
gcggctgaca gcagtgggcg tcgtgct                                          27

<210> 11
<211> 28
<212> DNA
<213> Artificial Sequence

<220>
<223> Primer

<400> 11
agcacgacgc ccactgctgt cagccgcg                                         28

<210> 12
<211> 31
<212> DNA
<213> Artificial Sequence

<220>
<223> Primer

<400> 12
agcagtgggc gtagcgctca aagtgtggac g                                     31

<210> 13
<211> 31
<212> DNA
<213> Artificial Sequence

<220>
<223> Primer

<400> 13
gtccacactt tgagcgctac gcccactgct g                                     31

<210> 14
<211> 40
<212> DNA
<213> Artificial Sequence

<220>
<223> Primer

<400> 14
gtctcctcag tgcacctagt ccctgcagag cccgtctatc                            40

<210> 15
<211> 40
<212> DNA
<213> Artificial Sequence

<220>
<223> Primer

<400> 15
atagacgggc tctgcaggga ctaggtgcac tgaggagacg                                40

<210> 16
<211> 40
<212> DNA
<213> Artificial Sequence

<220>
<223> Primer

<400> 16
gtgcacctag tccctcgtgg ttccgcagag cccgtctatc                                40

<210> 17
<211> 41
<212> DNA
<213> Artificial Sequence

<220>
<223> Primer

<400> 17
atagacgggc tctgcggaac cacgagggac taggtgcact g                              41

<210> 18
<211> 40
<212> DNA
<213> Artificial Sequence

<220>
<223> Primer

<400> 18
gtctcctcag tgcactcagg ccgtagtgct caagcagagc                                40

<210> 19
<211> 52
<212> DNA
<213> Artificial Sequence

<220>
<223> Primer

<400> 19
ctggatagac gggctctgct tgagcactac ggcctgagtg cactgaggag ac                  52

<210> 20
<211> 1500
<212> DNA
<213> Artificial Sequence

<220>
<223> proaerolysin with a uPA cleavage site in the
      activation sequence

<400> 20
atgcaaaaaa taaaactaac tggcttgtca ttaatcatat ccggcctgct gatggcacag 60
gcgcaagcgg cagagcccgt ctatccagac cagcttcgct tgttttcatt gggccaaggg 120
gtctgtggcg acaagtatcg ccccgtcaat cgagaagaag cccaaagcgt taaaagcaat 180
attgtcggca tgatggggca atggcaaata agcgggctgg ccaacggctg ggtcattatg 240
gggccgggtt ataacggtga aataaaacca gggacagcgt ccaatacctg gtgttatccg 300
accaatcctg ttaccggtga aataccgaca ctgtctgccc tcgagattcc agatggtgac 360
gaagtcgatg tgcagtggcg actggtacat gacagtgcga atttcatcaa accaaccagc 420
tatctggccc attacctcgg ttatgcctgg gtgggcggca atcacagcca atatgtcggc 480
gaagacatgg atgtgacccg tgatggcgac ggctgggtga tccgtggcaa caatgacggc 540
ggctgtgacg gctatcgctg tggtgacaag acggccatca aggtcagcaa cttcgcctat 600

aacctggatc ccgacagctt caagcatggc gatgtcaccc agtccgaccg ccagctggtc 660
aagactgtgg tgggctgggc ggtcaacgac agcgacaccc cccaatccgg ctatgacgtc 720
accctgcgct acgacacagc caccaactgg tccaagacca acacctatgg cctgagcgag 780
aaggtgacca ccaagaacaa gttcaagtgg ccactggtgg gggaaaccga actctccatc 840
gagattgctg ccaatcagtc ctgggcgtcc cagaacgggg gctcgaccac cacctccctg 900
tctcagtccg tgcgaccgac tgtgccggcc cgctccaaga tcccggtgaa gatagagctc 960
tacaaggccg acatctccta tccctatgag ttcaaggccg atgtcagcta tgacctgacc 1020
ctgagcggct tcctgcgctg gggcggcaac gcctggtata cccacccgga caaccgtccg 1080
aactggaacc acaccttcgt cataggtccg tacaaggaca aggcgagcag cattcggtac 1140
cagtgggaca agcgttacat cccgggtgaa gtgaagtggt gggactggaa ctggaccata 1200
cagcagaacg gtctgtctac catgcagaac aacctggcca gagtgctgcg cccggtgcgg 1260
gcggggatca ccggtgattt cagtgccgag agccagtttg ccggcaacat agagatcggt 1320
gctcccgtgc cgctcgcggc tgacagcagt gggcgtagcg ctcaaagtgt ggacggcgct 1380
ggtcaaggcc tgaggctgga gatcccgctc gatgcgcaag agctctccgg gcttggcttc 1440
aacaacgtca gcctcagcgt gacccctgct gccaatcaac atcatcatca tcatcattaa 1500

<210> 21
<211> 499
<212> PRT
<213> Artificial Sequence

<220>
<223> proaerolysin with a uPA cleavage site in the
      activation sequence

<400> 21
Met Gln Lys Ile Lys Leu Thr Gly Leu Ser Leu Ile Ile Ser Gly Leu
1               5                   10                  15
Leu Met Ala Gln Ala Gln Ala Ala Glu Pro Val Tyr Pro Asp Gln Leu
            20                  25                  30
Arg Leu Phe Ser Leu Gly Gln Gly Val Cys Gly Asp Lys Tyr Arg Pro
        35                  40                  45
Val Asn Arg Glu Glu Ala Gln Ser Val Lys Ser Asn Ile Val Gly Met
    50                  55                  60
Met Gly Gln Trp Gln Ile Ser Gly Leu Ala Asn Gly Trp Val Ile Met
65                  70                  75                  80
Gly Pro Gly Tyr Asn Gly Glu Ile Lys Pro Gly Thr Ala Ser Asn Thr
                85                  90                  95
Trp Cys Tyr Pro Thr Asn Pro Val Thr Gly Glu Ile Pro Thr Leu Ser
            100                 105                 110
Ala Leu Glu Ile Pro Asp Gly Asp Glu Val Asp Val Gln Trp Arg Leu
        115                 120                 125
Val His Asp Ser Ala Asn Phe Ile Lys Pro Thr Ser Tyr Leu Ala His
    130                 135                 140
Tyr Leu Gly Tyr Ala Trp Val Gly Gly Asn His Ser Gln Tyr Val Gly
145                 150                 155                 160
Glu Asp Met Asp Val Thr Arg Asp Gly Asp Gly Trp Val Ile Arg Gly
                165                 170                 175
Asn Asn Asp Gly Gly Cys Asp Gly Tyr Arg Cys Gly Asp Lys Thr Ala
            180                 185                 190
Ile Lys Val Ser Asn Phe Ala Tyr Asn Leu Asp Pro Asp Ser Phe Lys
        195                 200                 205
His Gly Asp Val Thr Gln Ser Asp Arg Gln Leu Val Lys Thr Val Val
    210                 215                 220
Gly Trp Ala Val Asn Asp Ser Asp Thr Pro Gln Ser Gly Tyr Asp Val
225                 230                 235                 240
Thr Leu Arg Tyr Asp Thr Ala Thr Asn Trp Ser Lys Thr Asn Thr Tyr
                245                 250                 255
Gly Leu Ser Glu Lys Val Thr Thr Lys Asn Lys Phe Lys Trp Pro Leu
            260                 265                 270
Val Gly Glu Thr Glu Leu Ser Ile Glu Ile Ala Ala Asn Gln Ser Trp
        275                 280                 285
Ala Ser Gln Asn Gly Gly Ser Thr Thr Thr Ser Leu Ser Gln Ser Val
    290                 295                 300
Arg Pro Thr Val Pro Ala Arg Ser Lys Ile Pro Val Lys Ile Glu Leu
305                 310                 315                 320

Tyr Lys Ala Asp Ile Ser Tyr Pro Tyr Glu Phe Lys Ala Asp Val Ser
                325                 330                 335
Tyr Asp Leu Thr Leu Ser Gly Phe Leu Arg Trp Gly Gly Asn Ala Trp
            340                 345                 350
Tyr Thr His Pro Asp Asn Arg Pro Asn Trp Asn His Thr Phe Val Ile
        355                 360                 365
Gly Pro Tyr Lys Asp Lys Ala Ser Ser Ile Arg Tyr Gln Trp Asp Lys
    370                 375                 380
Arg Tyr Ile Pro Gly Glu Val Lys Trp Trp Asp Trp Asn Trp Thr Ile
385                 390                 395                 400
Gln Gln Asn Gly Leu Ser Thr Met Gln Asn Asn Leu Ala Arg Val Leu
                405                 410                 415
Arg Pro Val Arg Ala Gly Ile Thr Gly Asp Phe Ser Ala Glu Ser Gln
            420                 425                 430
Phe Ala Gly Asn Ile Glu Ile Gly Ala Pro Val Pro Leu Ala Ala Asp
        435                 440                 445
Ser Ser Gly Arg Ser Ala Gln Ser Val Asp Gly Ala Gly Gln Gly Leu
    450                 455                 460
Arg Leu Glu Ile Pro Leu Asp Ala Gln Glu Leu Ser Gly Leu Gly Phe
465                 470                 475                 480
Asn Asn Val Ser Leu Ser Val Thr Pro Ala Ala Asn Gln His His His
                485                 490                 495
His His His

<210> 22
<211> 1842
<212> DNA
<213> Artificial Sequence

<220>
<223> proaerolysin with a uPA cleavage site in the
      activation sequence, and an AFAI antibody fragment
      as a targeting unit

<400> 22
atgaaaaaaa ccgctatcgc gatcgcagtt gcactggctg gtttcgctac cgttgcgcag 60
gccgtcttcg atgtgcagct gcaggcgtct ggaggaggcg tggtgcagcc tgggggtct 120
ctgagactct cctgtgcagc ccatgatccc atcttcgata agaatctcat gggctggggc 180
cgccaggctc caggaaagca gcgcgaatat gtcgcgacta ttagtggtaa tggtggaaca 240
aattatgcaa gctccgttga gggccgattc accatctcta gagacaacgc caagaaaacg 300
gtgtatctgc aaatgaacga cctgaaacct gaggacacgg ccgtctatta ctgtaactca 360
gcttttgcta tctgggcca gggcatccag gtcaccgtct cctcagtgca cgcagagccc 420
gtctatccag accagcttcg cttgttttca ttgggccaag gggtctgtgg cgacaagtat 480
cgccccgtca atcgagaaga agcccaaagc gttaaaagca atattgtcgg catgatgggg 540
caatggcaaa taagcgggct ggccaacggc tgggtcatta tggggccggg ttataacggt 600
gaaataaaac cagggacagc gtccaatacc tggtgttatc cgaccaatcc tgttaccggt 660
gaaataccga cactgtctgc cctcgagatt ccagatggtg acgaagtcga tgtgcagtgg 720
cgactggtac atgacagtgc gaatttcatc aaaccaacca gctatctggc ccattacctc 780
ggttatgcct gggtgggcgg caatcacagc caatatgtcg gcgaagacat ggatgtgacc 840
cgtgatggcg acggctgggt gatccgtggc aacaatgacg gcggctgtga cggctatcgc 900
tgtggtgaca agacggccat caaggtcagc aacttcgcct ataacctgga tcccgacagc 960
ttcaagcatg gcgatgtcac ccagtccgac cgccagctgg tcaagactgt ggtgggctgg 1020
gcggtcaacg acagcgacac cccccaatcc ggctatgacg tcaccctgcg ctacgacaca 1080
gccaccaact ggtccaagac caacacctat ggcctgagcg agaaggtgac caccaagaac 1140
aagttcaagt ggccactggt gggggaaacc gaactctcca tcgagattgc tgccaatcag 1200
tcctgggcgt cccagaacgg gggctcgacc accacctccc tgtctcagtc cgtgcgaccg 1260
actgtgccgg cccgctccaa gatcccggtg aagatagagc tctacaaggc cgacatctcc 1320
tatccctatg agttcaaggc cgatgtcagc tatgacctga ccctgagcgg cttcctgcgc 1380
tggggcggca acgcctggta tacccacccg gacaaccgtc cgaactggaa ccacaccttc 1440
gtcataggtc cgtacaagga caaggcgagc agcattcggt accagtggga caagcgttac 1500
atcccgggtg aagtgaagtg gtgggactgg aactggacca tacagcagaa cggtctgtct 1560
accatgcaga acaacctggc cagagtgctg cgcccggtgc gggcggggat caccggtgat 1620
ttcagtgccg agagccagtt tgccggcaac atagagatcg gtgctcccgt gccgctcgcg 1680
gctgacagca gtgggcgtag cgctcaaagt gtggacggcg ctggtcaagg cctgaggctg 1740
gagatcccgc tcgatgcgca agagctctcc gggcttggct tcaacaacgt cagcctcagc 1800

gtgacccctg ctgccaatca acatcatcat catcatcatt ga                    1842

<210> 23
<211> 613
<212> PRT
<213> Artificial Sequence

<220>
<223> proaerolysin with a uPA cleavage site in the
      activation sequence, and an AFAI antibody fragment
      as a targeting unit

<400> 23
Met Lys Lys Thr Ala Ile Ala Ile Ala Val Ala Leu Ala Gly Phe Ala
1               5                   10                  15
Thr Val Ala Gln Ala Val Phe Asp Val Gln Leu Gln Ala Ser Gly Gly
            20                  25                  30
Gly Val Val Gln Pro Gly Gly Ser Val Arg Leu Ser Cys Ala Ala His
        35                  40                  45
Asp Pro Ile Phe Asp Lys Asn Leu Met Gly Trp Gly Arg Gln Ala Pro
    50                  55                  60
Gly Lys Gln Arg Glu Tyr Val Ala Thr Ile Ser Gly Asn Gly Gly Thr
65                  70                  75                  80
Asn Tyr Ala Ser Ser Val Glu Gly Arg Phe Thr Ile Ser Arg Asp Asn
                85                  90                  95
Ala Lys Lys Thr Val Tyr Leu Gln Met Asn Asp Leu Lys Pro Glu Asp
            100                 105                 110
Thr Ala Val Tyr Tyr Cys Asn Ser Ala Phe Ala Ile Trp Gly Gln Gly
        115                 120                 125
Ile Gln Val Thr Val Ser Ser Val His Ala Glu Pro Val Tyr Pro Asp
    130                 135                 140
Gln Leu Arg Leu Phe Ser Leu Gly Gln Gly Val Cys Gly Asp Lys Tyr
145                 150                 155                 160
Arg Pro Val Asn Arg Glu Glu Ala Gln Ser Val Lys Ser Asn Ile Val
                165                 170                 175
Gly Met Met Gly Gln Trp Gln Ile Ser Gly Leu Ala Asn Gly Trp Val
            180                 185                 190
Ile Met Gly Pro Gly Tyr Asn Gly Glu Ile Lys Pro Gly Thr Ala Ser
        195                 200                 205
Asn Thr Trp Cys Tyr Pro Thr Asn Pro Val Thr Gly Glu Ile Pro Thr
    210                 215                 220
Leu Ser Ala Leu Asp Ile Pro Asp Gly Asp Glu Val Asp Val Gln Trp
225                 230                 235                 240
Arg Leu Val His Asp Ser Ala Asn Phe Ile Lys Pro Thr Ser Tyr Leu
                245                 250                 255
Ala His Tyr Leu Gly Tyr Ala Trp Val Gly Gly Asn His Ser Gln Tyr
            260                 265                 270
Val Gly Glu Asp Met Asp Val Thr Arg Asp Gly Asp Gly Trp Val Ile
        275                 280                 285
Arg Gly Asn Asn Asp Gly Gly Cys Asp Gly Tyr Arg Cys Gly Asp Lys
    290                 295                 300
Thr Ala Ile Lys Val Ser Asn Phe Ala Tyr Asn Leu Asp Pro Asp Ser
305                 310                 315                 320
Phe Lys His Gly Asp Val Thr Gln Ser Asp Arg Gln Leu Val Lys Thr
                325                 330                 335
Val Val Gly Trp Ala Val Asn Asp Ser Asp Thr Pro Gln Ser Gly Tyr
            340                 345                 350
Asp Val Thr Leu Arg Tyr Asp Thr Ala Thr Asn Trp Ser Lys Thr Asn
        355                 360                 365
Thr Tyr Gly Leu Ser Glu Lys Val Thr Thr Lys Asn Lys Phe Lys Trp
    370                 375                 380
Pro Leu Val Gly Glu Thr Glu Leu Ser Ile Glu Ile Ala Ala Asn Gln
385                 390                 395                 400
Ser Trp Ala Ser Gln Asn Gly Gly Ser Thr Thr Thr Ser Leu Ser Gln
                405                 410                 415
Ser Val Arg Pro Thr Val Pro Ala Arg Ser Lys Ile Pro Val Lys Ile
            420                 425                 430

Glu Leu Tyr Lys Ala Asp Ile Ser Tyr Pro Tyr Glu Phe Lys Ala Asp
        435                 440                 445
Val Ser Tyr Asp Leu Thr Leu Ser Gly Phe Leu Arg Trp Gly Gly Asn
    450                 455                 460
Ala Trp Tyr Thr His Pro Asp Asn Arg Pro Asn Trp Asn His Thr Phe
465                 470                 475                 480
Val Ile Gly Pro Tyr Lys Asp Lys Ala Ser Ser Ile Arg Tyr Gln Trp
                485                 490                 495
Asp Lys Arg Tyr Ile Pro Gly Glu Val Lys Trp Trp Asp Trp Asn Trp
            500                 505                 510
Thr Ile Gln Gln Asn Gly Leu Ser Thr Met Gln Asn Asn Leu Ala Arg
        515                 520                 525
Val Leu Arg Pro Val Arg Ala Gly Ile Thr Gly Asp Phe Ser Ala Glu
    530                 535                 540
Ser Gln Phe Ala Gly Asn Ile Glu Ile Gly Ala Pro Val Pro Leu Ala
545                 550                 555                 560
Ala Asp Ser Ser Gly Arg Ser Ala Gln Ser Val Asp Gly Ala Gly Gln
                565                 570                 575
Gly Leu Arg Leu Glu Ile Pro Leu Asp Ala Gln Glu Leu Ser Gly Leu
            580                 585                 590
Gly Phe Asn Asn Val Ser Leu Ser Val Thr Pro Ala Ala Asn Gln His
        595                 600                 605
His His His His His
    610

<210> 24
<211> 1860
<212> DNA
<213> Artificial Sequence

<220>
<223> proaerolysin with a uPA cleavage site in the
      activation sequence, and an AFAI antibody fragment
      as a targeting unit, attached via a uPA-cleavable
      linker

<400> 24
atgaaaaaaa ccgctatcgc gatcgcagtt gcactggctg gtttcgctac cgttgcgcag 60
gccgtcttcg atgtgcagct gcaggcgtct ggaggaggcg tggtgcagcc tggggggtct 120
ctgagactct cctgtgcagc ccatgatccc atcttcgata agaatctcat gggctggggc 180
cgccaggctc caggaaagca gcgcgaatat gtcgcgacta ttagtggtaa tggtggaaca 240
aattatgcaa gctccgttga gggccgattc accatctcta gagacaacgc caagaaaacg 300
gtgtatctgc aaatgaacga cctgaaacct gaggacacgg ccgtctatta ctgtaactca 360
gcttttgcta tctggggcca gggcatccag gtcaccgtct cctcagtgca ctcaggccgt 420
agtgctcaag cagagcccgt ctatccagac cagcttcgct tgttttcatt gggccaaggg 480
gtctgtggcg acaagtatcg ccccgtcaat cgagaagaag cccaaagcgt taaaagcaat 540
attgtcggca tgatggggca atggcaaata agcgggctgg ccaacggctg ggtcattatg 600
gggccgggtt ataacggtga aataaaacca gggacagcgt ccaatacctg gtgttatccg 660
accaatcctg ttaccggtga aataccgaca ctgtctgccc tcgagattcc agatggtgac 720
gaagtcgatg tgcagtggcg actggtacat gacagtgcga atttcatcaa accaaccagc 780
tatctggccc attacctcgg ttatgcctgg gtgggcggca atcacagcca atatgtcggc 840
gaagacatgg atgtgacccg tgatggcgac ggctgggtga tccgtggcaa caatgacggc 900
ggctgtgacg gctatcgctg tggtgacaag acggccatca aggtcagcaa cttcgcctat 960
aacctggatc ccgacagctt caagcatggc gatgtcaccc agtccgaccg ccagctggtc 1020
aagactgtgg tgggctgggc ggtcaacgac agcgacaccc cccaatccgg ctatgacgtc 1080
accctgcgct acgacacagc caccaactgg tccaagacca acacctatgg cctgagcgag 1140
aaggtgacca ccaagaacaa gttcaagtgg ccactggtgg gggaaaccga actctccatc 1200
gagattgctg ccaatcagtc ctgggcgtcc cagaacgggg gctcgaccac cacctccctg 1260
tctcagtccg tgcgaccgac tgtgccggcc cgctccaaga tcccggtgaa gatagagctc 1320
tacaaggccg acatctccta tccctatgag ttcaaggccg atgtcagcta tgacctgacc 1380
ctgagcggct tcctgcgctg gggcggcaac gcctggtata cccacccgga caaccgtccg 1440
aactggaacc acaccttcgt cataggtccg tacaaggaca aggcgagcag cattcggtac 1500
cagtgggaca agcgttacat cccgggtgaa gtgaagtggt gggactggaa ctggaccata 1560
cagcagaacg gtctgtctac catgcagaac aacctggcca gagtgctgcg cccggtgcgg 1620
gcggggatca ccggtgattt cagtgccgag agccagtttg ccggcaacat agagatcggt 1680
gctcccgtgc cgctcgcggc tgacagcagt gggcgtagcg ctcaaagtgt ggacggcgct 1740

ggtcaaggcc tgaggctgga gatcccgctc gatgcgcaag agctctccgg gcttggcttc 1800
aacaacgtca gcctcagcgt gacccctgct gccaatcaac atcatcatca tcatcattga 1860

<210> 25
<211> 619
<212> PRT
<213> Artificial Sequence

<220>
<223> proaerolysin with a uPA cleavage site in the
      activation sequence, and an AFAI antibody fragment
      as a targeting unit, attached via a uPA-cleavable
      linker

<400> 25
Met Lys Lys Thr Ala Ile Ala Ile Ala Val Ala Leu Ala Gly Phe Ala
1               5                   10                  15
Thr Val Ala Gln Ala Val Phe Asp Val Gln Leu Gln Ala Ser Gly Gly
            20                  25                  30
Gly Val Val Gln Pro Gly Gly Ser Val Arg Leu Ser Cys Ala Ala His
        35                  40                  45
Asp Pro Ile Phe Asp Lys Asn Leu Met Gly Trp Gly Arg Gln Ala Pro
    50                  55                  60
Gly Lys Gln Arg Glu Tyr Val Ala Thr Ile Ser Gly Asn Gly Gly Thr
65                  70                  75                  80
Asn Tyr Ala Ser Ser Val Glu Gly Arg Phe Thr Ile Ser Arg Asp Asn
                85                  90                  95
Ala Lys Lys Thr Val Tyr Leu Gln Met Asn Asp Leu Lys Pro Glu Asp
            100                 105                 110
Thr Ala Val Tyr Tyr Cys Asn Ser Ala Phe Ala Ile Trp Gly Gln Gly
        115                 120                 125
Ile Gln Val Thr Val Ser Ser Val His Ser Gly Arg Ser Ala Gln Ala
    130                 135                 140
Glu Pro Val Tyr Pro Asp Gln Leu Arg Leu Phe Ser Leu Gly Gln Gly
145                 150                 155                 160
Val Cys Gly Asp Lys Tyr Arg Pro Val Asn Arg Glu Glu Ala Gln Ser
                165                 170                 175
Val Lys Ser Asn Ile Val Gly Met Met Gly Gln Trp Gln Ile Ser Gly
            180                 185                 190
Leu Ala Asn Gly Trp Val Ile Met Gly Pro Gly Tyr Asn Gly Glu Ile
        195                 200                 205
Lys Pro Gly Thr Ala Ser Asn Thr Trp Cys Tyr Pro Thr Asn Pro Val
    210                 215                 220
Thr Gly Glu Ile Pro Thr Leu Ser Ala Leu Asp Ile Pro Asp Gly Asp
225                 230                 235                 240
Glu Val Asp Val Gln Trp Arg Leu Val His Asp Ser Ala Asn Phe Ile
                245                 250                 255
Lys Pro Thr Ser Tyr Leu Ala His Tyr Leu Gly Tyr Ala Trp Val Gly
            260                 265                 270
Gly Asn His Ser Gln Tyr Val Gly Glu Asp Met Asp Val Thr Arg Asp
        275                 280                 285
Gly Asp Gly Trp Val Ile Arg Gly Asn Asn Asp Gly Gly Cys Asp Gly
    290                 295                 300
Tyr Arg Cys Gly Asp Lys Thr Ala Ile Lys Val Ser Asn Phe Ala Tyr
305                 310                 315                 320
Asn Leu Asp Pro Asp Ser Phe Lys His Gly Asp Val Thr Gln Ser Asp
                325                 330                 335
Arg Gln Leu Val Lys Thr Val Val Gly Trp Ala Val Asn Asp Ser Asp
            340                 345                 350
Thr Pro Gln Ser Gly Tyr Asp Val Thr Leu Arg Tyr Asp Thr Ala Thr
        355                 360                 365
Asn Trp Ser Lys Thr Asn Thr Tyr Gly Leu Ser Glu Lys Val Thr Thr
    370                 375                 380
Lys Asn Lys Phe Lys Trp Pro Leu Val Gly Glu Thr Glu Leu Ser Ile
385                 390                 395                 400
Glu Ile Ala Ala Asn Gln Ser Trp Ala Ser Gln Asn Gly Gly Ser Thr

                    405                 410                 415
Thr Thr Ser Leu Ser Gln Ser Val Arg Pro Thr Val Pro Ala Arg Ser
                420                 425                 430
Lys Ile Pro Val Lys Ile Glu Leu Tyr Lys Ala Asp Ile Ser Tyr Pro
            435                 440                 445
Tyr Glu Phe Lys Ala Asp Val Ser Tyr Asp Leu Thr Leu Ser Gly Phe
    450                 455                 460
Leu Arg Trp Gly Gly Asn Ala Trp Tyr Thr His Pro Asp Asn Arg Pro
465                 470                 475                 480
Asn Trp Asn His Thr Phe Val Ile Gly Pro Tyr Lys Asp Lys Ala Ser
                485                 490                 495
Ser Ile Arg Tyr Gln Trp Asp Lys Arg Tyr Ile Pro Gly Glu Val Lys
            500                 505                 510
Trp Trp Asp Trp Asn Trp Thr Ile Gln Gln Asn Gly Leu Ser Thr Met
        515                 520                 525
Gln Asn Asn Leu Ala Arg Val Leu Arg Pro Val Arg Ala Gly Ile Thr
    530                 535                 540
Gly Asp Phe Ser Ala Glu Ser Gln Phe Ala Gly Asn Ile Glu Ile Gly
545                 550                 555                 560
Ala Pro Val Pro Leu Ala Ala Asp Ser Ser Gly Arg Ser Ala Gln Ser
                565                 570                 575
Val Asp Gly Ala Gly Gln Gly Leu Arg Leu Glu Ile Pro Leu Asp Ala
            580                 585                 590
Gln Glu Leu Ser Gly Leu Gly Phe Asn Asn Val Ser Leu Ser Val Thr
        595                 600                 605
Pro Ala Ala Asn Gln His His His His His His
    610                 615


<210> 26
<211> 35
<212> DNA
<213> Artificial Sequence

<220>
<223> primer

<400> 26
ctcgcggctg acagccatcc ggtgcgtgct cgcag                                35

<210> 27
<211> 35
<212> DNA
<213> Artificial Sequence

<220>
<223> primer

<400> 27
ctgcgagcac gcaccggatg gctgtcagcc gcgag                                35

<210> 28
<211> 36
<212> DNA
<213> Artificial Sequence

<220>
<223> primer

<400> 28
gacagccatc cggtgggcct gctcagtgtg gacggc                               36

<210> 29
<211> 36
<212> DNA
<213> Artificial Sequence

<220>
<223> primer

<400> 29
gccgtccaca ctgagcaggc ccaccggatg gctgtc                                36

<210> 30
<211> 33
<212> DNA
<213> Artificial Sequence

<220>
<223> primer

<400> 30
ccggtgggcc tggtcgctcg cagtgtggac ggc                                   33

<210> 31
<211> 33
<212> DNA
<213> Artificial Sequence

<220>
<223> primer

<400> 31
gccgtccaca ctgcgagcga gcaggcccac cgg                                   33

<210> 32
<211> 36
<212> DNA
<213> Artificial Sequence

<220>
<223> primer

<400> 32
ggcctgctcg ctcgcggcgg ctcaagtgtg gacggc                                36

<210> 33
<211> 36
<212> DNA
<213> Artificial Sequence

<220>
<223> primer

<400> 33
gccgtccaca cttgagccgc cgcgagcgag caggcc                                36

<210> 34
<211> 33
<212> DNA
<213> Artificial Sequence

<220>
<223> primer

<400> 34
gctcgcggcg gctcaggccg tagtgtggag ggc                                   33

<210> 35
<211> 33
<212> DNA
<213> Artificial Sequence

<220>

<223> primer

<400> 35
gccgtccaca ctacggcctg agccgccgcg agc                                    33

<210> 36
<211> 33
<212> DNA
<213> Artificial Sequence

<220>
<223> primer

<400> 36
ggctcaggcc gtagtgcgca aagtgtggac ggc                                    33

<210> 37
<211> 33
<212> DNA
<213> Artificial Sequence

<220>
<223> primer

<400> 37
gccgtccaca ctttgcgcac tacggcctga gcc                                    33

<210> 38
<211> 1506
<212> DNA
<213> Artificial Sequence

<220>
<223> proaerolysin with an MMP2 cleavage site in the
      activation sequence

<400> 38
atgcaaaaaa taaaactaac tggcttgtca ttaatcatat ccggcctgct gatggcacag 60
gcgcaagcgg cagagcccgt ctatccagac cagcttcgct tgttttcatt gggccaaggg 120
gtctgtggcg acaagtatcg ccccgtcaat cgagaagaag cccaaagcgt taaaagcaat 180
attgtcggca tgatggggca atggcaaata agcgggctgg ccaacggctg ggtcattatg 240
gggccgggtt ataacggtga aataaaacca gggacagcgt ccaatacctg gtgttatccg 300
accaatcctg ttaccggtga aataccgaca ctgtctgccc tcgagattcc agatggtgac 360
gaagtcgatg tgcagtggcg actggtacat gacagtgcga atttcatcaa accaaccagc 420
tatctggccc attacctcgg ttatgcctgg gtgggcggca atcacagcca atatgtcggc 480
gaagacatgg atgtgacccg tgatggcgac ggctgggtga tccgtggcaa caatgacggc 540
ggctgtgacg gctatcgctg tggtgacaag acggccatca aggtcagcaa cttcgcctat 600
aacctggatc ccgacagctt caagcatggc gatgtcaccc agtccgaccg ccagctggtc 660
aagactgtgg tgggctgggc ggtcaacgac agcgacaccc cccaatccgg ctatgacgtc 720
accctgcgct acgacacagc caccaactgg tccaagacca acacctatgg cctgagcgag 780
aaggtgacca ccaagaacaa gttcaagtgg ccactggtgg gggaaaccga actctccatc 840
gagattgctg ccaatcagtc ctgggcgtcc cagaacgggg gctcgaccac cacctccctg 900
tctcagtccg tgcgaccgac tgtgccggcc cgctccaaga tcccggtgaa gatagagctc 960
tacaaggccg acatctccta tccctatgag ttcaaggccg atgtcagcta tgacctgacc 1020
ctgagcggct tcctgcgctg gggcggcaac gcctggtata cccacccgga caaccgtccg 1080
aactggaacc acaccttcgt cataggtccg tacaaggaca aggcgagcag cattcggtac 1140
cagtgggaca agcgttacat cccgggtgaa gtgaagtggt gggactggaa ctggaccata 1200
cagcagaacg gtctgtctac catgcagaac aacctggcca gagtgctgcg cccggtgcgg 1260
gcggggatca ccggtgattt cagtgccgag agccagtttg ccggcaacat agagatcggt 1320
gctcccgtgc cgctcgcggc tgacagccat ccggtgggcc tgctcgctcg cagtgtggac 1380
ggcgctggtc aaggcctgag gctggagatc ccgctcgatg cgcaagagct ctccgggctt 1440
ggcttcaaca acgtcagcct cagcgtgacc cctgctgcca atcaacatca tcatcatcat 1500
cattaa                                                                1506

<210> 39
<211> 501
<212> PRT

<213> Artificial Sequence

<220>
<223> proaerolysin with an MMP2 cleavage site in the
      activation sequence

<400> 39
Met Gln Lys Ile Lys Leu Thr Gly Leu Ser Leu Ile Ile Ser Gly Leu
1               5                   10                  15
Leu Met Ala Gln Ala Gln Ala Ala Glu Pro Val Tyr Pro Asp Gln Leu
            20                  25                  30
Arg Leu Phe Ser Leu Gly Gln Gly Val Cys Gly Asp Lys Tyr Arg Pro
        35                  40                  45
Val Asn Arg Glu Glu Ala Gln Ser Val Lys Ser Asn Ile Val Gly Met
    50                  55                  60
Met Gly Gln Trp Gln Ile Ser Gly Leu Ala Asn Gly Trp Val Ile Met
65                  70                  75                  80
Gly Pro Gly Tyr Asn Gly Glu Ile Lys Pro Gly Thr Ala Ser Asn Thr
                85                  90                  95
Trp Cys Tyr Pro Thr Asn Pro Val Thr Gly Glu Ile Pro Thr Leu Ser
            100                 105                 110
Ala Leu Glu Ile Pro Asp Gly Asp Glu Val Asp Val Gln Trp Arg Leu
        115                 120                 125
Val His Asp Ser Ala Asn Phe Ile Lys Pro Thr Ser Tyr Leu Ala His
    130                 135                 140
Tyr Leu Gly Tyr Ala Trp Val Gly Gly Asn His Ser Gln Tyr Val Gly
145                 150                 155                 160
Glu Asp Met Asp Val Thr Arg Asp Gly Asp Gly Trp Val Ile Arg Gly
                165                 170                 175
Asn Asn Asp Gly Gly Cys Asp Gly Tyr Arg Cys Gly Asp Lys Thr Ala
            180                 185                 190
Ile Lys Val Ser Asn Phe Ala Tyr Asn Leu Asp Pro Asp Ser Phe Lys
        195                 200                 205
His Gly Asp Val Thr Gln Ser Asp Arg Gln Leu Val Lys Thr Val Val
    210                 215                 220
Gly Trp Ala Val Asn Asp Ser Asp Thr Pro Gln Ser Gly Tyr Asp Val
225                 230                 235                 240
Thr Leu Arg Tyr Asp Thr Ala Thr Asn Trp Ser Lys Thr Asn Thr Tyr
                245                 250                 255
Gly Leu Ser Glu Lys Val Thr Thr Lys Asn Lys Phe Lys Trp Pro Leu
            260                 265                 270
Val Gly Glu Thr Glu Leu Ser Ile Glu Ile Ala Ala Asn Gln Ser Trp
        275                 280                 285
Ala Ser Gln Asn Gly Gly Ser Thr Thr Thr Ser Leu Ser Gln Ser Val
    290                 295                 300
Arg Pro Thr Val Pro Ala Arg Ser Lys Ile Pro Val Lys Ile Glu Leu
305                 310                 315                 320
Tyr Lys Ala Asp Ile Ser Tyr Pro Tyr Glu Phe Lys Ala Asp Val Ser
                325                 330                 335
Tyr Asp Leu Thr Leu Ser Gly Phe Leu Arg Trp Gly Gly Asn Ala Trp
            340                 345                 350
Tyr Thr His Pro Asp Asn Arg Pro Asn Trp Asn His Thr Phe Val Ile
        355                 360                 365
Gly Pro Tyr Lys Asp Lys Ala Ser Ser Ile Arg Tyr Gln Trp Asp Lys
    370                 375                 380
Arg Tyr Ile Pro Gly Glu Val Lys Trp Trp Asp Trp Asn Trp Thr Ile
385                 390                 395                 400
Gln Gln Asn Gly Leu Ser Thr Met Gln Asn Asn Leu Ala Arg Val Leu
                405                 410                 415
Arg Pro Val Arg Ala Gly Ile Thr Gly Asp Phe Ser Ala Glu Ser Gln
            420                 425                 430
Phe Ala Gly Asn Ile Glu Ile Gly Ala Pro Val Pro Leu Ala Ala Asp
        435                 440                 445
Ser His Pro Val Gly Leu Leu Ala Arg Ser Val Asp Gly Ala Gly Gln
    450                 455                 460
Gly Leu Arg Leu Glu Ile Pro Leu Asp Ala Gln Glu Leu Ser Gly Leu
465                 470                 475                 480

Gly Phe Asn Asn Val Ser Leu Ser Val Thr Pro Ala Ala Asn Gln His
                485                 490                 495
His His His His His
            500

<210> 40
<211> 1530
<212> DNA
<213> Artificial Sequence

<220>
<223> proaerolysin with an MMP2 cleavage site and a uPA
      cleavage  site in the activation sequence

<400> 40
atgcaaaaaa taaaactaac tggcttgtca ttaatcatat ccggcctgct gatggcacag 60
gcgcaagcgg cagagcccgt ctatccagac cagcttcgct tgttttcatt gggccaaggg 120
gtctgtggcg acaagtatcg ccccgtcaat cgagaagaag cccaaagcgt taaaagcaat 180
attgtcggca tgatggggca atggcaaata agcgggctgg ccaacggctg ggtcattatg 240
gggccgggtt ataacggtga aataaaacca gggacagcgt ccaatacctg gtgttatccg 300
accaatcctg ttaccggtga aataccgaca ctgtctgccc tcgagattcc agatggtgac 360
gaagtcgatg tgcagtggcg actggtacat gacagtgcga atttcatcaa accaaccagc 420
tatctggccc attacctcgg ttatgcctgg gtgggcggca atcacagcca atatgtcggc 480
gaagacatgg atgtgacccg tgatggcgac ggctgggtga tccgtggcaa caatgacggc 540
ggctgtgacg gctatcgctg tggtgacaag acggccatca aggtcagcaa cttcgcctat 600
aacctggatc ccgacagctt caagcatggc gatgtcaccc agtccgaccg ccagctggtc 660
aagactgtgg tgggctgggc ggtcaacgac agcgacaccc cccaatccgg ctatgacgtc 720
accctgcgct acgacacagc caccaactgg tccaagacca acacctatgg cctgagcgag 780
aaggtgacca ccaagaacaa gttcaagtgg ccactggtgg gggaaaccga actctccatc 840
gagattgctg ccaatcagtc ctgggcgtcc cagaacgggg gctcgaccac cacctccctg 900
tctcagtccg tgcgaccgac tgtgccggcc cgctccaaga tcccggtgaa gatagagctc 960
tacaaggccg acatctccta tccctatgag ttcaaggccg atgtcagcta tgacctgacc 1020
ctgagcggct tcctgcgctg gggcggcaac gcctggtata cccacccgga caaccgtccg 1080
aactggaacc acaccttcgt cataggtccg tacaaggaca aggcgagcag cattcggtac 1140
cagtgggaca agcgttacat cccgggtgaa gtgaagtggt gggactggaa ctggaccata 1200
cagcagaacg gtctgtctac catgcagaac aacctggcca gagtgctgcg cccggtgcgg 1260
gcggggatca ccggtgattt cagtgccgag agccagtttg ccggcaacat agagatcggt 1320
gctcccgtgc cgctcgcggc tgacagccat ccggtgggcc tgctcgctcg cggcggctca 1380
ggccgtagtg cgcaaagtgt ggacggcgct ggtcaaggcc tgaggctgga gatcccgctc 1440
gatgcgcaag agctctccgg gcttggcttc aacaacgtca gcctcagcgt gacccctgct 1500
gccaatcaac atcatcatca tcatcattaa                                  1530

<210> 41
<211> 509
<212> PRT
<213> Artificial Sequence

<220>
<223> proaerolysin with an MMP2 cleavage site and a uPA
      cleavage  site in the activation sequence

<400> 41
Met Gln Lys Ile Lys Leu Thr Gly Leu Ser Leu Ile Ile Ser Gly Leu
1               5                   10                  15
Leu Met Ala Gln Ala Gln Ala Ala Glu Pro Val Tyr Pro Asp Gln Leu
            20                  25                  30
Arg Leu Phe Ser Leu Gly Gln Gly Val Cys Gly Asp Lys Tyr Arg Pro
        35                  40                  45
Val Asn Arg Glu Glu Ala Gln Ser Val Lys Ser Asn Ile Val Gly Met
    50                  55                  60
Met Gly Gln Trp Gln Ile Ser Gly Leu Ala Asn Gly Trp Val Ile Met
65                  70                  75                  80
Gly Pro Gly Tyr Asn Gly Glu Ile Lys Pro Gly Thr Ala Ser Asn Thr
                85                  90                  95
Trp Cys Tyr Pro Thr Asn Pro Val Thr Gly Glu Ile Pro Thr Leu Ser
            100                 105                 110

Ala Leu Glu Ile Pro Asp Gly Asp Glu Val Asp Val Gln Trp Arg Leu
        115                 120                 125
Val His Asp Ser Ala Asn Phe Ile Lys Pro Thr Ser Tyr Leu Ala His
    130                 135                 140
Tyr Leu Gly Tyr Ala Trp Val Gly Gly Asn His Ser Gln Tyr Val Gly
145                 150                 155                 160
Glu Asp Met Asp Val Thr Arg Asp Gly Asp Gly Trp Val Ile Arg Gly
                165                 170                 175
Asn Asn Asp Gly Gly Cys Asp Gly Tyr Arg Cys Gly Asp Lys Thr Ala
            180                 185                 190
Ile Lys Val Ser Asn Phe Ala Tyr Asn Leu Asp Pro Asp Ser Phe Lys
        195                 200                 205
His Gly Asp Val Thr Gln Ser Asp Arg Gln Leu Val Lys Thr Val Val
    210                 215                 220
Gly Trp Ala Val Asn Asp Ser Asp Thr Pro Gln Ser Gly Tyr Asp Val
225                 230                 235                 240
Thr Leu Arg Tyr Asp Thr Ala Thr Asn Trp Ser Lys Thr Asn Thr Tyr
                245                 250                 255
Gly Leu Ser Glu Lys Val Thr Thr Lys Asn Lys Phe Lys Trp Pro Leu
            260                 265                 270
Val Gly Glu Thr Glu Leu Ser Ile Glu Ile Ala Ala Asn Gln Ser Trp
        275                 280                 285
Ala Ser Gln Asn Gly Gly Ser Thr Thr Thr Ser Leu Ser Gln Ser Val
    290                 295                 300
Arg Pro Thr Val Pro Ala Arg Ser Lys Ile Pro Val Lys Ile Glu Leu
305                 310                 315                 320
Tyr Lys Ala Asp Ile Ser Tyr Pro Tyr Glu Phe Lys Ala Asp Val Ser
                325                 330                 335
Tyr Asp Leu Thr Leu Ser Gly Phe Leu Arg Trp Gly Gly Asn Ala Trp
            340                 345                 350
Tyr Thr His Pro Asp Asn Arg Pro Asn Trp Asn His Thr Phe Val Ile
        355                 360                 365
Gly Pro Tyr Lys Asp Lys Ala Ser Ser Ile Arg Tyr Gln Trp Asp Lys
    370                 375                 380
Arg Tyr Ile Pro Gly Glu Val Lys Trp Trp Asp Trp Asn Trp Thr Ile
385                 390                 395                 400
Gln Gln Asn Gly Leu Ser Thr Met Gln Asn Asn Leu Ala Arg Val Leu
                405                 410                 415
Arg Pro Val Arg Ala Gly Ile Thr Gly Asp Phe Ser Ala Glu Ser Gln
            420                 425                 430
Phe Ala Gly Asn Ile Glu Ile Gly Ala Pro Val Pro Leu Ala Ala Asp
        435                 440                 445
Ser His Pro Val Gly Leu Leu Ala Arg Gly Gly Ser Gly Arg Ser Ala
    450                 455                 460
Gln Ser Val Asp Gly Ala Gly Gln Gly Leu Arg Leu Glu Ile Pro Leu
465                 470                 475                 480
Asp Ala Gln Glu Leu Ser Gly Leu Gly Phe Asn Asn Val Ser Leu Ser
                485                 490                 495
Val Thr Pro Ala Ala Asn Gln His His His His His His
            500                 505

<210> 42
<211> 1500
<212> DNA
<213> Artificial Sequence

<220>
<223> proaerolysin with R336A mutation to large lobe
      binding domain

<400> 42
atgcaaaaaa taaaactaac tggcttgtca ttaatcatat ccggcctgct gatggcacag 60
gcgcaagcgg cagagcccgt ctatccagac cagcttcgct tgttttcatt gggccaaggg 120
gtctgtggcg acaagtatcg ccccgtcaat cgagaagaag cccaaagcgt taaaagcaat 180
attgtcggca tgatggggca atggcaaata agcgggctgg ccaacggctg ggtcattatg 240
gggccgggtt ataacggtga aataaaacca gggacagcgt ccaatacctg gtgttatccg 300

accaatcctg ttaccggtga aataccgaca ctgtctgccc tcgagattcc agatggtgac 360
gaagtcgatg tgcagtggcg actggtacat gacagtgcga atttcatcaa accaaccagc 420
tatctggccc attacctcgg ttatgcctgg gtgggcggca atcacagcca atatgtcggc 480
gaagacatgg atgtgacccg tgatggcgac ggctgggtga tccgtggcaa caatgacggc 540
ggctgtgacg gctatcgctg tggtgacaag acggccatca aggtcagcaa cttcgcctat 600
aacctggatc ccgacagctt caagcatggc gatgtcaccc agtccgaccg ccagctggtc 660
aagactgtgg tgggctgggc ggtcaacgac agcgacaccc cccaatccgg ctatgacgtc 720
accctgcgct acgacacagc caccaactgg tccaagacca acacctatgg cctgagcgag 780
aaggtgacca ccaagaacaa gttcaagtgg ccactggtgg gggaaaccga actctccatc 840
gagattgctg ccaatcagtc ctgggcgtcc cagaacgggg gctcgaccac cacctccctg 900
tctcagtccg tgcgaccgac tgtgccggcc cgctccaaga tcccggtgaa gatagagctc 960
tacaaggccg acatctccta tccctatgag ttcaaggccg atgtcagcta tgacctgacc 1020
ctgagcggct tcctgcgctg gggcggcaac gcctggtata cccacccgga caacgcaccg 1080
aactggaacc acaccttcgt cataggtccg tacaaggaca aggcgagcag cattcggtac 1140
cagtgggaca agcgttacat cccgggtgaa gtgaagtggt gggactggaa ctggaccata 1200
cagcagaacg gtctgtctac catgcagaac aacctggcca gagtgctgcg cccggtgcgg 1260
gcggggatca ccggtgattt cagtgccgag agccagtttg ccggcaacat agagatcggt 1320
gctcccgtgc cgctcgcggc tgacagcaag gtgcgtcgtg ctcgcagtgt ggacggcgct 1380
ggtcaaggcc tgaggctgga gatcccgctc gatgcgcaag agctctccgg gcttggcttc 1440
aacaacgtca gcctcagcgt gacccctgct gccaatcaac atcatcatca tcatcattga 1500

<210> 43
<211> 499
<212> PRT
<213> Artificial Sequence

<220>
<223> proaerolysin with R336A mutation to large lobe
      binding domain

<400> 43
Met Gln Lys Ile Lys Leu Thr Gly Leu Ser Leu Ile Ile Ser Gly Leu
1               5                   10                  15
Leu Met Ala Gln Ala Gln Ala Ala Glu Pro Val Tyr Pro Asp Gln Leu
            20                  25                  30
Arg Leu Phe Ser Leu Gly Gln Gly Val Cys Gly Asp Lys Tyr Arg Pro
        35                  40                  45
Val Asn Arg Glu Glu Ala Gln Ser Val Lys Ser Asn Ile Val Gly Met
    50                  55                  60
Met Gly Gln Trp Gln Ile Ser Gly Leu Ala Asn Gly Trp Val Ile Met
65                  70                  75                  80
Gly Pro Gly Tyr Asn Gly Glu Ile Lys Pro Gly Thr Ala Ser Asn Thr
                85                  90                  95
Trp Cys Tyr Pro Thr Asn Pro Val Thr Gly Glu Ile Pro Thr Leu Ser
            100                 105                 110
Ala Leu Asp Ile Pro Asp Gly Asp Glu Val Asp Val Gln Trp Arg Leu
        115                 120                 125
Val His Asp Ser Ala Asn Phe Ile Lys Pro Thr Ser Tyr Leu Ala His
    130                 135                 140
Tyr Leu Gly Tyr Ala Trp Val Gly Gly Asn His Ser Gln Tyr Val Gly
145                 150                 155                 160
Glu Asp Met Asp Val Thr Arg Asp Gly Asp Gly Trp Val Ile Arg Gly
                165                 170                 175
Asn Asn Asp Gly Gly Cys Asp Gly Tyr Arg Cys Gly Asp Lys Thr Ala
            180                 185                 190
Ile Lys Val Ser Asn Phe Ala Tyr Asn Leu Asp Pro Asp Ser Phe Lys
        195                 200                 205
His Gly Asp Val Thr Gln Ser Asp Arg Gln Leu Val Lys Thr Val Val
    210                 215                 220
Gly Trp Ala Val Asn Asp Ser Asp Thr Pro Gln Ser Gly Tyr Asp Val
225                 230                 235                 240
Thr Leu Arg Tyr Asp Thr Ala Thr Asn Trp Ser Lys Thr Asn Thr Tyr
                245                 250                 255
Gly Leu Ser Glu Lys Val Thr Thr Lys Asn Lys Phe Lys Trp Pro Leu
            260                 265                 270
Val Gly Glu Thr Glu Leu Ser Ile Glu Ile Ala Ala Asn Gln Ser Trp

275                 280                 285
Ala Ser Gln Asn Gly Gly Ser Thr Thr Thr Ser Leu Ser Gln Ser Val
    290                 295                 300
Arg Pro Thr Val Pro Ala Arg Ser Lys Ile Pro Val Lys Ile Glu Leu
305                 310                 315                 320
Tyr Lys Ala Asp Ile Ser Tyr Pro Tyr Glu Phe Lys Ala Asp Val Ser
                325                 330                 335
Tyr Asp Leu Thr Leu Ser Gly Phe Leu Arg Trp Gly Gly Asn Ala Trp
            340                 345                 350
Tyr Thr His Pro Asp Asn Arg Pro Asn Trp Asn His Thr Phe Val Ile
        355                 360                 365
Gly Pro Tyr Lys Asp Lys Ala Ser Ser Ile Arg Tyr Gln Trp Asp Lys
    370                 375                 380
Arg Tyr Ile Pro Gly Glu Val Lys Trp Trp Asp Trp Asn Trp Thr Ile
385                 390                 395                 400
Gln Gln Asn Gly Leu Ser Thr Met Gln Asn Asn Leu Ala Arg Val Leu
                405                 410                 415
Arg Pro Val Arg Ala Gly Ile Thr Gly Asp Phe Ser Ala Glu Ser Gln
            420                 425                 430
Phe Ala Gly Asn Ile Glu Ile Gly Ala Pro Val Pro Leu Ala Ala Asp
        435                 440                 445
Ser Lys Val Arg Arg Ala Arg Ser Val Asp Gly Ala Gly Gln Gly Leu
    450                 455                 460
Arg Leu Glu Ile Pro Leu Asp Ala Gln Glu Leu Ser Gly Leu Gly Phe
465                 470                 475                 480
Asn Asn Val Ser Leu Ser Val Thr Pro Ala Ala Asn Gln His His His
                485                 490                 495
His His His

<210> 44
<211> 1842
<212> DNA
<213> Artificial Sequence

<220>
<223> proaerolysin with R336A mutation to large lobe
      binding domain, and AFAI as an artificial
      regulatory domain

<400> 44
atgaaaaaaa ccgctatcgc gatcgcagtt gcactggctg gtttcgctac cgttgcgcag 60
gccgtcttcg atgtgcagct gcaggcgtct ggaggaggcg tggtgcagcc tggggggtct 120
ctgagactct cctgtgcagc ccatgatccc atcttcgata agaatctcat gggctggggc 180
cgccaggctc caggaaagca gcgcgaatat gtcgcgacta ttagtggtaa tggtggaaca 240
aattatgcaa gctccgttga gggccgattc accatctcta gagacaacgc caagaaaacg 300
gtgtatctgc aaatgaacga cctgaaacct gaggacacgg ccgtctatta ctgtaactca 360
gcttttgcta tctggggcca gggcatccag gtcaccgtct cctcagtgca cgcagagccc 420
gtctatccag accagcttcg cttgttttca ttgggccaag gggtctgtgg cgacaagtat 480
cgccccgtca atcgagaaga agcccaaagc gttaaaagca atattgtcgg catgatgggg 540
caatggcaaa taagcgggct ggccaacggc tgggtcatta tggggccggg ttataacggt 600
gaaataaaac cagggacagc gtccaatacc tggtgttatc cgaccaatcc tgttaccggt 660
gaaataccga cactgtctgc cctcgagatt ccagatggtg acgaagtcga tgtgcagtgg 720
cgactggtac atgacagtgc gaatttcatc aaaccaacca gctatctggc ccattacctc 780
ggttatgcct gggtgggcgg caatcacagc caatatgtcg gcgaagacat ggatgtgacc 840
cgtgatggcg acggctgggt gatccgtggc aacaatgacg gcggctgtga cggctatcgc 900
tgtggtgaca agacggccat caaggtcagc aacttcgcct ataacctgga tcccgacagc 960
ttcaagcatg gcgatgtcac ccagtccgac cgccagctgg tcaagactgt ggtgggctgg 1020
gcggtcaacg acagcgacac cccccaatcc ggctatgacg tcaccctgcg ctacgacaca 1080
gccaccaact ggtccaagac caacacctat ggcctgagcg agaaggtgac caccaagaac 1140
aagttcaagt ggccactggt gggggaaacc gaactctcca tcgagattgc tgccaatcag 1200
tcctgggcgt cccagaacgg gggctcgacc accacctccc tgtctcagtc cgtgcgaccg 1260
actgtgccgg cccgctccaa gatcccggtg aagatagagc tctacaaggc cgacatctcc 1320
tatccctatg agttcaaggc cgatgtcagc tatgacctga ccctgagcgg cttcctgcgc 1380
tggggcggca acgcctggta tacccacccg gacaacgcac cgaactggaa ccacaccttc 1440
gtcataggtc cgtacaagga caaggcgagc agcattcggt accagtggga caagcgttac 1500

atcccgggtg aagtgaagtg gtgggactgg aactggacca tacagcagaa cggtctgtct 1560
accatgcaga acaacctggc cagagtgctg cgcccggtgc gggcggggat caccggtgat 1620
ttcagtgccg agagccagtt tgccggcaac atagagatcg gtgctcccgt gccgctcgcg 1680
gctgacagca aggtgcgtcg tgctcgcagt gtggacggcg ctggtcaagg cctgaggctg 1740
gagatcccgc tcgatgcgca agagctctcc gggcttggct tcaacaacgt cagcctcagc 1800
gtgacccctg ctgccaatca acatcatcat catcatcatt ga 1842

<210> 45
<211> 613
<212> PRT
<213> Artificial Sequence

<220>
<223> proaerolysin with R336A mutation to large lobe
      binding domain, and AFAI as an artificial
      regulatory domain

<400> 45
Met Lys Lys Thr Ala Ile Ala Ile Ala Val Ala Leu Ala Gly Phe Ala
1               5                   10                  15
Thr Val Ala Gln Ala Val Phe Asp Val Gln Leu Gln Ala Ser Gly Gly
            20                  25                  30
Gly Val Val Gln Pro Gly Gly Ser Leu Arg Leu Ser Cys Ala Ala His
        35                  40                  45
Asp Pro Ile Phe Asp Lys Asn Leu Met Gly Trp Gly Arg Gln Ala Pro
    50                  55                  60
Gly Lys Gln Arg Glu Tyr Val Ala Thr Ile Ser Gly Asn Gly Gly Thr
65                  70                  75                  80
Asn Tyr Ala Ser Ser Val Glu Gly Arg Phe Thr Ile Ser Arg Asp Asn
                85                  90                  95
Ala Lys Lys Thr Val Tyr Leu Gln Met Asn Asp Leu Lys Pro Glu Asp
            100                 105                 110
Thr Ala Val Tyr Tyr Cys Asn Ser Ala Phe Ala Ile Trp Gly Gln Gly
        115                 120                 125
Ile Gln Val Thr Val Ser Ser Val His Ala Glu Pro Val Tyr Pro Asp
    130                 135                 140
Gln Leu Arg Leu Phe Ser Leu Gly Gln Gly Val Cys Gly Asp Lys Tyr
145                 150                 155                 160
Arg Pro Val Asn Arg Glu Glu Ala Gln Ser Val Lys Ser Asn Ile Val
                165                 170                 175
Gly Met Met Gly Gln Trp Gln Ile Ser Gly Leu Ala Asn Gly Trp Val
            180                 185                 190
Ile Met Gly Pro Gly Tyr Asn Gly Glu Ile Lys Pro Gly Thr Ala Ser
        195                 200                 205
Asn Thr Trp Cys Tyr Pro Thr Asn Pro Val Thr Gly Glu Ile Pro Thr
    210                 215                 220
Leu Ser Ala Leu Glu Ile Pro Asp Gly Asp Glu Val Asp Val Gln Trp
225                 230                 235                 240
Arg Leu Val His Asp Ser Ala Asn Phe Ile Lys Pro Thr Ser Tyr Leu
                245                 250                 255
Ala His Tyr Leu Gly Tyr Ala Trp Val Gly Gly Asn His Ser Gln Tyr
            260                 265                 270
Val Gly Glu Asp Met Asp Val Thr Arg Asp Gly Asp Gly Trp Val Ile
        275                 280                 285
Arg Gly Asn Asn Asp Gly Gly Cys Asp Gly Tyr Arg Cys Gly Asp Lys
    290                 295                 300
Thr Ala Ile Lys Val Ser Asn Phe Ala Tyr Asn Leu Asp Pro Asp Ser
305                 310                 315                 320
Phe Lys His Gly Asp Val Thr Gln Ser Asp Arg Gln Leu Val Lys Thr
                325                 330                 335
Val Val Gly Trp Ala Val Asn Asp Ser Asp Thr Pro Gln Ser Gly Tyr
            340                 345                 350
Asp Val Thr Leu Arg Tyr Asp Thr Ala Thr Asn Trp Ser Lys Thr Asn
        355                 360                 365
Thr Tyr Gly Leu Ser Glu Lys Val Thr Thr Lys Asn Lys Phe Lys Trp
    370                 375                 380
Pro Leu Val Gly Glu Thr Glu Leu Ser Ile Glu Ile Ala Ala Asn Gln

385                 390                 395                 400
Ser Trp Ala Ser Gln Asn Gly Gly Ser Thr Thr Thr Ser Leu Ser Gln
                405                 410                 415
Ser Val Arg Pro Thr Val Pro Ala Arg Ser Lys Ile Pro Val Lys Ile
            420                 425                 430
Glu Leu Tyr Lys Ala Asp Ile Ser Tyr Pro Tyr Glu Phe Lys Ala Asp
        435                 440                 445
Val Ser Tyr Asp Leu Thr Leu Ser Gly Phe Leu Arg Trp Gly Gly Asn
    450                 455                 460
Ala Trp Tyr Thr His Pro Asp Asn Ala Pro Asn Trp Asn His Thr Phe
465                 470                 475                 480
Val Ile Gly Pro Tyr Lys Asp Lys Ala Ser Ser Ile Arg Tyr Gln Trp
                485                 490                 495
Asp Lys Arg Tyr Ile Pro Gly Glu Val Lys Trp Trp Asp Trp Asn Trp
            500                 505                 510
Thr Ile Gln Gln Asn Gly Leu Ser Thr Met Gln Asn Asn Leu Ala Arg
        515                 520                 525
Val Leu Arg Pro Val Arg Ala Gly Ile Thr Gly Asp Phe Ser Ala Glu
    530                 535                 540
Ser Gln Phe Ala Gly Asn Ile Glu Ile Gly Ala Pro Val Pro Leu Ala
545                 550                 555                 560
Ala Asp Ser Lys Val Arg Arg Ala Arg Ser Val Asp Gly Ala Gly Gln
                565                 570                 575
Gly Leu Arg Leu Glu Ile Pro Leu Asp Ala Gln Glu Leu Ser Gly Leu
            580                 585                 590
Gly Phe Asn Asn Val Ser Leu Ser Val Thr Pro Ala Ala Asn Gln His
        595                 600                 605
His His His His His
    610

<210> 46
<211> 6
<212> PRT
<213> Artificial Sequence

<220>
<223> Caspase 1 cleavage site

<400> 46
Tyr Val Ala Asp Ile Xaa
1               5

<210> 47
<211> 4
<212> PRT
<213> Artificial Sequence

<220>
<223> tissue-type plasminogen activator cleavage site

<400> 47
Phe Gly Arg Xaa
1

<210> 48
<211> 10
<212> PRT
<213> Artificial Sequence

<220>
<223> matrx metalloprotease 14 cleavage site

<400> 48
Gly Gly Pro Leu Gly Leu Tyr Ala Gly Gly

1               5                   10

<210> 49
<211> 7
<212> PRT
<213> Artificial Sequence

<220>
<223> human glandular kallikrein 2 cleavage site

<400> 49
Gly Lys Ala Phe Arg Arg Xaa
1               5

<210> 50
<211> 6
<212> PRT
<213> Artificial Sequence

<220>
<223> thrombin cleavage site

<400> 50
Leu Val Pro Arg Gly Ser
1               5

<210> 51
<211> 6
<212> PRT
<213> Artificial Sequence

<220>
<223> urokinase-type plasminagen activator cleavage site

<400> 51
Ser Gly Arg Ser Ala Gln
1               5

<210> 52
<211> 8
<212> PRT
<213> Artificial Sequence

<220>
<223> matrix metalloprotease 2 cleavage site

<400> 52
His Pro Val Gly Leu Leu Ala Arg
1               5

<210> 53
<211> 31
<212> DNA
<213> Artificial Sequence

<220>
<223> primer

<400> 53
atagacgggc tctgcgtgca ctgaggagac g                                    31

<210> 54

<211> 31
<212> DNA
<213> Artificial Sequence

<220>
<223> primer

<400> 54
gtctcctcag tgcacgcaga gcccgtctat c                                    31

<210> 55
<211> 27
<212> DNA
<213> Artificial Sequence

<220>
<223> primer

<400> 55
cacccggaca acgcaccgaa ctggaac                                         27

<210> 56
<211> 27
<212> DNA
<213> Artificial Sequence

<220>
<223> primer

<400> 56
gttccagttc ggtgcgttgt ccgggtg                                         27

<210> 57
<211> 4
<212> PRT
<213> Artificial Sequence

<220>
<223> Factor Xa cleavage site

<400> 57
Ile Glu Gly Arg
1

<210> 58
<211> 5
<212> PRT
<213> Artificial Sequence

<220>
<223> Enterokinase cleavage site

<400> 58
Asp Asp Asp Asp Lys
1               5

<210> 59
<211> 5
<212> PRT
<213> Artificial Sequence

<220>
<223> Thrombin cleavage site

<400> 59

Leu Val Pro Arg Gly
1               5

<210> 60
<211> 32
<212> DNA
<213> Artificial Sequence

<220>
<223> Primer

<400> 60
cacccggaca actgtccgaa ctggaaccac ac                                    32

<210> 61
<211> 34
<212> DNA
<213> Artificial Sequence

<220>
<223> Primer

<400> 61
ggttccagtt cggacagttg tccgggtggg taaa                                  34

## Description

### FIELD OF THE INVENTION

The present invention relates to the field of modified pore-forming protein toxins and their use to kill cancer cells.

### BACKGROUND

Many cytolytic proteins have been described (Lesieur et al Mol Membr Biol. 14:45064, 1997). These naturally occurring cytotoxic proteins include mammalian proteins such as perforin, and bacterial proteins such as aerolysin (produced by *Aeromonas hydrophila*), α-hemolysin (produced by *Staphylococcus aureus*), alpha toxin (produced by *Clostridium septicum*), and δ-toxin (produced by *Bacillus thuringiensis*), anthrax protective antigen, Vibrio cholerae VCC toxin, *Staphylococcus* leucocidins, LSL toxin from *Laetiporus sulphureus,* epsilon toxin from *Clostridium perfringens,* and hydralysins produced by *Cnidaria* spp

Some of these cytotoxic proteins are synthesized as inactive protoxins, for example proaerolysin and alpha toxin These protoxins contain discrete functionalities including a binding domain, which allows binding of the protoxin to a cell, a toxin domain, and either an N-terminal or a C-terminal inhibitory peptide domain that contains a protease cleavage site Cleavage of the inhibitory peptide domain at the protease cleavage site results in activation of the protoxin, leading to oligomerization of the cytotoxin and insertion into the plasma membrane, producing pores that lead to rapid cytolytic cell death (Rossjohn et al. J. Struct. Biol. 121:92-100,1998) Pore formation disrupts the cell membranes, and results in death of cells in all phases of the cell cycle, including non-proliferating cells (i.e. Go arrested) These cytotoxins are not specific in the type of cells they are able to kill, as their binding domains target molecules that are found on most cells, and they may be activated by proteases that are not cell-specific

Cancer is characterized by an increase in the number of abnormal, or neoplastic cells derived from a normal tissue which proliferate to form a tumor mass, the invasion of adjacent tissues by these neoplastic tumor cells, and the generation of malignant cells which eventually spread via the blood or lymphatic system to regional lymph nodes and to distant sites via a process called metastasis. Many strategies for developing therapeutics for the treatment of cancer have focused on taking advantage of the differences in gene expression between normal cells and cancer cells, and targeting cancer cells using molecular markers that are specific to cancer cells.

Cytolytic pore-forming proteins or modified versions of these proteins have been proposed as potential therapeutics for the treatment of cancer. For example, U.S. Patent No. 5,777,078 describes pore-forming agents that are activated at the surface of a cell by a number of conditions, including proteolysis, to lyse the cell. These pore-forming agents can be used generally to destroy unwanted cells associated with a pathological condition in an animal. WO 98/020135 describes methods and compositions relating to *Pseudomonas* exotoxin proproteins modified for selective toxicity. The exotoxin is modified to be activated by a desired protease by insertion of a protease activatable sequence in the proprotein. In one example the exotoxin is modified to insert a prostate specific antigen (PSA) cleavage site for the purpose of targeting and killing prostate cancer cells. In another example, the exotoxin is modified to include a urokinase activatable sequence U.S. Patent Application No 2004/0235095 describes the use of modified cytolytic proteins, in particular proaerolysin, for the treatment of prostate cancer. The cytolytic proteins can be modified to include a prostate-specific cleavage site, and/or a prostate-specific binding domain and can be used to selectively target and kill prostate cancer cells.

Modification of cytolytic peptides to include an inhibitory or targeting domain have been described. U.S. Patent Application No 2002/0045736 describes the modification of cytotoxic peptides, including proaerolysin and homologs, via attachment of an inhibitory molecule that acts to inhibit formation of the active conformation of the cytolytic peptide. This application also describes attachment of targeting molecules to the cytolytic peptide, or molecules that can act as both a targeting molecule and an inhibitory molecule These inhibitory and/or targeting molecules are attached to the cytolytic peptide via a linker that may or may not be cleavable. U.S Patent No. 4,867,973 describes antibody therapeutic enzyme conjugates in which the antibody is linked to a therapeutic agent via a protease cleavable linker The therapeutic agent may include toxins or fragments of toxins. In addition, International Patent Application No PCT/US94/04016 (WO 94/25616) describes a chimeric compound active at a cell surface having a delivery component, for example, an antibody or other ligands binding specifically to a target cell that is linked to a pore-forming component such as aerolysin. This application also describes modifications designed to inactivate the pore-forming agent, which can then be specifically activated by a cell-associated substance or condition. Finally, International Patent Application No PCT/CA2004/000309 (WO 2004/078097) describes peptides including aerolysin or an aerolysin homolog, linked to an agent that specifically binds to a lung cancer cell, as well as nucleic acids that encode such peptides Methods of using these peptides and nucleic acid sequences to treat lung cancer are also described.

This background information is provided for the purpose of making known information believed by the applicant to be of possible relevance to the present invention No admission is necessarily intended, nor should be construed, that any of the preceding information constitutes prior art against the present invention.

### SUMMARY OF THE INVENTION

An object of the present invention is to provide modified pore forming protein toxins and use thereof In accordance with an aspect of the present invention, there is provided a broad-spectrum anti-cancer agent comprising a modified pore forming protein toxin, said modified pore forming protein toxin derived from a naturally occurring aerolysin-related pore forming protein and comprising a modified activation sequence in which a native protease cleavage site has been functionally deleted and replaced with: one or more general cleavage sites, each cleavable by an enzyme associated with a plurality of cancers, or two or more specific cleavage sites, each cleavable by an enzyme associated with the presence of a specific cancer, wherein cleavage of said modified activation sequence provides an activated pore forming protein toxin capable of killing cancer cells

In accordance with another aspect of the invention, there is provided an isolated polynucleotide encoding a broad-spectrum anti-cancer agent according to the present invention.

In accordance with another aspect of the invention, there is provided a vector comprising the polynucleotide encoding a broad-spectrum anti-cancer agent according to the present invention, wherein the polynucleotide is operatively linked to one or more expression control sequences.

In accordance with another aspect of the invention, there is provided a host cell comprising the vector comprising a polynucleotide encoding the broad-spectrum anti-cancer agent according to the present invention.

In accordance with a further aspect of the invention, there is provided a modified pore forming protein toxin derived from proaerolysin and comprising a modified activation sequence in which a native protease cleavage site has been functionally deleted and replaced with one or more general cleavage sites cleavable by urokinase-type plasminogen activator or matrix metalloprotease 2, said modified pore forming protein toxin comprising an amino acid sequence substantially identical to the sequence as set forth in any one of SEQ ID NOs: 21, 23, 25, 39, or 41.

In accordance with still another aspect of the invention, there is provided a pharmaceutical composition comprising a broad-spectrum anti-cancer agent according to the present invention

In accordance with another aspect of the invention, there is provided a pharmaceutical composition comprising the isolated polynucleotide encoding a broad-spectrum anti-cancer agent according to the present invention

In accordance with another aspect of the invention, there is provided a pharmaceutical composition comprising a modified pore forming protein toxin according to the present invention

In accordance with yet another aspect of the invention, there is provided a broad-spectrum anti-cancer agent according to the present invention, for use in decreasing the size of a tumor in a subject.

In accordance with another aspect of the invention, there is provided a broad-spectrum anti-cancer agent according to the present invention, for use in the treatment of cancer in a subject.

In accordance with anther aspect of the invention, there is provided a use of a modified pore forming protein toxin in the preparation of a medicament for decreasing the size of a tumor in a subject, said modified pore forming protein toxin derived from a naturally occurring aerolysin-related pore forming protein and comprising a modified activation sequence in which a native protease cleavage site has been functionally deleted and replaced with: one or more general cleavage sites, each cleavable by an enzyme associated with a plurality of cancers, or two or more specific cleavage sites each cleavable by an enzyme associated with the presence of a specific cancer, wherein cleavage of said modified activation sequence provides an activated pore forming protein toxin capable of killing cancer cells

In accordance with another aspect of' the invention, there is provided a use of a modified pore forming protein toxin in the preparation of a medicament for the treatment of cancer in a subject, said modified pore forming protein toxin derived from a naturally occurring aerolysin-related pore forming protein and comprising a modified activation sequence in which a native protease cleavage site has been functionally deleted and replaced with: one or more general cleavage sites each cleavable by an enzyme associated with a plurality of cancers, or two or more specific cleavage sites each cleavable by an enzyme associated with the presence of a specific cancer, wherein cleavage of said modified activation sequence provides an activated pore forming protein toxin capable of killing cancer cells

In accordance with another aspect of the invention, there is provided a method of decreasing the size of a tumor comprising administering to a subject having cancer an effective amount of a broad-spectrum anti-cancer agent according to the present invention.

In accordance with another aspect of the invention, there is provided a method of treating cancer comprising administering to a subject having cancer an effective amount of' a broad-spectrum anti-cancer agent according to the present invention.

In accordance with another aspect of the invention, there is provided a method of preparing a broad-spectrum anti-cancer agent, said method comprising: providing a native pore forming protein toxin wherein said native pore forming protein toxin is proaerolysin or *Clostridium septicum* alpha toxin; and modifying the activation sequence of said native pore forming protein toxin such that a native protease cleavage site is functionally deleted and replaced by one or more general cleavage sites each cleavable by an enzyme associated with a plurality of cancers, or by two or more specific cleavage sites each cleavable by an enzyme associated with the presence of a specific cancer, wherein cleavage of said modified activation sequence provides an activated pore forming protein toxin capable of killing cancer cells

### BRIEF DESCRIPTION OF THE FIGURES

**Figure 1** depicts the nucleotide sequence of proaerolysin from *Aeromonas hydrophila* **(SEQ** ID NO:1).
**Figure 2** depicts the amino acid sequence of proaerolysin from *Aeromonas hydrophila* (SEQ ID NO:2).
**Figure 3** depicts the nucleotide sequence of preproaerolysin from *Aeromonas hydrophila,* including the signal sequence (SEQ ID NO:3).
**Figure 4** depicts the amino acid sequence of preproaerolysin from *Aeromanas hydrophila,* including the signal sequence (underlined) (SEQ ID NO:4).
**Figure 5** depicts the nucleotide sequence of alpha toxin from *Clostridium septicum* (SEQ ID NO:5)
**Figure 6** depicts the amino acid sequence of alpha toxin from *Clostridium septicum* (SEQ ID NO:6).
**Figure 7** depicts the nucleotide sequence of alpha toxin from *Clostridium septicum,* including the signal sequence (underlined) (SEQ ID NO:7)
**Figure 8** depicts the amino acid sequence of alpha toxin from *Clostridium septicum,* including the signal sequence (underlined). (SEQ ID NO:8)
**Figure 9** depicts the amino acid sequence of the AFAI antibody fragment (SEQ ID NO:9).
**Figure 10** depicts the nucleotide sequence of' a modified proaerolysin (MPPT1; SEQ ID NO:20) in accordance with one embodiment of the present invention having a modified activation sequence comprising a uPA cleavage site and further comprising a His-tag
**Figure 11** depicts the amino acid sequence of a modified proaerolysin (MPPT1; SEQ ID NO:21) in accordance with one embodiment of the present invention having a modified activation sequence comprising a uPA cleavage site and further comprising a His-tag.
**Figure 12** depicts the nucleotide sequence of a modified proaerolysin (MPPT2; SEQ ID NO:22) in accordance with one embodiment of the present invention having a modified activation sequence comprising a uPA cleavage site, an AFAI targeting unit and further comprising a His-tag.
**Figure 13** depicts the nucleotide sequence of a modified proaerolysin (MPPT3; SEQ ID NO:24) in accordance with one embodiment of the present invention having a modified activation sequence comprising a uPA cleavage site, an AFAI targeting unit attached via a linker comprising a uPA cleavage site and further comprising a His-tag.
**Figure 14** depicts the amino acid sequence of a modified proaerolysin (MPPT2; SEQ ID NO:23) in accordance with one embodiment of the present invention having a modified activation sequence comprising a uPA cleavage site, an AFAI targeting unit and further comprising a His-tag
**Figure 15** depicts the amino acid sequence of a modified proaerolysin (MPPT3; SEQ ID NO:25) in accordance with one embodiment of the present invention having a modified activation sequence comprising a uPA cleavage site, an AFAI targeting unit attached via a linker comprising a uPA cleavage site and further comprising a His-tag.
**Figure 16** depicts an SDS-PAGE gel showing the results of digestion by uPA of a modified proaerolysin (MPPT1) in accordance with one embodiment of the present invention.
**Figure 17** depicts a SDS-PAGE gel showing the results of' digestion of two modified proaerolysins MPPT2 and MPPT3 by uPA
**Figure 18** depicts the toxicity of the modified proaerolysins MPPT1, MPPT2, and MPPT3 against A549 cells.
**Figure 19** depicts the effect of *in vivo* administration of the modified proaerolysins MPPT1 and MPPT2 on body weight in mice.
**Figure 20** depicts the effect of the modified proaerolysins MPPT1 and MPPT2 on rate of tumor growth in mice.
**Figure 21** depicts an SDS-PAGE gel showing the cleavage of the modified proaerolysin MPPT1 by uPA.
**Figure 22** depicts the ability of the modified proaerolysin MPPT1 to kill HeLa human cervical cancer cells.
**Figure 23** depicts the ability of the modified proaerolysin MPPT1 to kill A2058 human melanoma cells
**Figure 24** depicts the ability of the modified proaerolysin MPPT1 to kill EL4 mouse lymphoma cells.
**Figure 25** depicts the nucleotide sequence of a modified proaerolysin (MPPT4; SEQ ID NO:38) in accordance with one embodiment of the present invention having a modified activation sequence comprising a MMP2 cleavage site
**Figure 26** depicts the amino acid sequence of a modified proaerolysin (MPPT4; SEQ ID NO:39) in accordance with one embodiment of the present invention having a modified activation sequence comprising a MMP2 cleavage site.
**Figure 27** depicts a silver stained SDS-PAGE gel showing the cleavage of proaerolysin and the modified proaerolysin MPPT4 by MMP2
**Figure 28** depicts a Western blot showing the cleavage of proaerolysin and the modified proaerolysin MPPT4 by MMP2.
**Figure 29** depicts cell killing curves for HT 1080 human fibrosarcoma cells treated with proaerolysin or the modified proaerolysin MPPT4.
**Figure 30** depicts cell killing curves for EL4 mouse lymphoma cells treated with proaerolysin or the modified proaerolysin MPPT4
**Figure 31** depicts the nucleotide sequence of a modified proaerolysin (MPPT5; SEQ ID NO:40) in accordance with one embodiment of the present invention having a modified activation sequence comprising a uPA cleavage site and a MMP2 cleavage site
**Figure 32** depicts the amino acid sequence of a modified proaerolysin (MPPT5; SEQ ID NO:41) in accordance with one embodiment of the present invention having a modified activation sequence comprising a uPA cleavage site and a MMP2 cleavage site.
**Figure 33** depicts an SDS-PAGE gel showing cleavage of the modified proaerolysin MPPT5 by MMP2 and uPA.
**Figure 34** depicts cell killing curves for HT 1080 human fibrosarcoma cells treated with proaerolysin or the modified proaerolysin MPPT5.
**Figure 35** depicts cell killing curves for HeLa human cervical cancer cells treated with proaerolysin or the modified proaerolysin MPPT5.
**Figure 36** depicts cell killing curves for A2058 human melanoma cells treated with proaerolysin or the modified proaerolysin MPPT5.
**Figure 37** depicts cell killing curves for EL4 mouse lymphoma cells treated with proaerolysin or the modified proaerolysin MPPT5.
**Figure 38** depicts the nucleotide sequence of a mutant proaerolysin (PA-R336A; SEQ ID NO:42), which comprises an arginine to alanine substitution at amino acid 336 in the large binding domain of proaerolysin
**Figure 39** depicts the amino acid sequence of' a mutant proaerolysin (PA-R335A; SEQ ID NO:43), which comprises an arginine to alanine substitution at amino acid 336 in the large binding domain of proaerolysin.
**Figure 40** depicts the ability of the mutant proaerolysin PA-R336A to bind to EL4 mouse lymphoma cells.
**Figure 41** depicts the ability of the mutant proaerolysin PA-R336A to kill EL4 mouse lymphoma cells
**Figure 42** depicts the nucleotide sequence of a mutant proaerolysin (AFA-PA-R336A; SEQ ID NO:44), which comprises an arginine to alanine substitution at amino acid 336 in the large binding domain of proaerolysin and an AFAI targeting unit
**Figure 43** depicts the amino acid sequence of a mutant proaerolysin (AFA-PA-R336A; SEQ ID NO:45), which comprises an arginine to alanine substitution at amino acid 336 in the larger binding domain of proaerolysin and an AFAI targeting unit.
**Figure 44** depicts the ability of the mutant proaerolysin AFA-PA-R336A to kill (A) EL4 mouse lymphoma cells, and (B) A549 human lung cancer cells.

### DETAILED DESCRIPTION OF THE INVENTION

The present invention provides modified pore-forming protein toxins (MPPTs) capable of killing cancer cells. The MPPTs of the present invention are derived from naturally-occurring pore-forming protein toxins (nPPTs) such as aerolysin and aerolysin-related toxins, and comprise a modified activation sequence that permits activation of the MPPTs in a variety of different cancer types Thus, in contrast to pore-forming molecules such as those described in International Patent Application No. PCT/US02/27061 (WO 03/018611) which have been engineered to selectively target a specific type of cancer, the MPPTs of the present invention are capable of acting as "broad spectrum" anti-cancer agents, The modified activation sequence of the MPPTs comprises a functional deletion of a native protease cleavage site and addition of one or more cleavage sites, each cleavable by an enzyme which is associated with a plurality of cancer types (a general activating agent) or a plurality of' cleavage sites, each of which is cleavable by an enzyme that is associated with a specific type of cancer (a specific activating agent)

The MPPTs according to the present invention can optionally further comprise one or more additional modifications which allow the MPPTs to be "tailored" for selective activation in a specific cancer. These modifications include, but are not limited to, the addition of an artificial regulatory domain capable of either targeting the MPPT to a specific type of cell, and/or inhibiting the activity of the MPPT in such a way that inhibition of the MPPT is released at a target cell, as well as modifications to the cell binding domain(s) of the MPPTs to decrease non-selective binding of' the MPPTs

Thus, the MPPTs of the present invention are designed to act as generally effective anti-cancer cell therapeutics that can be broadly applied to kill numerous types of cancer cells

Further refinement of these broad spectrum molecules to target them to kill a selected type of cancer cells however, is also encompassed by the present invention. The MPPTs, therefore, are effective in decreasing the growth of a variety of tumors, and may be used in the treatment of various types of cancer.

### Definitions

Unless defined otherwise, all technical and scientific terms used herein have the same meaning as commonly understood by one of ordinary skill in the art to which this invention belongs

Generally, the nomenclature used herein and the laboratory procedures in drug discovery, cell culture, molecular genetics, diagnostics, amino acid and nucleic acid chemistry, and sugar chemistry described below are those well known and commonly employed in the art. Standard techniques are typically used for signal detection, recombinant nucleic acid methods, polynucleotide synthesis, and microbial culture and transformation (e.g, electroporation, lipofection).

The techniques and procedures are generally performed according to conventional methods in the art and various general references (see generally, Sambrook et al Molecular Cloning: A Laboratory Manual, 2d ed. (1989) Cold Spring Harbor Laboratory Press, Cold Spring Harbor, NY) Standard techniques are used for chemical syntheses, chemical analyses, and biological assays. As employed throughout the disclosure, the following terms, unless otherwise indicated, shall be understood to have the following meanings:

"Binding pair" refers to two moieties (e.g, chemical or biochemical) that have an affinity for one another. Examples of binding pairs include homo-dimers, hetero-dimers, antigen/antibodies, lectin/avidin, target polynucleotide/probe, oligonucleotide, antibody/anti-antibody, receptor/ligand, enzyme/ligand and the like. "One member of' a binding pair" refers to one moiety of the pair, such as an antigen or ligand.

"Isolated polynucleotide" refers to a polynucleotide of genomic, cDNA, or synthetic origin or some combination there of, which (1) is not associated with the cell in which the "isolated polynucleotide" is found in nature, or (2) is operably linked to a polynucleotide which it is not linked to in nature

"Operably linked" refers to a juxtaposition wherein the components so described are in a relationship permitting them to function in their intended manner A control sequence "operably linked" to a coding sequence is ligated in such a way that expression of the coding sequence is achieved under conditions compatible with the control sequences.

"Control sequence" refers to polynucleotide sequences which are necessary to effect the expression of coding and non-coding sequences to which they are ligated The nature of' such control sequences differs depending upon the host organism; in prokaryotes, such control sequences generally include one or more of: promoter, ribosomal binding site, and transcription termination sequence; in eukaryotes, generally, such control sequences include one or more of: promoters and transcription termination sequences The term "control sequences" is intended to include, at a minimum, components whose presence can influence expression, and can also include additional components whose presence is advantageous, for example, leader sequences and fusion partner sequences.

"Polynucleotide" refers to a polymeric form of nucleotides of at least 10 nucleotides in length, either ribonucleotides or deoxynucleotides or a modified form of either type of nucleotide.

The term includes single and double stranded forms of DNA or RNA

The terms "label" or "labeled" refer to incorporation of a detectable marker, e.g., by incorporation of a radiolabeled amino acid or attachment to a polypeptide of biotinyl moieties that can be detected by marked avidin (e.g, streptavidin containing a fluorescent marker or enzymatic activity that can be detected by optical or colorimetric methods) Various methods of labeling polypeptides and glycoproteins are known in the art and may be used Examples of labels for polypeptides include, but are not limited to, the following: radioisotopes (e.g., ³H, ¹⁴C, ³⁵S,¹²⁵I, ¹³¹I), fluorescent labels (e.g., FITC, rhodamine, lanthanide phosphors), enzymatic labels (or reporter genes) (eg, horseradish peroxidase, β-galactosidase, β-latamase, luciferase, alkaline phosphatase), chemiluminescent, biotinyl groups, predetermined polypeptide epitopes recognized by a secondary reporter (e.g., leucine zipper pair sequences, binding sites for secondary antibodies, metal binding domains, epitope tags). In some embodiments, labels are attached by linker arms of various lengths to reduce potential steric hindrance

The term "gene," as used herein, refers to a segment of nucleic acid that encodes an individual protein or RNA (also referred to as a "coding sequence" or "coding region") together with associated regulatory regions such as promoters, operators, terminators and the like, that may be located upstream or downstream of the coding sequence.

The term "selectively hybridize," as used herein, refers to the ability of a nucleic acid to bind detectably and specifically to a second nucleic acid Polynucleotides selectively hybridize to target nucleic acid strands under hybridization and wash conditions that minimize appreciable amounts of detectable binding to non-specific nucleic acids. High stringency conditions can be used to achieve selective hybridization conditions as known in the art and discussed herein Typically, hybridization and washing conditions are performed at high stringency according to conventional hybridization procedures Washing conditions are typically 1-3 x SSC, 0.1-1% SDS, 50-70°C with a change of wash solution after about 5-30 minutes

The term "corresponding to" or "corresponds to" indicates that a polynucleotide sequence is identical to all or a portion of a reference polynucleotide sequence. In contradistinction, the term "complementary to" is used herein to indicate that the polynucleotide sequence is identical to all or a portion of the complementary strand of a reference polynucleotide sequence. For illustration, the nucleotide sequence "TATAC" corresponds to a reference sequence "TATAC" and is complementary to a reference sequence "GTATA."

The following terms are used herein to describe the sequence relationships between two or more polynucleotides or two or more polypeptides: "reference sequence," "window of comparison," "sequence identity," "percent sequence identity," and "substantial identity." A "reference sequence" is a defined sequence used as a basis for a sequence comparison; a reference sequence may be a subset of a larger sequence, for example, as a segment of a full-length cDNA, gene or protein sequence, or may comprise a complete cDNA, gene or protein sequence. Generally, a reference polynucleotide sequence is at least 20 nucleotides in length, and often at least 50 nucleotides in length A reference polypeptide sequence is generally at least 7 amino acids in length and often at least 17 amino acids in length

A "window of comparison", as used herein, refers to a conceptual segment of the reference sequence of at least 15 contiguous nucleotide positions or at least 5 contiguous amino acid positions over which a candidate sequence may be compared to the reference sequence and wherein the portion of the candidate sequence in the window of comparison may comprise additions or deletions (*i.e*. gaps) of 20 percent or less as compared to the reference sequence (which does not comprise additions or deletions) for optimal alignment of the two sequences. The present invention contemplates various lengths for the window of comparison, up to and including the full length of either the reference or candidate sequence. Optimal alignment of sequences for aligning a comparison window may be conducted using the local homology algorithm of Smith and Waterman (Adv. Appl. Math. (1981) 2:482), the homology alignment algorithm of Needleman and Wunsch (J. Mol Biol. (1970) 48:443), the search for similarity method of Pearson and Lipman (Proc. Natl Acad Sci (U.S.A.) (1988) 85:2444), using computerised implementations of these algorithms (such as GAP, BESTFIT, FASTA, and TFASTA in the Wisconsin Genetics Software Package Release 7.0, Genetics Computer Group, 573 Science Dr., Madison, WI), using publicly available computer software such as ALIGN or Megalign (DNASTAR), or by inspection The best alignment (*i.e*. resulting in the highest percentage of identity over the comparison window) is then selected.

The term "sequence identity" means that two polynucleotide or polypeptide sequences are identical (*i.e*. on a nucleotide-by-nucleotide or amino acid-by-amino acid basis) over the window of comparison.

The term "percent (%) sequence identity," as used herein with respect to a reference sequence is defined as the percentage of nucleotide of amino acid residues in a candidate sequence that are identical with the residues in the reference polypeptide sequence over the window of comparison after optimal alignment of the sequences and introducing gaps, if necessary, to achieve the maximum percent sequence identity, without considering any conservative substitutions as part of the sequence identity.

The term "substantial identity" as used herein denotes a characteristic of a polynucleotide or polypeptide sequence, wherein the polynucleotide or polypeptide comprises a sequence that has at least 50% sequence identity as compared to a reference sequence over the window of comparison. Polynucleotide and polypeptide sequences with at least 60% sequence identity, at least 70% sequence identity, at least 80% sequence identity, and at least 90% sequence identity as compared to a reference sequence over the window of comparison are also considered to have substantial identity with the reference sequence

The terms "therapy" and "treatment," as used interchangeably herein, refer to an intervention performed with the intention of improving a subject's status The improvement can be subjective or objective and is related to ameliorating the symptoms associated with, preventing the development of, or altering the pathology of a disease or disorder being treated Thus, the terms therapy and treatment are used in the broadest sense, and include the prevention (prophylaxis), moderation, reduction, and curing of a disease or disorder at various stages Preventing deterioration of a subject's status is also encompassed by the term. Subjects in need of therapy/treatment thus include those already having the disease or disorder as well as those prone to, or at risk of developing, the disease or disorder and those in whom the disease or disorder is to be prevented.

The term "ameliorate" includes the arrest, prevention, decrease, or improvement in one or more the symptoms, signs, and features of the disease or disorder being treated, both temporary and long-term

The term "subject" or "patient" as used herein refers to an animal in need of treatment

The term "animal," as used herein, refers to both human and non-human animals, including, but not limited to, mammals, birds and fish.

Administration of the compounds of the invention "in combination with" one or more further therapeutic agents, is intended to include simultaneous (concurrent) administration and consecutive administration Consecutive administration is intended to encompass administration of' the therapeutic agent(s) and the compound(s) of the invention to the subject in various orders and via various routes.

The term "inhibit," as used herein, means to decrease, reduce, slow-down or prevent

The term "polypeptide" is used herein as a generic term to refer to an amino acid sequence of at least 20 amino acids in length that can be a wild-type (naturally-occurring) protein sequence, a fragment of a wild-type protein sequence, a variant of a wild-type protein sequence, a derivative of a wild-type protein sequence, or an analogue of a wild-type protein sequence. Hence, native protein sequences and fragments, variants, derivatives and analogues of native protein sequences, as defined herein, are considered to be species of the polypeptide genus.

The term "isolated polypeptide," as used herein, refers to a polypeptide which by virtue of' its origin is not associated with other polypeptides with which it is normally associated with in nature, and/or is isolated from the cell in which it normally occurs and/or is free of other polypeptides from the same cellular source and/or is expressed by a cell from a different species, and/or does not occur in nature.

"Polypeptide fragment" refers to a polypeptide that has an amino-terminal and/or carboxy-terminal deletion, in which the remaining amino acid sequence is usually identical to the corresponding positions in the naturally-occurring sequence Fragments typically are at least 5, 6, 8 or 10 amino acids long, at least 14 amino acids long, at least 20 amino acids long, at least 50 amino acids long, or at least 70 amino acids long.

"Naturally-occurring," or "native" as used herein, as applied to an object, refers to the fact that an object can be found in nature. For example, a polypeptide or polynucleotide sequence that is present in an organism (including viruses) that can be isolated from a source in nature and which has not been intentionally modified by man in the laboratory is naturally-occurring.

The term "activity" as used with respect to a "pore-forming activity" or "activity of modified pore-forming protein toxins" refers to the ability of a naturally occurring pore-forming protein toxin or a modified pore-forming protein toxin to exhibit one on more of: the ability to bind to a cell, the ability to be activated by protease cleavage, the ability to oligomerize, or the ability to insert into or form pores in a cell membrane.

The term "general activating agent" refers to an enzyme, the presence of which is associated with a plurality of different cancer types By "associated with" is meant that the expression of the enzyme is up-regulated in a cancer cell when compared to a normal cell, or that the enzyme is localized to cancer cells as compared to normal cells, or that the enzyme is produced and/or activated by cells associated with cancerous tissue or cells. In this context, the terms "plurality" and "variety" mean at least two different cancer types. By "different cancer types" is meant cancers originating in different tissues (for example, lung cancer and breast cancer), or in different cells within a tissue (for example lymphoma and leukemia, or large cell, alveolar cell, and small cell lung cancers). In one embodiment, the term "general activating agent" refers to an agent, the presence of which is associated with cells of at least 3 different cancer types In another embodiment, the term "general activating agent" refers to an agent, the presence of which is associated with cells of at least 4 different cancer types. In a further embodiment, the term "general activating agent" refers to an agent, the presence of which is associated with cells of' at least 5 different cancer types In yet another embodiment, the term "general activating agent" refers to an agent, the presence of' which is associated with cells of at least 8 different cancer types.

The term "specific activating agent" refers to an enzyme, the presence of which is associated with a specific type of cancer. By "specific type of cancer" is meant a cancer originating in one tissue (for example breast tissue, lung tissue, or colon tissue), or involving one cell type in a tissue (for example lymphoma, or non-small cell lung cancer cells) or originating in specific cells within a tissue (for example lymphoma and leukemia cells, or large cell, alveolar cell, and small cell lung cancer cells).

The term "mutation," as used herein, refers to a deletion, insertion, substitution, inversion, or combination thereof, of' one or more nucleotides in a polynucleotide sequence when compared to the naturally occurring polynucleotide sequence, or a deletion, insertion, substitution or combination thereof of one or more amino acids in a polypeptide sequence. The term "cleavage site" as used herein, refers to a sequence of amino acids, nucleotides, sugars or modified versions thereof, which is recognized and selectively cleaved by either a general or specific activating agent

The term "activation sequence" as used herein, refers to a flexible loop structure in a pore-forming protein toxin that comprises an amino acid sequence that can be cleaved by an appropriate protease, resulting in activation of the pore-forming ability of the protein toxin

The term "amino acid residue," as used herein, encompasses both naturally-occurring amino acids and non-naturally-occurring amino acids. Examples of non-naturally occurring amino acids include, but are not limited to, D-amino acids (*i.e*. an amino acid of an opposite chirality to the naturally-occurring form), N-α-methyl amino acids, C-α-methyl amino acids, β-methyl amino acids and D- or L-β-amino acids. Other non-naturally occurring amino acids include, for example, β-alanine (β-Ala), norleucine (Nle), norvaline (Nva), homoarginine (Har), 4-aminobutyric acid (y-Abu), 2-aminoisobutyric acid (Aib), 6-aminohexanoic acid (ε-Ahx), ornithine (orn), sarcosine, α-amino isobutyric acid, 3-aminopropionic acid, 2,3-diaminopropionic acid (2,3-diaP), D- or L-phenylglycine, D-(trifluoromethyl)-phenylalanine, and D-p-fluorophenylalanine

As used herein, "peptide bond" can be a naturally-occurring peptide bond or a non-naturally occurring (*i.e*. modified) peptide bond Examples of suitable modified peptide bonds are well known in the art and include, but are not limited to, -CH₂NH-, -CH₂S-, -CH₂CH₂-, -CH=CH-(*cis* or *trans*), -COCH₂-, -CH(OH)CH₂-, -CH₂SO-, -CS-NH- and -NH-CO- (*i.e.* a reversed peptide bond) (see, for example, Spatola, Vega Data Vol, 1, Issue 3, (1983); Spatola, in Chemistry and Biochemistry of Amino Acids Peptides and Proteins, Weinstein, ed., Marcel Dekker, New York, p. 267 (1983); Morley, J. S, Trends Pharm Sci. pp. 463-468 (1980); Hudson et al., Int. J. Pept. Prot. Res. 14:177-185 (1979); Spatola et al., Life Sci. 38:1243-1249 (1986); Hann, J. Chem Soc, Perkin Trans. I 307-314 (1982); Almquist et al., J Med Chem. 23:1392-1398 (1980); Jennings-White et al, Tetrahedron Lett. 23:2533 (1982); Szelke et al., EP 45665 (1982); Holladay et al, Tetrahedron Lett. 24:4401-4404 (1983); and Hruby, Life Sci. 31:189-199 (1982)).

Naturally-occurring amino acids are identified throughout by the conventional three-letter or one-letter abbreviations indicated below, which are as generally accepted in the peptide art and are recommended by the IUPAC-IUB commission in biochemical nomenclature:

**Table 1. Amino acid codes**

| Name | 3-letter code | 1-letter code | Name | 3-letter code | 1-letter code |
|---|---|---|---|---|---|
| Alanine | Ala | A | Leucine | Leu | L |
| Arginine | Arg | R | Lysine | Lys | K |
| Asparagine | Asn | N | Methionine | Met | M |
| Aspartic acid | Asp | D | Phenylalanine | Phe | F |
| Cysteine | Cys | C | Proline | Pro | P |
| Glutamic acid | Glu | E | Serine | Ser | S |
| Glutamine | Gln | Q | Threonine | Thr | T |
| Glycine | Gly | G | Tryptophan | Trp | W |
| Histidine | His | H | Tyrosine | Tyr | Y |
| Isoleucine | Ile | I | Valine | Val | V |

The peptide sequences set out herein are written according to the generally accepted convention whereby the N-terminal amino acid is on the left and the C-terminal amino acid is on the right By convention, L-amino acids are represented by upper case letters and D-amino acids by lower case letters.

The term "alkyl," as used herein, refers to a straight chain or branched hydrocarbon of one to ten carbon atoms or a cyclic hydrocarbon group of three to ten carbon atoms. Said alkyl group is optionally substituted with one or more substituents independently selected from the group of: alkyl, alkenyl, alkynyl, aryl, heteroalkyl, aralkyl, hydroxy, alkoxy, aralkyloxy, aryloxy, carboxy, acyl, aroyl, halo, nitro, trihalomethyl, cyano, alkoxycarbonyl, aryloxycarbonyl, aralkoxycarbonyl, acylamino, aroylamino, dialkylamino, carbamoyl, alkylcarbamoyl, dialkylcarbamoyl, alkylthio, aralkylthio, arylthio, alkylene and NZ₁Z₂ where Z₁ and Z₂ are independently hydrogen, alkyl, aryl, and aralkyl, This term is exemplified by such groups as methyl, ethyl, *n*-propyl, *i*-,propyl, *n*-butyl, t-butyl, 1-butyl (or 2-methylpropyl), cyclopropylmethyl, *i*-amyl, *n*-amyl, hexyl, cyclopropyl, cyclobutyl, cyclopentyl, and the like The term "alkenyl" refers to a straight chain or branched hydrocarbon of two to ten carbon atoms having at least one carbon to carbon double bond Said alkenyl group can be optionally substituted with one or more substituents as defined above Exemplary groups include allyl and vinyl.

The term "alkynyl" refers to a straight chain or branched hydrocarbon of two to ten carbon atoms having at least one carbon to carbon triple bond. Said alkynyl group can be optionally substituted with one or more substituents as defined above Exemplary groups include ethynyl and propargyl.

The term "heteroalkyl," as used herein, refers to an alkyl group of 2 to 10 carbon atoms, wherein at least one carbon is replaced with a hetero atom, such as N, O or S

The term "aryl" (or "Ar"), as used herein, refers to an aromatic carbocyclic group containing about 6 to about 10 carbon atoms or multiple condensed rings in which at least one ring is an aromatic carbocyclic group containing 6 to about 10 carbon atoms. Said aryl or Ar group can be optionally substituted with one or more substituents as defined above. Exemplary aryl groups include phenyl, tolyl, xylyl, biphenyl, naphthyl, 1,2,3,4-tetrahydronaphthyl, anthryl, phenanthryl, 9-fluorenyl, and the like.

The term "aralkyl," as used herein, refers to a straight or branched chain alkyl, alkenyl or alkynyl group, wherein at least one of the hydrogen atoms is replaced with an aryl group, wherein the aryl group can be optionally substituted with one or more substituents as defined above. Exemplary aralkyl groups include benzyl, 4-phenylbutyl, 3,3-diphenylpropyl and the like

The term "alkoxy," as used herein, refers to RO-, wherein R is alkyl, alkenyl or alkynyl in which the alkyl, alkenyl and alkynyl groups are as previously described. Exemplary alkoxy groups include methoxy, ethoxy, n-propoxy, I-propoxy, n-butoxy, and heptoxy

The term "aryloxy" as used herein, refers to an "aryl-O-" group in which the aryl group is as previously described Exemplary aryloxy groups include phenoxy and naphthoxy.

The term "alkylthio," as used herein, refers to RS-, wherein R is alkyl, alkenyl or alkynyl in which the alkyl, alkenyl and alkynyl groups are as previously described, Exemplary alkylthio groups include methylthio, ethylthio, I-propylthio and hepthylthio.

The term "arylthio," as used herein, refers to an "aryl-S-" group in which the aryl group is as previously described Exemplary arylthio groups include phenylthio and naphthylthio

The term "aralkyloxy," as used herein, refers to an "aralkyl-O-" group in which the aralkyl group is as previously described Exemplary aralkyloxy groups include benzyloxy

The term "aralkylthio," as used herein, refers to an "aralkyl-S-" group in which the aralkyl group is as previously described. Exemplary aralkylthio groups include benzylthio.

The term "dialkylamino," as used herein, refers to an -NZ₁Z₂ group wherein Z₁ and Z₂ are independently selected from alkyl, alkenyl or alkynyl, wherein alkyl, alkenyl and alkynyl are as previously described Exemplary dialkylamino groups include ethylmethylamino, dimethylamino and diethylamino.

The term "alkoxycarbonyl," as used herein, refers to R-O-CO-, wherein R is alkyl, alkenyl or alkynyl, wherein alkyl, alkenyl and alkynyl are as previously described. Exemplary alkoxycarbonyl groups include methoxy-carbonyl and ethoxy-carbonyl.

The term "aryloxycarbonyl," as used herein, refers to an "aryl-O-CO-", wherein aryl is as defined previously. Exemplary aryloxycarbonyl groups include phenoxy-carbonyl and naphtoxy-carbonyl

The term "aralkoxycarbonyl," as used herein, refers to an "aralkyl-O-CO-," wherein aralkyl is as defined previously Exemplary aralkoxycarbonyl groups include benzyloxycarbonyl

The term "acyl" as used herein, refers to RC(O)-, wherein R is alkyl, alkenyl, alkynyl, heteroalkyl, a heterocyclic ring, or a heteroaromatic ring, wherein alkyl, alkenyl, alkynyl, heteroalkyl, heterocyclic, and heteroaromatic are as defined previously.

The term "aroyl" as used herein, refers to an ArC(O)- group, wherein Ar is as defined previously.

The term "carboxy" as used herein, refers to ROC(O)-, wherein R is H, alkyl, alkenyl or alkynyl, and wherein alkyl, alkenyl or alkynyl are as defined previously.

The term "carbamoyl," as used herein, refers to a H₂N-CO- group.

The term "alkylcarbamoyl," as used herein, refers to an "Z₁Z₂N-CO-" group wherein one of the Z₁ and Z₂ is hydrogen and the other of Z₁ and Z₂ is independently selected from alkyl, alkenyl or alkynyl and wherein alkyl, alkenyl and alkynyl are as defined previously.

The term "dialkylcarbamoyl," as used herein, refers to a "Z₁Z₂N-CO-" group wherein Z₁ and Z₂ are independently selected from alkyl, alkenyl or alkynyl and wherein alkyl, alkenyl and alkynyl are as defined previously

The term "acylamino", as used herein, refers to an "acyl-NH-" group, wherein acyl is as defined previously.

The term "halo" as used herein, refers to fluoro, chloro, bromo or iodo In one embodiment, "halo" refers to fluoro, chloro or bromo

"As used herein, the term "about" refers to a +/-10% variation from the nominal value. It is to be understood that such a variation is always included in any given value provided herein, whether or not it is specifically referred to

Other chemistry terms herein are used according to conventional usage in the art, as exemplified by The McGraw-Hill Dictionary of Chemical Terms (ed, Parker, S, 1985), McGraw Hill, San Francisco, incorporated herein by reference).

### 1. Modified Pore-forming Protein Toxins

The modified pore-forming protein toxins (MPPTs) according to the present invention are derived from naturally occurring pore-forming protein toxins (nPPTs) such as aerolysin or aerolysin-related polypeptides Suitable aerolysin-related nPPTs have the following features: a pore-forming activity that is activated by removal of an inhibitory domain via protease cleavage, and the ability to bind to receptors that are present on cell membranes through one or more binding domains Examples include, but are not limited to, preproaerolysin and proaerolysin from *Aeromonas hydrophila, Aeromonas trota* and *Aeromonas salmonicida,* alpha toxin from *Clostridium septicum,* anthrax protective antigen, *Vibrio cholerae* VCC toxin, epsilon toxin from *Clostridium perfringens,* and *Bacillus thuringiensis* delta toxins

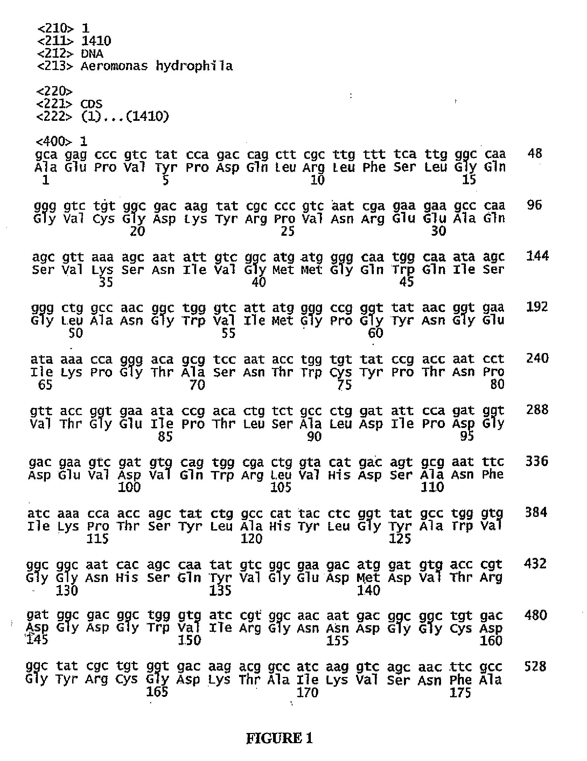

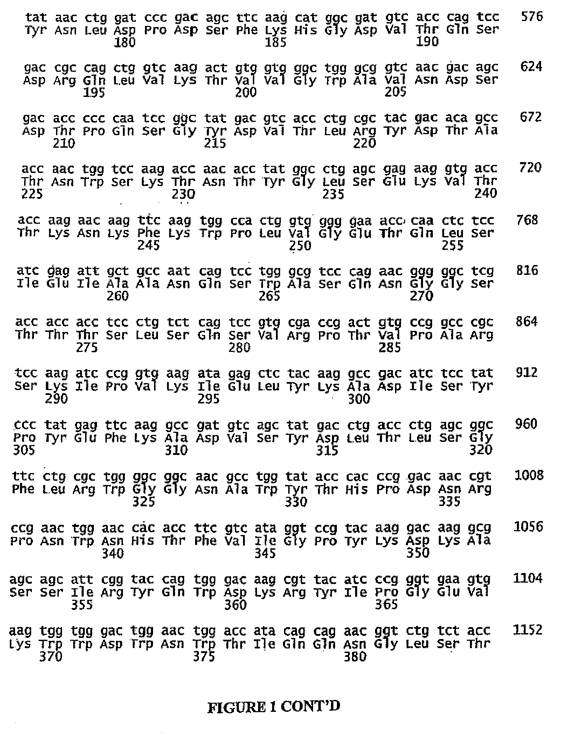

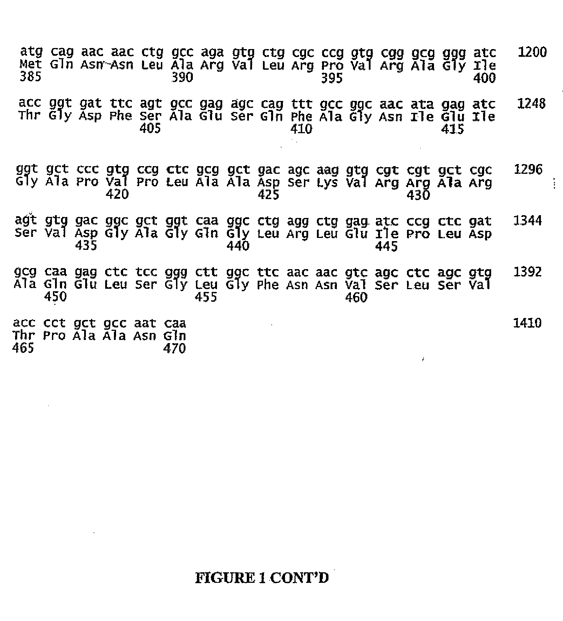

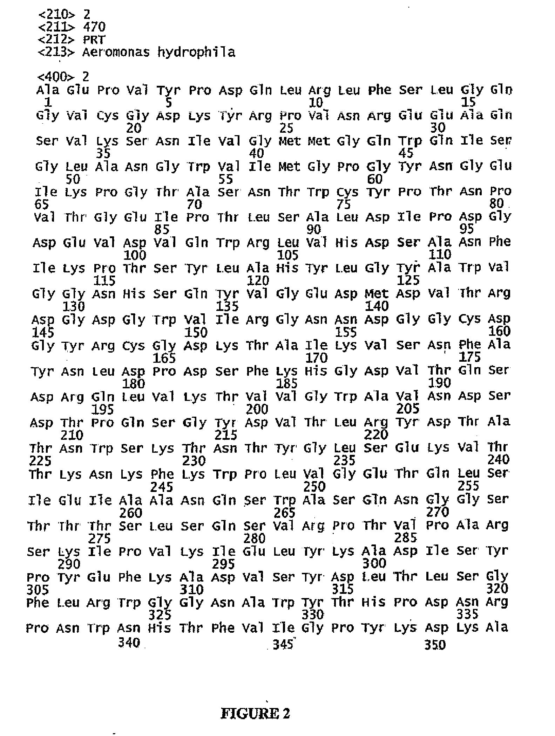

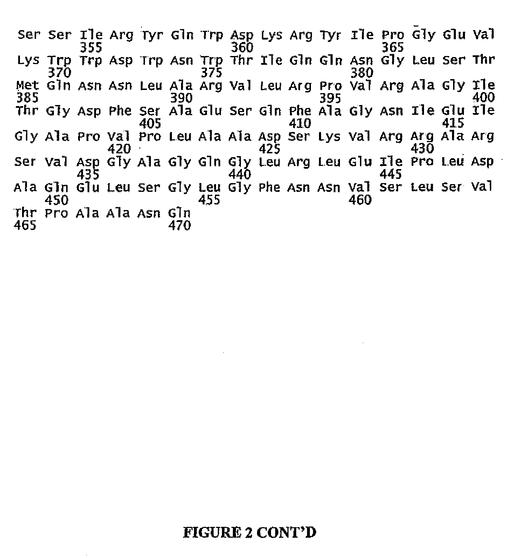

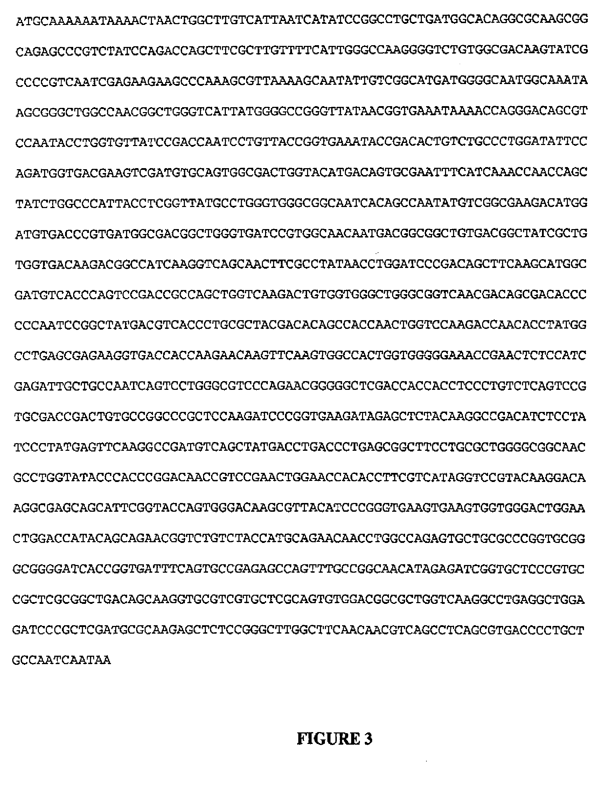

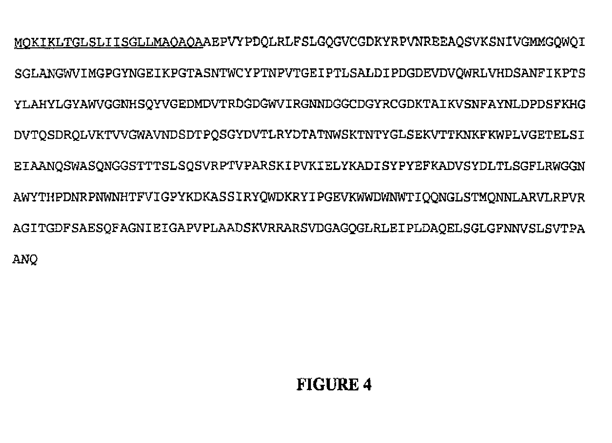

Proaerolysin (PA) polypeptides from the *Aeromonas* species noted above have been characterized These polypeptides exhibit greater than 80% pairwise sequence identity between them (Parker et al, Progress in Biophysics & Molecular Biology 88 (2005) 91-142). Each of these PA polypeptides is an approximately 52 kDa protoxin with approximately 470 amino acid residues A cDNA sequence for wild-type PA from *A hydrophila* is shown in SEQ ID NO: 1 (Figure 1) and the corresponding amino acid sequence of this wild-type PA is shown in SEQ ID NO:2 (Figure 2). Where applicable, one of skill in the art will understand that MPPTs can be designed based on the sequence of the nPPT with or without a signal sequence For example, MPPTs can be designed based on the nucleotide sequence for preproaerolysin as shown in Figure 3 (SEQ ID NO:3) or based on the amino acid sequence for preproaerolysin as shown in Figure 4 (SEQ ID NO:4). The nucleotide and protein sequences for numerous naturally occurring nPPTs are known in the art and non-limiting examples are listed in the following Table:

**Table 2: Exemplary nPPTs and corresponding GenBank™ Accession Numbers**

| nPPT | Nucleotide sequence (GenBank^{™} Accession No.) | Amino acid sequence (GenBank^{™} Accession No.) |
|---|---|---|
| Aeromonas hydrophila aerolysin | Buckley AerA, not corrected: M16495 | Buckley AerA corrected P09167 |
| *A. sobria* proaerolysin (Husslein, Chakraborty) | Y00559 | CAA68642 |
| *A. sobria* hemolysin (Hirono, I, Aoki, T., Asao, T and Kozaki, S) | X65046 | CAA46182 |
| *A*. *trota* proaerolysin, (Khan et al) | AF064068 | AAC26217 |
| *A salmonicida* hemolysin (Hirono & Aoki) | X65048 | CAA46184 |

The *A hydrophila* PA protein includes a binding domain (approximately amino acids I-83 of SEQ ID NO: 2) in what is known as the small lobe of the polypeptide and referred to herein as the small lobe binding domain (SBD), and a C-terminal inhibitory peptide (CIP) domain (approximately amino acids 427-470 of SEQ ID NO: 2) that is removed by protease cleavage at an activation sequence to activate PA. Cleavage at the activation sequence to remove the CIP domain can be carried out by a number of' ubiquitous proteases including furin and trypsin, The amino acid residues from approximately 84-426 of SEQ ID NO: 2 are known as the large lobe of the PA polypeptide, and contain a toxin domain and other functional domains, including a second binding domain, referred to herein as the large lobe binding domain (LBD).

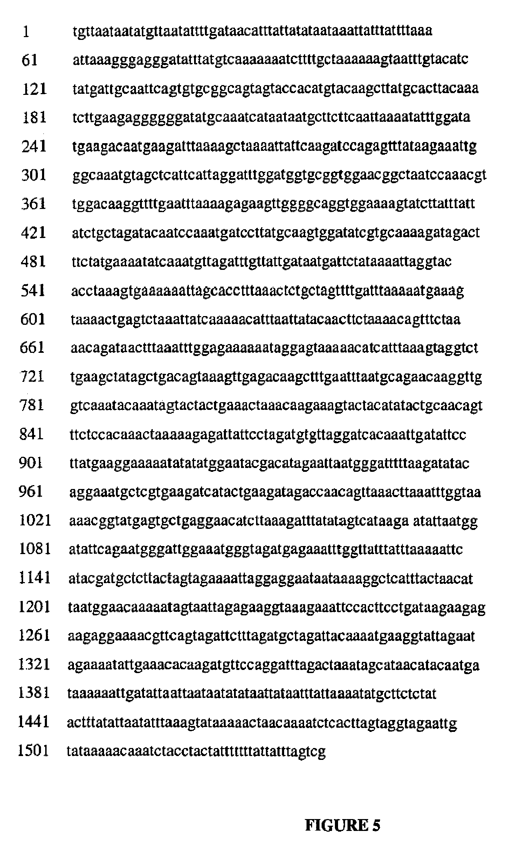

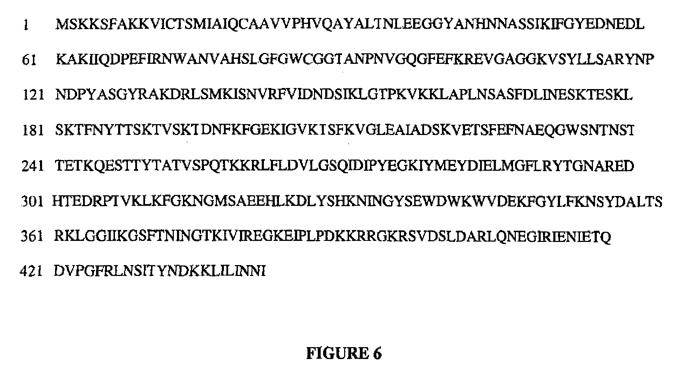

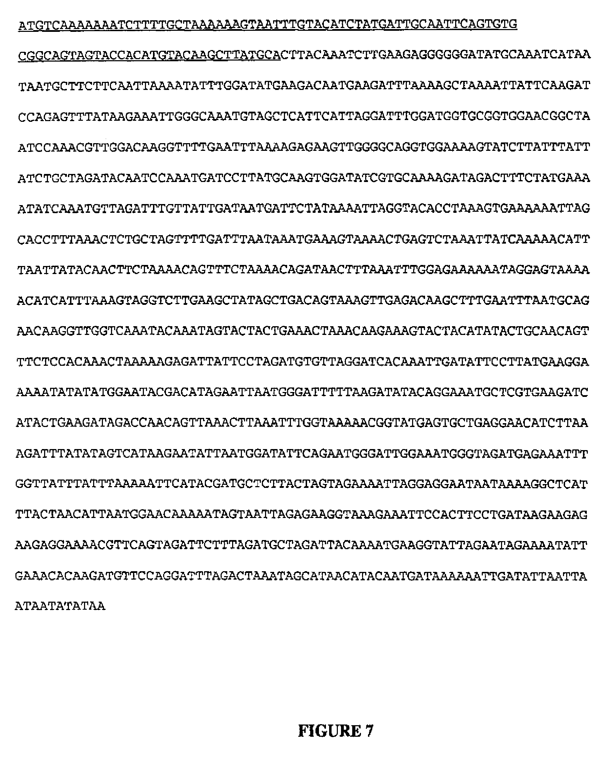

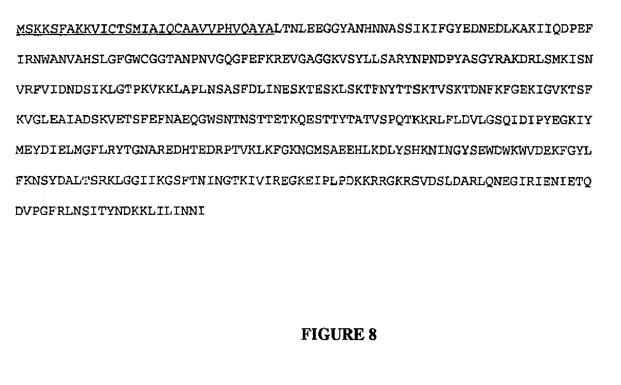

Alpha toxin from *C septicum* is considered to be a homologue of proaerolysin based on significant sequence identity and other similarities (Parker *et al, supra*) Alpha toxin is secreted as a 46,450 Da protoxin (approximately 443 amino acids) that is activated by protease cleavage to remove a C-terminal inhibitory peptide (CIP) domain, and it also binds to glycosyl-phosphatidylinositol (GPI)-anchored proteins. Alpha toxin, however, does not have a region corresponding to the small lobe of PA. An example of a *Clostridium septicum* alpha toxin nucleic acid sequence is provided in GenBank™ Accession No 575954 *A Clostridium septicum* alpha toxin nucleotide sequence is also shown in Figure 5 (SEQ ID NO:5). An example of a *Clostridium septicum* alpha toxin protein sequence is provided in GenBank™ Accession No AAB32892. SEQ ID NO:6, as shown in Figure 6, is also an example of a *Clostridium septicum* protein sequence. The sequence of a *Clostridium septicum* nucleotide sequence including signal sequence (SEQ ID NO:7) is shown in Figure 7 (signal sequence underlined) An exemplary *Clostridium septicum* nucleotide sequence including signal sequence (SEQ ID NO:8) is shown in Figure 8 (signal sequence underlined). Based on sequence homology, alpha toxin is thought to have a similar structure and similar GPI-anchored protein binding activity to that of proaerolysin.

In one embodiment, the MPPTs according to the present invention comprise a modified proaerolysin polypeptide In a further embodiment, the MPPTs comprise a modified proaerolysin polypeptide from *A. hydrophila,* In another embodiment of the invention, the MPPTs comprise a modified alpha toxin polypeptide

The present invention further includes MPPTs that are derived from fragments of nPPTs Suitable fragments include those that are capable of' being activated to form pores in target cells by removal of the CIP domain *i.e* are "functional fragments " For example, in the case of PA, a suitable fragment would be one that comprised the large lobe of the protein as well as the CIP domain and activation sequence Thus, in one embodiment of the invention, the MPPT is derived from a fragment of proaerolysin that includes the large lobe, the CIP domain and the activation sequence In another embodiment, the MPPT is derived from a fragment of proaerolysin, that comprises the small lobe, the large lobe, the activation sequence, but only part of a CIP domain. Other functional fragments could be readily determined by by the skilled technician using standard techniques known in the art

MPPTs according to the present invention comprise a modification to the naturally occurring activation sequence that permits activation of' the MPPT in a variety of different cancer types The modification functionally deletes a native protease cleavage site and introduces one or more general cleavage sites, each of which is cleavable by an enzyme, the presence of which is associated with a variety of cancer types, or a plurality of specific cleavage sites, each of which is cleavable by an enzyme, the presence of which is associated with a specific type of cancer These modifications result in a single MPPT molecule that is capable of being activated to kill numerous types of cancer cells. These modifications are described in greater detail below In addition, one or more further optional modifications may be made to the MPPTs. These optional modifications allow the MPPT to be selectively activated to kill selected cancer cells These modifications include one or more modification of the native small lobe binding domain (SBD), one or more modifications of' the native large lobe binding domain (LBD), or addition of one or more artificial regulatory domains (ARD). These optional modifications are also described in detail below.

In one embodiment of the invention, the MPPT comprises a modified activation sequence In another embodiment of the invention, the MPPT comprises a modified activation sequence and one or more modifications to the SBD. In still another embodiment, the MPPT comprises a modified activation sequence and one or more mutations to the LBD In yet another embodiment, the MPPT comprises a modified activation sequence and one or more ARD In a further embodiment, the MPPT comprises a modified activation sequence, one or more modifications to the SBD, and one or more modifications to the LBD In a still further embodiment, the MPPT comprises a modified activation sequence, one or more modifications to the SBD and one or more ARD In yet a further embodiment, the MPPT comprises a modified activation sequence, one or more modifications to the LBD and one or more ARD In one more embodiment, the MPPT comprises a modified activation sequence, one or more modifications to the LBD, one or more modifications to the SBD, and one or more ARD.

In one embodiment of the invention, the MPPT comprises a modified activation sequence comprising one or more general cleavage sites In another embodiment of the invention, the MPPT comprises a modified activation sequence comprising one or more general cleavage sites and one or more modifications to the SBD In still another embodiment, the MPPT comprises a modified activation sequence comprising one or more general cleavage sites and one or more modifications to the LBD In yet another embodiment, the MPPT comprises a modified activation sequence comprising one or more general cleavage sites and one or more ARD In a further embodiment, the MPPT comprises a modified activation sequence comprising one or more general cleavage sites, one or more modifications to the SBD, and one or more modifications to the LBD In a still further embodiment, the MPPT comprises a modified activation sequence comprising one or more general cleavage sites, one or more modifications to the SBD and one or more ARD In yet a further embodiment, the MPPT comprises a modified activation sequence comprising one or more general cleavage sites, one or more modifications to the LBD and one or more ARD In one more embodiment, the MPPT comprises a modified activation sequence comprising one or more general cleavage sites, one or more modifications to the LBD, one or more modifications to the SBD, and one or more ARD

In one embodiment of the invention, the MPPT comprises a modified activation sequence comprising a plurality of specific cleavage sites In another embodiment of the invention, the MPPT comprises a modified activation sequence comprising a plurality of specific cleavage sites and one or more modifications to the SBD In still another embodiment, the MPPT comprises a modified activation sequence comprising a plurality of specific cleavage sites and one or more mutation to the LBD In yet another embodiment, the MPPT comprises a modified activation sequence comprising a plurality of specific cleavage sites and one or more ARD In a further embodiment, the MPPT comprises a modified activation sequence comprising a plurality of specific cleavage sites, one or more modifications to the SBD, and one or more modifications to the LBD In a still further embodiment, the MPPT comprises a modified activation sequence comprising a plurality of specific cleavage sites, one or more modifications to the SBD and one or more ARD In yet a further embodiment, the MPPT comprises a modified activation sequence comprising a plurality of specific cleavage sites, one or more modifications to the LBD and one or more ARD In one more embodiments, the MPPT comprises a modified activation sequence comprising a plurality of specific cleavage sites, one or more modifications to the LBD, one or more modifications to the SBD, and one or more ARD.

### 1.1. Modification of Activation Sequence

MPPTs according to the present invention comprise modifications of the naturally occurring activation sequence of the nPPT permitting activation of' the MPPTs in a variety of different cancer types The modified activation sequence comprises one or more general cleavage site modifications, or a plurality of specific cleavage site modifications, resulting in a single MPPT molecule that is capable of being activated to kill numerous types of cancer cells

### 1.1.1 Modifications comprising one or more general cleavage sites

MPPTs with one or more general cleavage site modifications comprise a modification of the naturally occurring activation sequence to provide one or more cleavage sites for a general activating agent. A general activating agent is an enzyme, the presence of which is associated with a variety of different cancer types For example, the expression of the enzyme can be up-regulated in a cancer cell compared to a normal cell, or the enzyme can be localized to cancer cells as compared to normal cells, or the enzyme may be produced and/or activated by cancer associated tissue or cells A general activating agent may be, for example, a protease In one embodiment, the MPPT comprises an activation sequence modified to include two or more general cleavage sites, each of the general cleavage sites can be cleaved by the same general activating agent. Alternatively, each of the general cleavage sites can be cleaved by a different activating agent When more than one general cleavage site is present, these cleavage sites may either be adjacent to each other, may overlap or may be separated by intervening sequences of varying lengths as is known in the art. In another embodiment, the MPPT comprises an activation sequence modified to include one general cleavage site In still another embodiment, the MPPT comprises an activation sequence modified to include two general cleavage sites. In yet another embodiment, the MPPT comprises an activation sequence modified to include less than five general cleavage sites.

The one or more general cleavage site modifications to the naturally occurring activation sequence may be achieved as is known in the art. This modification results in functional deletion of the naturally occurring activation sequence, or of one or more naturally occurring cleavage sites in the activation sequence. Functional deletion is achieved by mutation, which can result in, for example, partial or complete deletion, insertion, or other variation made to the naturally occurring activation sequence that renders it inactive In one embodiment, the native activation sequence of the nPPT may be functionally deleted by insertion of one or more general cleavage site In another embodiment, functional deletion of the naturally occurring activation sequence, of of one or more naturally occurring cleavage sites in the activation sequence is achieved via mutations in one or more amino acid residues of the native activation sequence which result in the creation of one or more general cleavage sites, each of which can be cleaved by a general activating agent. In an alternate embodiment, the native activation sequence of the nPPT is functionally deleted by replacing the naturally occurring activation sequence, or one or more naturally occurring cleavage sites in the activation sequence with one or more general cleavage sites, each of which can be cleaved by a general activating agent.

As described above, the MPPTs according the present invention comprise one or more general cleavage site modifications that provide one or more cleavage sites, each recognized by a general activating agent that is a protease, the presence of which is associated with a variety of different cancer types In one embodiment of the invention, the general activating agent is a protease that is associated with cancer invasion and metastasis in general. Examples of such proteases include the matrix metalloproteinase (MMP) family, the caspases, elastase, and the plasminogen activator family, as well as fibroblast activation protein Members of the MMP family include collagenases, stromelysin, gelatinases, and membrane-type metalloproteases In particular, MMP-2 (gelatinase A), MMP-9 (gelatinase B), and membrane-type 1 MMP (MT1-MMP) have been reported to be most related to invasion and metastasis in various human cancers Examples of proteases of the plasminogen activator family include uPA (urokinase-type plasminogen activator) and tPA (tissue-type plasminogen activator)

In another embodiment, the protease is up-regulated and/or secreted by cancer cells Examples of these proteases include some matrix metalloproteases, some cathepsins, tPA, some caspases, and some kallikreins In still another embodiment, the protease is secreted by cancer-associated cells Examples of these proteases include matrix metalloproteases, elastase, plasmin, thrombin, and uPA In a further embodiment, the protease is activated by enzymes expressed by cancer cells In still another embodiment, the protease is activated by receptors expressed by cancer cells A non-limiting example of such a protease is uPA, which is activated by the receptor uPAR (urokinase-type plasminogen activator receptor)

As is known in the art, the proteases described above recognize certain cleavage sites. Non-limiting examples of selected cleavage sites recognized by some of these proteases are shown in Table 3 Cleavage sites recognized by other proteases listed above are known in the art

**Table 3: Peptide sequences favored by proteases**

| Protease | Favoured cleavage sequence "/" indicates site of cleavage | SEQ ID NO |
|---|---|---|
| Caspase 1 | YVADI/X | 46 |
| tPA | FGR/X | 47 |
| MMP14 | GGPLG/LYAGG | 48 |
| HK2 | GKAFRR/X | 49 |
| Thrombin | LVPR/GS | 50 |
| uPA | SGR/SAQ | 51 |
| MMP2 | HPVG/LLAR | 52 |

One of skill in the art would understand that cleavage sites other than those listed in Table 3 are recognized by the listed proteases, and can be used as a general protease cleavage site according to the invention

In another embodiment, the general activating agent is uPA In another embodiment of the invention the cleavage site added to the activation sequence is a uPA cleavage site An example of a uPA cleavage site is: SGRSAQ (SEQ ID NO:51) In another embodiment, the general activating agent is a protease that is associated with angiogenesis in general Examples of such proteases are matrix metalloproteases and caspases

In one embodiment, the MPPT comprises a general cleavage site that is recognized by uPA, and optionally one or more other general cleavage sites In another embodiment, the MPPT comprises a general cleavage site that is recognized by a MMP

### 1.1.2 Modifications comprising a plurality of specific cleavage sites

MPPTs with a plurality of specific cleavage site modifications comprise modification of the naturally occurring activation sequence to include two or more different types of specific cleavage sites, each type capable of being cleaved by a specific activating agent Each specific cleavage site is recognized by a different specific activating agent The two or more different types of cleavage sites may further comprise a cleavage site for a general activating agent. A specific activating agent is an enzyme, the presence of which is associated with a specific type of cancer For example, expression of' the enzyme can be up-regulated in a specific type of cancer cell, or the enzyme can be localized to a specific type of cancer cell, or the enzyme may be produced by a cell that is associated with a specific type of cancer A specific activating agent may be, for example, a protease

Modifications comprising a plurality of specific cleavage sites may be achieved as is known in the art, and described above. This modification also results in functional deletion of the naturally occurring activation sequence, or of one or more naturally occurring cleavage sites in the activation sequence In one embodiment, the native activation sequence of the nPPT is functionally deleted by insertion of a plurality of specific cleavage sites In another embodiment, functional deletion of the naturally occurring activation sequence is achieved via mutations in the amino acid sequence of the naturally occurring activation sequence, resulting in the addition of two or more specific cleavage sites, each of' which can be cleaved by a specific activating agent In an alternate embodiment, the native activation sequence of the nPPT may be replaced with two or more specific cleavage sites, each of which is capable of being cleaved by a specific activating agent. As is known in the art, the specific cleavage sites may either be adjacent to each other, may overlap or may be separated by intervening sequences of varying lengths as is known in the art.

In another embodiment of the invention, the plurality of specific cleavage site modifications adds two or more cleavage sites, each of which is recognized by a specific activating agent that is a protease. In another embodiment of the invention, the specific activating agent is a protease that is associated with invasion and metastasis of a specific cancer In a further embodiment of the invention, the specific activating agent is a protease, the expression of which is up-regulated in a specific cancer. In still another embodiment, the specific activating agent is a protease that is produced by a cell that is associated with a specific cancer.

In another embodiment, the specific activating agent is a protease that is associated with lung cancer.

### 1.2. Modifications to Binding Domain(s)

MPPTs according to the present invention are derived from nPPTs that comprise one or more binding domains, as known in the art. In the context of the present invention, when an nPPT comprises one binding domain, it is considered to be a "large lobe binding domain " MPPTs according to the present invention may comprise modifications to one or more binding domains, as applicable For example, native proaerolysin from *Aeromonas* species comprises two binding domains, a small lobe binding domain, and a large lobe binding domain. In contrast, native alpha toxin from *Clostridium septicum* comprises only a large lobe binding domain In one embodiment, modifications of the binding domains include functional deletion of' a binding domain A functionally deleted binding domain in an MPPT results in an MPPT that has an attenuated ability to bind to its cell surface receptors, yet still retains pore-forming ability Functional deletions can be made by deleting or mutating one or more binding domain of an MPPT In one embodiment, the entire binding domain or portions thereof, may be deleted. In an additional embodiment, insertion of heterologous sequences into the binding domain may also be used to functionally delete the binding domain Addition of these heterologous sequences may confer an additional functionality to the MPPT For example, addition of a heterologous sequence can result in the addition of a region that can function as an ARD as described in Section 13 below. In an alternative embodiment, an MPPT may comprise a blocking group that functions to prevent interaction of the binding domain with its cell membrane receptor. Methods of attaching blocking groups to MPPTs are known in the art and described in Section 2 below. In still another embodiment, point mutations to the amino acid sequence of the native binding domain of the nPPT can also be made to decrease the ability of' the binding domain to bind to its receptor. Further details regarding these modifications are described below

MPPTs with functional deletions in the binding domain may be carried out using methods known in the art. These methods include the use of recombinant DNA technology as described in Sambrook et al, *supra.* Alternatively, functional deletions of' the binding domain may also be achieved by direct modification of the protein itself according to methods known in the art, such as proteolysis to generate fragments of the MPPT, which can then be chemically linked together (See Section 2 2).

In one embodiment of the invention, the MPPT is modified by functional deletion of its small lobe binding domain (SBD). Exemplary functional deletions of' the SBD may be made in the *A hydrophila* proaerolysin polypeptide as follows. The entire SBD, corresponding to amino acid 1-83 of SEQ ID NO:2 may be deleted, or portions of this region may be deleted, for example amino acids 45-66 of SEQ ID NO:2. Alternatively, one or more point mutations can be made at the following positions: W45, 147, M57, Y61, K66 (amino acid numbers refer to SEQ ID NO: 2). Exemplary mutations include, but are not limited to W45A, 147E, M57A,Y61A, Y61C, K66Q (amino acid numbers refer to SEQ ID NO: 2) and as described in Mackenzie et al. J Biol Chem 274: 22604-22609, 1999.

In one embodiment of' the invention, the MPPT is modified by functional deletion of its large lobe binding domain (LBD). Exemplary functional deletions of the LBD of MPPTs, based on proaerolysin (contained in approximately amino acid residues 84-426 of SEQ ID NO:2) may be made as follows. The entire LBD of proaerolysin may be deleted Alternatively, in one embodiment of the invention, the MPPT based on proaerolysin comprises one or more point mutations in the LBD to amino acid residues Y162, W324, R323, R336, and/or W127 In another embodiment of the invention, the MPPT based on proaerolysin comprises one or more point mutations in W127 and/or R336 In still another embodiment, the MPPT based on proaerolysin comprises the point mutations Y162A and/or W324A In a further embodiment the MPPT based on proaerolysin comprises the point mutations R336A, R336C, and/or W127T In another embodiment, MPPTs comprise mutations to other residues that interact directly with the GPI-protein ligand

Exemplary mutations to the LBD of MPPTs derived from alpha toxin are noted below and include at least one substituted amino acid in the receptor binding domains of the alpha toxin which include amino acid residues 53, 54, 62, 84-102, 259-274 and 309-315 of the sequence of the native alpha toxin as shown in SEQ ID NO: 6 In one embodiment of the invention, MPPTs derived from alpha toxin include mutations to one or more of the following residues: W85, Y128, R292, Y293, and R305.

### 1.3. Addition of Artificial Regulatory Domain (ARD)

The MPPTs according to the present invention may be optionally modified by the addition of one or more artificial regulatory domains (ARDs) ARDs that can be added to MPPTs include targeting units that are capable of selectively targeting the MPPT to one or more target cell (for example one or more organs, tissues or cell types), and inhibitory units that are capable of inhibiting the activity of the MPPT. One skilled in the art will recognize the possibility that an ARD can function as both a targeting unit and an inhibitory unit According to one embodiment of the invention, an MPPT is modified by the addition of one or more ARD that is a targeting unit In another embodiment of the invention, the MPPT is modified by the addition of one or more ARD that is an inhibitory unit In still another embodiment the MPPT is modified by the addition of an ARD that can function as both a targeting unit and an inhibitory unit In an additional embodiment, the ARD is capable of functioning to inhibit binding to normal cells, yet able to direct binding to cancer cells. In a further embodiment, the addition of an ARD can function to both target the MPPT to cancer cells, and inhibit the ability of the MPPT to bind to cell surface receptors that are recognized by the nPPT it is derived from ARDs according to the present invention may be proteins, peptides, or other moieties.

### 1.3.1 Targeting units

The targeting units according to the present invention may be used to provide target selectivity to the MPPT For example, the targeting unit can direct the MPPT to the target cell, where the MPPT can be activated and subsequently kill the target cell. Additionally, the targeting unit may act as an inactivator of' the MPPT. In this regard, the targeting unit may charge neutralize or sterically inhibit the MPPT from pore formation. The targeting unit may be added to the N- or C-terminus, or both Alternatively, the targeting unit may be added to any region of the MPPT, including the SBD or the LBD, as long as it does not interfere with the pore-forming activity of the MPPT.

The targeting unit can be a ligand that binds selectively to the target cell, In one embodiment, the targeting unit is an antibody. Antibodies contemplated by the instant invention can be a full-length (i.e, naturally occurring or formed by normal immunoglobulin gene fragment recombinatorial processes) immunoglobulin molecule (for example, an IgG antibody) or an immunologically active (i.e., specifically binding) portion of an immunoglobulin molecule, and may or may not be humanized. Suitable antibodies can be polyclonal or monoclonal antibodies Alternatively, the antibody may be a single chain antibody as known in the art and described in Bird et al Science 242:423-6, 1988; and Huston et al., Proc. Natl Acad Sci. 85:5879-83, 1988. In one embodiment, an antibody includes camelized antibodies (for example see Tanha et al, J. Biol Chem 276:24774-80, 2001)

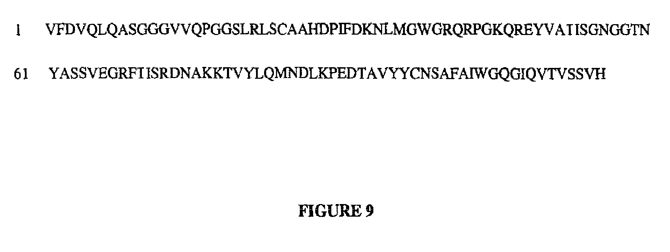

In one embodiment the targeting unit is an antibody that selectively binds a specific type of cancer cell In another embodiment, the targeting unit is an antibody that selectively binds a lung cancer cell In yet another embodiment, the targeting unit is an antibody that selectively binds non-small cell lung cancer cells. For example, the targeting unit may be an AFAI antibody or fragment thereof as shown in Figure 9 (SEQ ID NO:9) that is capable of selectively binding lung cancer cells. AFAI is a single domain antibody that binds cell adhesion molecule 6. The pentavalent form of the antibody is known as ES1 (see Mai, K.T., et al, 2006, Histopathyology, 49:515-522) and is also suitable for use as a targeting unit in accordance with the present invention

In another embodiment, the targeting unit is an antibody that recognizes one of: carcino embryonic antigen CEA (for example, catalog number AB 10036 from Abcam Inc., Cambridge, MA, recognizes colorectal cancer cells), CA-15-3 (for example, catalog number RDI-CA153-AG from Research Diagnostics and recognizes breast cancer cells), thyroid transcription factor 1 (TTF1, for example, catalog number AB 869 from Abcam Inc, Cambridge, MA, recognizes lung and thryroid carcinomas), and cytokeratin 7 (for example, catalog number RDI-PR061025 from Research Diagnostics Inc., Flanders, NJ, recognizes epithelial cells of ovary, lung and breast) In another embodiment, the targeting unit is an antibody that recognizes p97 melanotransferrin antigen in melanoma cells, or the L6 antigen on renal cell carcinomas.

In another embodiment, the targeting unit is the antibody Rituxan, which targets CD20 and B-cell non-Hodgkin's lymphoma cells. Alternatively, the targeting unit is Herceptin®. (Genentech) which recognizes some breast cancers and lymphomas; Alemtuzumab (MabCampath®) which binds to CD52, a molecule found on white blood cells and recognizes chronic lymphocytic leukemia cells; Lym-1 (Oncolym®, Schering), which binds to the HLA-DR-encoded histocompatibility antigen that can be expressed at high levels on lymphoma cells; Bevacizumab (Avastin®, Avastin), which binds to vascular endothelial growth factor (VEGF) thus blocking its action and depriving the tumor of its blood supply, or Cetuximab (Erbitux®, Merck) which binds to colorectal cancers.

In another embodiment, the targeting unit is a molecule or ligand that recognizes or is capable of specific binding to a second molecule that is selectively expressed on the target cell. In one embodiment, the targeting unit is a ligand that is specific for a receptor that is selectively expressed on the target cell Examples of such ligands are: hormones such as steroid hormones, or peptide hormones; neuroactive substances, for example opioid peptides; insulin; growth factors, e.g., epidermal growth factor, insulin-like growth factor, fibroblast growth factor, platelet derived growth factor, tumor necrosis factor; cytokines, for example, an interleukin (IL), e.g., IL-2, IL-4, or IL-5; melanocyte stimulating hormone; a substance or receptor which has affinity for a particular class of cells (or viruses) for example, cancer cells, virally infected cells, immune cells, for example, B cells or T cells or a subset thereof, for example, soluble fragments of CD4, which bind to the protein gp120 expressed on HIV-infected cells; or a substance with an affinity for a class of molecules, for example, a lectin, such as concanavalin A, which binds a subset of glycoproteins. Adhesion molecules, for example, molecules expressed on cells of hematopoietic origin, such as CD2, CD4, CD8 which are expressed on T cells, selectins, integrins, as well as adhesion molecules expressed on non-immune cells, may also be used as targeting units to direct the MPPT of the invention to target cells Since some cancer cells abnormally express certain adhesion molecules, receptors or recognition motifs for such adhesion molecules may also be used as targeting units For example, RGD motifs, which function as integrin binding motifs, can be used as targeting units

In one embodiment, the targeting unit selectively directs the MPPT to cancer cells. In another embodiment the targeting unit selectively directs the MPPT to cells that are actively proliferating. In another embodiment the targeting unit selectively directs the MPPT to a specific tissue or organ In still another embodiment, the targeting unit selectively directs the MPPT to a lung cancer cell. In a further embodiment, the targeting unit selectively directs the MPPT to cells required for cancer growth, for example, cells forming vasculature for a tumor

In yet another embodiment, the targeting unit is additionally capable of acting as an inhibitory unit For example, the AFAI antibody fragment described above is capable of reducing the activity of the MPPT, as well as selectively targeting the MPPT to a lung cancer cell. Similarly, other proteins of similar size are capable of acting as an inhibitory unit

### 1.3.2 Inhibitory Units

As noted above, the ARD may be an inhibitory unit. Without being limited by mechanism, an inhibitory unit may inactivate the MPPT by, for example, charge neutralization, and/or by sterically inhibiting either the ability of the MPPT to bind to its receptor on the cell membrane, or the ability of the MPPT to form pores in the cell membrane. Examples of suitable inhibitory units include, but are not limited to, antibodies, antibody fragments, enzymes, carbohydrates, peptides, ubiquitin, a phage (via phage engineering) or a streptavidin microbead via peptide biotinylation

In one embodiment of the invention, the inhibitory unit is an antibody. In a further embodiment, the inhibitory unit is an AFAI antibody fragment. In still another embodiment the inhibitory unit is also capable of functioning as a targeting unit

In another embodiment, the inhibitory unit is an enzyme In a further embodiment, the inhibitory unit is a lipase

### 1.3.3 Addition of ARDs

The ARDs according to the present invention may be added to any region of the MPPT, including, but not limited to, the N- or C-terminus of the MPPT, provided that, for those ARDs that are not cleaved from the MPPT after fulfilling their targeting unit function, the ARD does not interfere with the ability of the MPPT to form pores Moreover, the ARD may replace or functionally delete a functional domain of the nPPT, such as, for example, a binding domain The ARD may be directly added to the MPPT, or it may be added via an appropriate linker. Methods of adding additional domains, such as ARDs, to proteins are known in the art. Such methods include covalent linkage of the domain to the MPPT. For example, ARDs may be added to the MPPT via covalent crosslinking (see Woo et al., Arch Pharm Res 22(5):459-63, 1999 and Debinski and Pastan, Clin. Cancer Res. 1(9):1015-22, 1995). Crosslinking can be non-specific, for example by using a homobifunctional-lysine-reactive crosslinking agent, or it can be specific, for example by using a crosslinking agent that reacts with amino groups on the ARD and with cysteine residue located in the MPPT For example, in the proaerolysin polypeptide, amino acids Cys19, Cys75, Cys159, and/or Cys164 as noted in SEQ ID NO: 2 may be used to crosslink an ARD to the proaerolysin polypeptide Many other cross-linking agents and linkers are known in the art and are suitable for use in the present invention and include those described in Section 1.3.4.

If the ARD is a protein, recombinant DNA technology can be used to add the ARD to produce the MPPT Details of suitable recombinant DNA technology can be found, for example, in Sambrook et al.(ed), Molecular Cloning: A Laboratory Manual 2nd ed , vol. 1-3, Cold Spring Harbor Laboratory Press, Cold Spring, Harbor, N Y., 1989.

In one embodiment, the ARD is added to the N-terminus of the MPPT In another embodiment, the ARD is added to the N-terminus of a MPPT derived from proaerolysin In still another embodiment, the ARD is added to the N-terminus of a MPPT derived from alpha toxin In a further embodiment the ARD is added to the C-terminus of the MPPT. In another embodiment, the ARD is added to the C-terminus of a MPPT derived from proaerolysin. In yet another embodiment, the ARD is added to the C-terminus of a MPPT derived from alpha toxin. In still another embodiment, the ARD is added to a MPPT derived from proaerolysin using recombinant DNA methods, such as those described in Section 2

### 1.3.4. Addition of ARD via a linker

According to the present invention, ARDs may be covalently attached to MPPTs through an appropriate linker or spacer. In the context of the present invention, the linker acts as a molecular bridge to link the ARD entity to the MPPT entity. The linker can serve, for example, simply as a convenient way to link the two entities, as a means to spatially separate the two entities, to provide an additional functionality to the MPPT, or a combination thereof. For example, it may be desirable to spatially separate the ARD and the MPPT to prevent the ARD from interfering with the activity of the MPPT and/or vice versa. The linker can also be used to provide, for example, lability to the connection between the ARD and the MPPT, an enzyme cleavage site (for example a cleavage site for a protease), a stability sequence, a molecular tag, a detectable label, or various combinations thereof

The selected linker can be bifunctional or polyfunctional, i e. contains at least a first reactive functionality at, or proximal to, a first end of the linker that is capable of bonding to, or being modified to bond to, the ARD and a second reactive functionality at, or proximal to, the opposite end of the linker that is capable of bonding to, or being modified to bond to, the MPPT. The two or more reactive functionalities can be the same (i.e the linker is homobifunctional) or they can be different (i.e. the linker is heterobifunctional). A variety af bifunctional or polyfunctional cross-linking agents are known in the art that are suitable for use as linkers (for example, those commercially available from Pierce Chemical Co., Rockford, IL). Alternatively, these reagents can be used to add the linker to the ARD and/or MPPT

The length and composition of the linker can be varied considerably provided that it can fulfill its purpose as a molecular bridge. The length and composition of the linker are generally selected taking into consideration the intended function of the linker, and optionally other factors such as ease of synthesis, stability, resistance to certain chemical and/or temperature parameters, and biocompatibility For example, the linker should not significantly interfere with the regulatory ability of the ARD relating to targeting or inhibition of the MPPT, or with the activity of the MPPT relating to activation, or pore-forming ability.

Linkers suitable for use according to the present invention may be branched, unbranched, saturated, or unsaturated hydrocarbon chains, including peptides as noted above. Furthermore, if the linker is a peptide, the linker can be attached to the MPPT and/or the ARD (if the ARD is also a peptide or protein) using recombinant DNA technology. Such methods are well-known in the art and details of this technology can be found, for example, in Sambrook *et al*., *supra.*

In one embodiment of the present invention, the linker is a branched or unbranched, saturated or unsaturated, hydrocarbon chain having from 1 to 100 carbon atoms, wherein one or more of the carbon atoms is optionally replaced by -O- or -NR- (wherein R is H, or C1 to C6 alkyl), and wherein the chain is optionally substituted on carbon with one or more substituents selected from the group of (C1-C6) alkoxy, (C3-C6) cycloalkyl, (C1-C6) alkanoyl, (C1-C6) alkanoyloxy, (C1-C6) alkoxycarbonyl, (C1-C6) alkylthio, amide, azido, cyano, nitro, halo, hydroxy, oxo (=O), carboxy, aryl, aryloxy, heteroaryl, and heteroaryloxy.

Examples of suitable linkers include, but are not limited to, peptides having a chain length of' 1 to 100 atoms, and linkers derived from groups such as ethanolamine, ethylene glycol, polyethylene with a chain length of 6 to 100 carbon atoms, polyethylene glycol with 3 to 30 repeating units, phenoxyethanol, propanolamide, butylene glycol, butyleneglycolamide, propyl phenyl, and ethyl, propyl, hexyl, steryl, cetyl, and palmitoyl alkyl chains.

In one embodiment, the linker is a branched or unbranched, saturated or unsaturated, hydrocarbon chain, having from 1 to 50 carbon atoms, wherein one or more of the carbon atoms is optionally replaced by -O- or -NR- (wherein R is as defined above), and wherein the chain is optionally substituted on carbon with one or more substituents selected from the group of (C1-C6) alkoxy, (C1-C6) alkanoyl, (C1-C6) alkanoyloxy, (C1-C6) alkoxycarbonyl, (C1-C6) alkylthio, amide, hydroxy, oxo (=O), carboxy, aryl and aryloxy

In another embodiment, the linker is an unbranched, saturated hydrocarbon chain having from 1 to 50 carbon atoms, wherein one or more of the carbon atoms is optionally replaced by -O- or -NR- (wherein R is as defined above), and wherein the chain is optionally substituted on carbon with one or more substituents selected from the group of (C1-C6) alkoxy, (C1-C6) alkanoyl, (C1-C6) alkanoyloxy, (C1-C6) alkoxycarbonyl, (C1-C6) alkylthio, amide, hydroxy, oxo (=O), carboxy, aryl and aryloxy.

In a specific embodiment of the present invention, the linker is a peptide having a chain length of 1 to 50 atoms. In another embodiment, the linker is a peptide having a chain length of 1 to 40 atoms

Peptide linkers which are susceptible to cleavage by enzymes of the complement system, urokinase, tissue plasminogen activator, trypsin, plasmin, or another enzyme having proteolytic activity may be used in one embodiment of the present invention. According to another embodiment of the present invention, an MPPT is attached via a linker susceptible to cleavage by enzymes having a proteolytic activity such as a urokinase, a tissue plasminogen activator, plasmin, thrombin or trypsin. In addition, MPPTs may be attached via disulfide bonds (for example, the disulfide bonds on a cystine molecule) to the ARD molecule Since many tumors naturally release high levels of glutathione (a reducing agent) this can reduce the disulfide bonds with subsequent release of the MPPT at the site of' delivery

In one embodiment, the ARD is linked to an MPPT by a cleavable linker region. In another embodiment of the invention, the cleavable linker region is a protease-cleavable linker, although other linkers, cleavable for example by small molecules, may be used. Examples of protease cleavage sites are those cleaved by factor Xa, thrombin and collagenase In one embodiment of the invention, the protease cleavage site is one that is cleaved by a protease that is up-regulated or associated with cancers in general. Examples of such proteases are uPA, the matrix metalloproteinase (MMP) family, the caspases, elastase, and the plasminogen activator family, as well as fibroblast activation protein In still another embodiment, the cleavage site is cleaved by a protease secreted by cancer-associated cells. Examples of these proteases include matrix metalloproteases, elastase, plasmin, Thrombin, and uPA In another embodiment, the protease cleavage site is one that is up-regulated or associated with a specific cancer Various cleavage sites recognised by proteases are known in the art and the skilled person will have no difficulty in selecting a suitable cleavage site. Non-limiting examples of cleavage sites are provided in Table 3. Other examples include, but are not limited to, a protease cleavage site targeted by Factor Xa: IEGR (SEQ ID NO:57); a protease cleavage site targeted by Enterokinase is DDDDK (SEQ ID NO:58); and a protease cleavage site targeted by Thrombin is LVPRG (SEQ ID NO:59). As is known in the art, other protease cleavage sites recognized by these proteases can also be used In one embodiment, the cleavable linker region is one which is targeted by endocellular proteases

As known in the art, the attachment of a linker to a MPPT (or of a linker element to an ARD, or an ARD to a MPPT) need not be a particular mode of attachment or reaction Various reactions providing a product of suitable stability and biological compatibility is acceptable.

### 1.4. Other modifications

The present invention contemplates further modification of MPPTs that do not affect the ability of the MPPTs to selectively target cancer cells Such modifications include amino acid substitutions, insertions or deletions, and modifications, for example, to reduced antigenicity of the MPPT, to enhance the stability of the MPPT and/or to improve the pharmacokinetics of the MPPTs In one embodiment, further modifications to MPPTs result in a polypeptide that differs by only a small number of amino acids from the MPPT Such modifications include deletions (for example of 1-3 or more amino acids), insertions (for example of 1-3 or more residues), or substitutions that do not interfere with the ability of the MPPTs to selectively target and kill cancer cells In one embodiment, further modifications to the MPPTs result in a polypeptide that retains at least 70%, 80%, 85%, 90%, 95%, 98%, or greater sequence identity to the MPPT and maintains the ability of the MPPT to selectively target and kill cancer cells.

MPPTs may be modified by substitution whereby at least one residue in the amino acid sequence has been removed and a different residue inserted in its place. In one embodiment, the substitution is a conservative substitution. A conservative substitution is one in which one or more amino acids (for example 2, 5 or 10 residues) are substituted with amino acid residues having similar biochemical properties. Typically, conservative substitutions have little to no impact on the activity of a resulting polypeptide. For example, ideally, an MPPT including one or more conservative substitutions retains the activity of the corresponding nPPT. Examples of amino acids which may be substituted for an original amino acid in a protein and which are regarded as conservative substitutions include: Ser for Ala; Lys for Arg; Gln or His for Asn; Glu for Asp; Ser for Cys; Asn for Gln; Asp for Glu; Pro for Gly; Asn or Gln for His; Leu or Val for Ile; Ile or Val for Leu; Arg or Gln for Lys; Leu or Ile for Met; Met, Leu or Tyr for Phe; Thr for Ser; Ser for Thr; Tyr for Trp; Trp or Phe for Tyr; and Ile or Leu for Val.

In another embodiment the substitution is a permissive substitution Permissive substitutions are non-conservative amino acid substitutions, but also do not significantly alter MPP activity An example is substitution of Cys for Ala at position 300 of SEQ ID NO: 2 in a proaerolysin polypeptide Other non-conservative substitutions that do not affect the activity of the MPPT can be readily determined by the skilled technician

An MPPT can be modified to include one or more substitutions by manipulating the nucleotide sequence that encodes that polypeptide using, for example, standard procedures such as site-directed mutagenesis or PCR Further information about substitutions can be found in, among other locations, Ben-Bassat et al, (J Bacteriol 169:751-7, 1987), O'Regan et al., (Gene 77:237-51, 1989), Sahin-Toth et al., (Protein Sci 3:240-7, 1994), Hochuli et al., (Bio/Technology 6:1321-5, 1988), WO 00/67796 (Curd et al) and in standard textbooks of genetics and molecular biology

In one embodiment, MPPTs are modified to include 1 or more amino acid substitutions of single residues In another embodiment, the MPPTs are modified to include 1 amino acid substitution In another embodiment, the MPPTs are modified to include from about 2 to about 10 amino acid substitutions In another embodiment, the MPPTs are modified to include about 3 to about 5 amino acid substitutions

Non-limiting examples of further modifications that may be made to MPPTs derived from proaerolysin in various embodiments of' the invention include substitutions at one or more of positions 22, 107, 114, 121, 127, 135, 159, 164, 171, 186, 198, 201, 202, 203, 216, 220, 238, 248, 249, 250, 252, 253, 254, 256, 258, 259, 263, 284, 285, 293, 294, 296, 299, 300, 309, 332, 341, 349, 361, 369, 371, 372, 373, 416, 417, 418, 445 and 449. Specific non-limiting examples are listed in Table 4

**Table 4: Exemplary single mutations of MPPs derived from a native proaerolysin polypeptide**

| | | | | |
|---|---|---|---|---|
| H107N | G202C | G251C | T284C | H341N |
| K22C | H121N | W203C | E252C | V285C |
| W127T | T253S | V293C | K361C | N459C |
| C164S | D216C | T253C | K294C | K369Q |
| Q254C | K294Q | W371L | D372N | I445C |
| Y135A | R220Q | E296C | K299C | K349C |
| Y135F | K171C | K238C | W373L | A418C |
| K22C | A300C | S256C | K309C | H332N |
| H186N | P248C | E258C | I416C | Q263C |
| K198C | L249C | I259C | G417C | |
| K114C | C159S | V201C | V250C | |

Peptidomimetic and organomimetic embodiments are also contemplated, whereby the three-dimensional arrangement of the chemical constituents of' such peptido- and organomimetics mimic the three-dimensional arrangement of the polypeptide backbone and component amino acid side chains in the polypeptide, resulting in such peptido- and organomimetics of an MPPT which have the ability to lyse cancer cells For computer modeling applications, a pharmacophore is an idealized, three-dimensional definition of the structural requirements for biological activity. Peptido- and organomimetics can be designed to fit each pharmacophore with current computer modeling software (using computer assisted drug design or CADD) See Walters, "Computer-Assisted Modeling of Drugs", in Klegerman & Groves, eds , 1993, Pharmaceutical Biotechnology, Interpharm Press: Buffalo Grove, Ill , pp 165-174 and Principles of Pharmacology (ed Munson, 1995), chapter 102 for a description of techniques used in CADD

Other modifications that may be made to the MPPTs include, for example, modifications to the carboxylic acid groups of the MPPT, whether carboxyl-terminal or side chain, in which these groups are in the form of a salt of a pharmaceutically-acceptable cation or esterified to form a C₁-C₁₆ ester, or converted to an amide of formula NR₁R₂ wherein R₁ and R₂ are each independently H or C₁-C₁₆ alkyl, or combined to form a heterocyclic ring, such as a 5- or 6-membered ring Amino groups of the polypeptide, whether amino-terminal or side chain, can be in the form of a pharmaceutically-acceptable acid addition salt, such as the HCl, HBr, acetic, benzoic, toluene sulfonic, maleic, tartaric and other organic salts, or may be modified to C₁-C₁₆ alkyl or dialkyl amino or further converted to an amide.

Other modifications include conversion of hydroxyl groups of the polypeptide side chain to C₁-C₁₆ alkoxy or to a C₁-C₁₆ ester using well-recognized techniques. Phenyl and phenolic rings of the polypeptide side chain can be substituted with one or more halogen atoms, such as F, Cl, Br or I, or with C₁-C₁₆ alkyl, C₁-C₁₆ alkoxy, carboxylic acids and esters thereof, or amides of such carboxylic acids Methylene groups of the polypeptide side chains can be extended to homologous C₂-C₄ alkylenes Thiols can be protected with any one of a number of well-recognized protecting groups, such as acetamide groups Those skilled in the art will also recognize methods for introducing cyclic structures into the polypeptides described herein to select and provide conformational constraints to the structure that result in enhanced stability For example, a carboxyl-terminal or amino-terminal cysteine residue can be added to the polypeptide, so that when oxidized the polypeptide will contain a disulfide bond, generating a cyclic peptide. Other peptide cyclizing methods include the formation of thioethers and carboxyl- and amino-terminal amides and esters.

The present invention further contemplates that the MPPT can comprise further modifications intended to improve the pharmacokinetic properties of' the molecule when administered to a subject Various modifications to reduce immunogenicity and/or improve the half-life of therapeutic proteins are known in the art For example, the MMPTs can undergo glycosylation, isomerization, or deglycosylation according to standard methods known in the art Similarly, the MPPT can be modified by non-naturally occurring covalent modification for example by addition of polyethylene glycol moieties (pegylation) or lipidation. In one embodiment, the MPPTs of the invention are conjugated to polyethylene glycol (PEGylated) to improve their pharmacokinetic profiles. Conjugation can be carried out by techniques known to those skilled in the art (see, for example, Deckert et al Int. J. Cancer 87: 382-390, 2000; Knight et al, Platelets 15: 409-418, 2004; Leong et al., Cytokine 16: 106-119, 2001; and Yang et al., Protein Eng. 16: 761-770, 2003) In one embodiment, antigenic epitopes can be identified and altered by mutagenesis Methods of identifying antigenic epitopes are known in the art (see for example, Sette et al., Biologicals 29:271-276), as are methods of mutating such antigenic epitopes

### 2. Preparation of Modified Pore-forming Protein Toxins

Modified pore-forming protein toxins (MPPTs) according to the present invention can be prepared by many methods, as known in the art Modifications to the MPPT can be made, for example, by engineering the nucleic acid encoding the MPPT using recombinant DNA technology Alternatively, modifications to the MPPT may be made by modifying the MPPT polypeptide itself, using chemical modifications and/or limited proteolysis. Combinations of these methods may also be used to prepare the MPPTs according to the present invention, as is also known in the art.

### 2.1 Preparation of MPPTs using recombinant methods

As is known in the art, genetic engineering of a protein using recombinant DNA technology generally requires that the nucleic acid encoding the protein first be isolated and cloned. Sequences for various nPPTs are available from GenBank™ as noted herein. Isolation and cloning of' the nucleic acid sequence encoding these proteins can thus be achieved using standard techniques [see, for example, Ausubel et al., Current Protocols in Molecular Biology, Wiley & Sons, NY (1997 and updates); Sambrook *et al.,* supra]. For example, the nucleic acid sequence can be obtained directly from a suitable organism, such as *Aeromonas hydrophila,* by extracting the mRNA by standard techniques and then synthesizing cDNA from the mRNA template (for example, by RT-PCR) or by PCR-amplifying the gene from genomic DNA Alternatively, the nucleic acid sequence encoding the nPPT can be obtained from an appropriate cDNA library by standard procedures. The isolated cDNA is then inserted into a suitable vector One skilled in the art will appreciate that the precise vector used is not critical to the instant invention Examples of suitable vectors include, but are not limited to, plasmids, phagemids, cosmids, bacteriophage, baculoviruses, retroviruses or DNA viruses The vector may be a cloning vector or it may be an expression vector.

Once the nucleic acid sequence encoding the nPPT has been obtained, mutations in either the binding domains or activation sequence can be introduced at specific, pre-selected locations by *in vitro* site-directed mutagenesis techniques well-known in the art Mutations can be introduced by deletion, insertion, substitution, inversion, or a combination thereof, of one or more of the appropriate nucleotides making up the coding sequence. This can be achieved, for example, by PCR based techniques for which primers are designed that incorporate one or more nucleotide mismatches, insertions or deletions The presence of the mutation can be verified by a number of standard techniques, for example by restriction analysis or by DNA sequencing

If desired, after introduction of the appropriate mutation or mutations, the nucleic acid sequence encoding the MPPT can be inserted into a suitable expression vector Examples of suitable expression vectors include, but are not limited to, plasmids, phagemids, cosmids, bacteriophages, baculoviruses and retroviruses, and DNA viruses.

One skilled in the art will understand that the expression vector may further include regulatory elements, such as transcriptional elements, required for efficient transcription of the MPPT-encoding sequences Examples of regulatory elements that can be incorporated into the vector include, but are not limited to, promoters, enhancers, terminators, and polyadenylation signals. The present invention, therefore, provides vectors comprising a regulatory element operatively linked to a nucleic acid sequence encoding a genetically engineered MPPT. One skilled in the art will appreciate that selection of suitable regulatory elements is dependent on the host cell chosen for expression of' the genetically engineered MPPT and that such regulatory elements may be derived from a variety of sources, including bacterial, fungal, viral, mammalian or insect genes

In the context of the present invention, the expression vector may additionally contain heterologous nucleic acid sequences that facilitate the purification of the expressed MPPT Examples of such heterologous nucleic acid sequences include, but are not limited to, affinity tags such as metal-affinity tags, histidine tags, avidin / streptavidin encoding sequences, glutathione-S-transferase (GST) encoding sequences and biotin encoding sequences. The amino acids corresponding to expression of the nucleic acids can be removed from the expressed MPPT prior to use according to methods known in the art. Alternatively, the amino acids corresponding to expression of heterologous nucleic acid sequences can be retained on the MPPT, providing that they do not interfere with the ability of the MPPT to target and kill cancer cells

In one embodiment of the invention, the MPPT is expressed as a histidine tagged protein. The histidine tag is located at the carboxyl terminus of the MPPT.

The expression vectors can be introduced into a suitable host cell or tissue by one of a variety of methods known in the art Such methods can be found generally described in Ausubel et al., Current Protocols in Molecular Biology, Wiley & Sons, NY (1997 and updates); Sambrook et al., Molecular Cloning: A Laboratory Manual, Cold-Spring Harbor Press, NY (2001) and include, for example, stable or transient transfection, lipofection, electroporation, and infection with recombinant viral vectors One skilled in the art will understand that selection of the appropriate host cell for expression of the MPP will be dependent upon the vector chosen Examples of host cells include, but are not limited to, bacterial, yeast, insect, plant and mammalian cells.

In addition, a host cell may be chosen which modulates the expression of the inserted sequences, or modifies and processes the gene product in a specific, desired fashion Such modifications (e.g , glycosylation) and processing (e.g., cleavage) of protein products may be important for the function of' the protein Different host cells have characteristic and specific mechanisms for the post-translational processing and modification of proteins and gene products Appropriate cell lines or host systems can be chosen to ensure the correct modification and processing of' the foreign protein expressed To this end, eukaryotic host cells that possess the cellular machinery for proper processing of the primary transcript, and for post-translational modifications such as glycosylation and phosphorylation of the gene product can be used Such mammalian host cells include, but are not limited to, CHO, VERO, BHK, HeLa, COS, MDCK, 293, 3T3, WI38.

Methods of cloning and expressing proteins are well known in the art, detailed descriptions of techniques and systems for the expression of recombinant proteins can be found, for example, in Current Protocols in Protein Science (Coligan, J.E., et al., Wiley & Sons, New York) Those skilled in the field of molecular biology will understand that a wide variety of expression systems can be used to provide the recombinant protein The precise host cell used is not critical to the invention Accordingly, the present invention contemplates that the MPPTs can be produced in a prokaryotic host (e g., *E. coli, A salmonicida* or *B. subtilis*) or in a eukaryotic host (e.g., *Saccharomyces* or *Pichia;* mammalian cells, e.g., COS, NIH 3T3, CHO, BHK, 293, or HeLa cells; or insect cells).

The MPPTs can be purified from the host cells by standard techniques known in the art. If desired, the changes in amino acid sequence engineered into the protein can be determined by standard peptide sequencing techniques using either the intact protein or proteolytic fragments thereof

As an alternative to a directed approach to introducing mutations into naturally occurring pore-forming proteins, a cloned gene expressing a pore-forming protein can be subjected to random mutagenesis by techniques known in the art. Subsequent expression and screening of the mutant forms of the protein thus generated would allow the identification and isolation of MPPTs according to the present invention.

The MPPTs according to the present invention can also be prepared as fragments or fusion proteins. A fusion protein is one which includes an MPPT linked to other amino acid sequences that do not inhibit the ability of the MPPT to selectively target and kill normal cancer cells In one embodiment, the other amino acid sequence encodes an ARD. In an alternative embodiment, the other amino acid sequences are short sequences of, for example, up to about 5, about 6, about 7, about 8, about 9, about 10, about 20, about 30, about 50 or about 100 amino acid residues in length

Methods for making fusion proteins are well known to those skilled in the art For example U.S. Pat No 6,057,133 discloses methods for making fusion molecules composed of human interleukin-3 (hIL-3) variant or mutant proteins functionally joined to a second colony stimulating factor, cytokine, lymphokine, interleukin, hematopoietic growth factor or IL-3 variant U.S. Pat No. 6,072,041 to Davis et al discloses the generation of fusion proteins comprising a single chain Fv molecule directed against a transcytotic receptor covalently linked to a therapeutic protein

Similar methods can be used to generate fusion proteins comprising MPPTs (or variants, fragments, etc, thereof) linked to other amino acid sequences, such as the ARDs described herein Linker regions can be used to space the two portions of the protein from each other and to provide flexibility between them The linker region is generally a polypeptide of between 1 and 500 amino acids in length, for example less than 30 amino acids in length In general, the linker joining the two molecules can be designed to (1) allow the two molecules to fold and act independently of each other, (2) not have a propensity for developing an ordered secondary structure which could interfere with the functional domains of the two proteins, (3) have minimal hydrophobic or charged characteristic which could interact with the functional protein domains and/or (4) provide steric separation of the two regions. Typically surface amino acids in flexible protein regions include Gly, Asn and Ser. Other neutral amino acids, such as The and Ala, can also be used in the linker sequence. Additional amino acids can be included in the linker to provide unique restriction sites in the linker sequence to facilitate construction of' the fusions Other moieties can also be included, as desired. These can include a binding region, such as avidin or an epitope, or a tag such as a polyhistidine tag, which can be useful for purification and processing of the fusion protein. In addition, detectable markers can be attached to the fusion protein, so that the traffic of the fusion protein through a body or cell can be monitored conveniently Such marker include radionuclides, enzymes, fluorophores, and the like

Fusing of the nucleic acid sequences of the MPPT with the nucleic acid sequence of another protein (or variant, fragment etc thereof), can be accomplished by the use of intermediate vectors. Alternatively, one gene can be cloned directly into a vector containing the other gene. Linkers and adapters can be used for joining the nucleic acid sequences, as well as replacing lost sequences, where a restriction site was internal to the region of interest. Genetic material (DNA) encoding one polypeptide, peptide linker, and the other polypeptide is inserted into a suitable expression vector which is used to transform prokaryotic or eukaryotic cells, for example bacteria, yeast, insect cells or mammalian cells The transformed organism is grown and the protein isolated by standard techniques, for example by using a detectable marker such as nickel-chelate affinity chromatography, if a polyhistidine tag is used. The resulting product is therefore a new protein, a fusion protein, which has the MPPT joined to a second protein, optionally via a linker. To confirm that the fusion protein is expressed, the purified protein can be, for example, subjected to electrophoresis in SDS-polyacrylamide gels, and transferred onto nitrocellulose membrane filters using established methods The protein products can be identified by Western blot analysis using antibodies directed against the individual components, i.e., polyhistidine tag and/or the MPPT

If the MPPTs according to the present invention are produced by expression of a fused gene, a peptide bond serves as the linker between the MPPT and the ARD For example, a recombinant fusion protein of a single chain Fv fragment of an antibody and a pore-forming protein toxin can be made according to methods known in the art, e.g., Huston et al., Meth Enzymol 203:46-88, 1991.

One of ordinary skill in the art will appreciate that the DNA can be altered in numerous ways without affecting the biological activity of the encoded protein. For example, PCR can be used to produce variations in the DNA sequence which encodes an MPPT Such variations in the DNA sequence encoding an MPPT can be used to optimize for codon preference in a host cell used to express the protein, or may contain other sequence changes that facilitate expression

### 2.2 Other methods of preparing MPPTs

The ARDs and linkers noted above may be added to the MPPTs of the present invention via a covalent or non-covalent bond, or both Non-covalent interactions can be ionic, hydrophobic, or hydrophilic, such as interactions involved in a leucine-zipper or antibody-Protein G interaction (Derrick et al., Nature 359:752, 1992). Examples of additional non-covalent interactions include but are not restricted to the following binding pairs: antigen or hapten with antibody; antibody with anti-antibody; receptor with ligand; enzyme or enzyme fragment with substrate, substrate analogue or ligand; biotin or lectin with avidin or streptavidin; lectin with carbohydrate; pairs of leucine zipper motifs (see, for example, U.S. Patent No. 5,643,731), as well as various homodimers and heterodimers known in the art As is known in the art, the MPPT may be modified to include one member of the binding pair, and the ARD or linker may be modified to include the other member of the binding pair

A covalent linkage may take various forms as is known in the art. For example, the covalent linkage may the form of a disulfide bond The DNA encoding one of the components can be engineered to contain a unique cysteine codon. The second component can be derivatized with a sulfhydryl group reactive with the cysteine of the first component. Alternatively, a sulfhydryl group, either by itself or as part of a cysteine residue, can be introduced using solid phase polypeptide techniques For example, the introduction of sulfhydryl groups into peptides is described by Hiskey (Peptides 3:137, 1981).

Proteins can be chemically modified by standard techniques to add a sulfhydryl group For example, Traut's reagent (2-iminothiolane-HCl) (Pierce Chemicals, Rockford, Ill.) can be used to introduce a sulfhydryl group on primary amines, such as lysine residues or N-terminal amines A protein or peptide modified with Traut's reagent can then react with a protein or peptide which has been modified with reagents such as N-succinimidyl 3-(2-pyridyldithio) propionate (SPDP) or succinimidyl 4-(N-maleimidomethyl)cyclohexane-1-carboxylate (SMCC) (Pierce Chemicals, Rockford, III)

Once the correct sulfhydryl groups are present on each component, the two components are purified, sulfur groups on each component are reduced; the components are mixed; and disulfide bond formation is allowed to proceed to completion at room temperature To improve the efficiency of the coupling reaction, the cysteine residue of one of the components, eg, cysteine-MPPT, can be activated prior to addition to the reaction mixture with 5,5'-dithiobis(2-nitrobenzoic) acid (DTNB) or 2,2'-dithiopyridine, using methods known in the art. Following the reaction, the mixture is dialyzed against phosphate buffered saline to remove unconjugated molecules Sephadex chromatography or the like is then carried out to separate the compound of the invention from its constituent parts on the basis of size.

The components can also be joined using the polymer, monomethoxy-polyethylene glycol (mPEG), as described in Maiti et al., Int J. Cancer Suppl. 3:17-22, 1988

The ARD and the nPPT or MPPT can also be conjugated through the use of standard conjugation chemistries as is known in the art, such as, carbodiimide-mediated coupling (for example, DCC, EDC or activated EDC), and the use of 2,-iminothiolane to convert epsilon amino groups to thiols for crosslinking and m-maleimidobenzoyl-n-hydroxysuccinimidyl ester (MBS) as a crosslinking agent. Various other methods of conjugation known in the art can be employed to join the ARD and the nPPT or MPPT.

### 2.3 Large Scale Preparation of MPPTs

The preparation of the MPPTs can also be conducted on a large scale, for example for manufacturing purposes, using standard techniques known in the art, such as large scale fermentation processes for production of recombinant proteins, and ultrafiltration, ion exchange chromatography, immobilized metal ion affinity chromatography for purification of recombinant proteins

### 3. Testing of Modified Pore-forming Protein Toxins

The MPPTs according to the present invention retain their pore-forming activity, are activated by general or specific activating agents, and thus, are able to kill cancer cells The ability of the MPPTs according to the present invention to kill cancer cells can be tested using standard techniques known in the art Exemplary methods of' testing candidate MPPTs are provided below and in the Examples included herein One skilled in the art will understand that other methods of testing the MPPTs are known in the art and are also suitable for testing candidate MPPTs.

### 3.1 In vitro methods

MPPTs according to the present invention that contain one or more modifications to the activation sequence can be tested for their ability to be cleaved by the appropriate activating agent according to methods known in the art. For example, if the one or more modifications result in the addition of one or more protease cleavage sites, the MPPT can be incubated with varying concentrations of the appropriate protease(s) The incubation products can be electrophoresed on SDS-PAGE gels and cleavage of the MPPT can be assessed by examining the size of the polypeptide on the gel.

In order to determine if the MPPTs that have been incubated with protease retain pore-forming activity, and thus the ability to kill cells, after incubation with the protease, the reaction products can be tested in a hemolysis assay as is known in the art An example of a suitable assay is described in Howard, S.P., and Buckley, J.T. 1985. Activation of the hole-forming toxin aerolysin by extracellular processing. J Bacteriol. 163:336-340.

MPPTs according to the present invention can be tested for their ability to kill cancer cells as is known in the art For example, the ability of the MPPTs to kill cells can be assayed *in vitro* using a suitable cell line, typically a cancer cell line. In general, cells of the selected test cell line are grown to an appropriate density and the candidate MPPT is added After an appropriate incubation time (for example, about 48 to 72 hours), cell survival is assessed +Methods of determining cell survival are well known in the art and include, but are not limited to, the resazurin reduction test (see Fields & Lancaster (1993) Am Biotechnol. Lab 11:48-50; O'Brien et al., (2000) Eur. J. Biochem. 267:5421-5426 and U.S. Patent No. 5,501,959), the sulforhodamine assay (Rubinstein et al., (1990) J. Natl Cancer Inst. 82:113-118) or the neutral red dye test (Kitano et al., (1991) Euro. J Clin. Investg. 21:53-58; West et al., (1992) J. Investigative Derm 99:95-100) or trypan blue assay. Numerous commercially available kits may also be used, for example the CellTiter 96® AQueous One Solution Cell Proliferation Assay (Promega) Cytotoxicity is determined by comparison of' cell survival in the treated culture with cell survival in one or more control cultures, for example, untreated cultures and/or cultures pre-treated with a control compound (typically a known therapeutic), or other appropriate control. MPPTs considered to be effective in killing cancer cells are capable of decreasing cell survival, for example, by at least 10%, at least 20%, at least 30%, at least 40%, or at least 50%

MPPTs comprising optional modifications that confer selectivity for a specific type of cancer may be tested for their ability to target that specific cancer cell type For example, an MPPT comprising an ARD that targets the MPPT to lung cancer cells can be assessed for its ability to selectively target lung cancer cells by comparing the ability of the MPPT to kill lung cancer cells to its ability to kill a normal cell, or a different type of cancer cell Alternatively, flow cytometric methods, as are known in the art, may be used to determine if an MPPT comprising an ARD is able to selectively target a specific type of cancer cell.

Furthermore, MPPTs comprising one or more general cleavage sites, or a plurality of specific cleavage sites can be tested for their ability to be activated by the appropriate general or specific activating agent associated with cancer cells. As an example, the MPPT comprising a general cleavage site recognized by uPA can be incubated with a cancer cell that is known to express or activate uPA, followed by analysis of resulting cell death, usually expressed as cell killing curves. Non-limiting examples of cell lines that may be used to determine the ability of MPPTs to kill cancer cells associated with uPA include A2058 cells and HeLa cells, An example of a cell line expressing MMP2 is HT1080 In these types of experiments, EL4 cells can be used as a control cell line which is not associated with uPA or MMP2

A variety of cancer cell-lines suitable for testing the candidate MPPTs are known in the art and many are commercially available (for example, from the American Type Culture Collection, Manassas, VA). In one embodiment of the present invention, *in vitro* testing of the candidate compounds is conducted in a human cancer cell-line In one embodiment of the present invention, *in vitro* testing of MPPTs is conducted in a human cancer cell-line. Examples of suitable cancer cell-lines for *in vitro* testing include, but are not limited to, mesothelial cell lines MSTO-211H, NCI-H2052 and NCI-H28, ovarian cancer cell-lines OV90 and SK-OV-3, breast cancer cell-lines MCF-7 and MDA-MB-231, colon cancer cell-lines CaCo, HCT116 and HT29, cervical cancer cell-line HeLa, non-small cell lung carcinoma cell-lines A549 and H1299, pancreatic cancer cell-lines MIA-PaCa-2 and AsPC-1, prostatic cancer-cell line PC-3, bladder cancer cell-line T24, liver cancer cell-lineHepG2, brain cancer cell-line U-87 MG, melanoma cell-line A2058, lung cancer cell-line NCI-H460. Other examples of' suitable cell-lines are known in the art and include the EL4 mouse lymphoma cell line.

If necessary, the toxicity of the MPPTs to non-cancerous cells can also be initially assessed *in vitro* using standard techniques For example, human primary fibroblasts can be transfected *in vitro* with the MPPTs and then tested at different time points following treatment for their viability using a standard viability assay, such as the assays described above, or the trypan-blue exclusion assay Cells can also be assayed for their ability to synthesize DNA, for example, using a thymidine incorporation assay, and for changes in cell cycle dynamics, for example, using a standard cell sorting assay in conjunction with a fluorescence activated cell sorter (FACS).

### 3.2 In vivo methods

The ability of' the MPPTs to kill tumor cells *in vivo* can be determined in an appropriate animal model using standard techniques known in the art (see, for example, Enna, et al., Current Protocols in Pharmacology, J. Wiley & Sons, Inc., New York, NY)

Current animal models for screening anti-tumor compounds include xenograft models, in which a human tumor has been implanted into an animal Examples of xenograft models of human cancer include, but are not limited to, human solid tumor xenografts, implanted by sub-cutaneous injection or implantation and used in tumor growth assays; human solid tumor isografts, implanted by fat pad injection and used in tumor growth assays; human solid tumor orthotopic xenografts, implanted directly into the relevant tissue and used in tumor growth assays; experimental models of lymphoma and leukemia in mice, used in survival assays, and experimental models of lung metastasis in mice. In addition to the implanted human tumor cells, the xenograft models can further comprise transplanted human peripheral blood leukocytes, which allow for evaluation of the anti-cancer immune response

Alternatively, murine cancer models can be used for screening anti-tumor compounds. Examples of appropriate murine cancer models are known in the art and include, but are not limited to, implantation models in which murine cancer cells are implanted by intravenous, subcutaneous, fat pad or orthotopic injection; murine metastasis models; transgenic mouse models; and knockout mouse models.

For example, the MPPTs can be tested *in vivo* on solid tumors using mice that are subcutaneously grafted bilaterally with 30 to 60 mg of a tumor fragment, or implanted with an appropriate number of cancer cells, on day 0. The animals bearing tumors are mixed before being subjected to the various treatments and controls. In the case of treatment of advanced tumors, tumors are allowed to develop to the desired size, animals having insufficiently developed tumors being eliminated. The selected animals are distributed at random to undergo the treatments and controls. Animals not bearing tumors may also be subjected to the same treatments as the tumor-bearing animals in order to be able to dissociate the toxic effect from the specific effect on the tumor. Chemotherapy generally begins from 3 to 22 days after grafting, depending on the type of' tumor, and the animals are observed every day The MPPTs of the present invention can be administered to the animals, for example, by i p injection, intravenous injection, direct injection into the tumor, or bolus infusion The different animal groups are weighed about 3 or 4 times a week until the maximum weight loss is attained, after which the groups are weighed at least once a week until the end of the trial.

The tumors are measured after a pre-determined time period, or they can be monitored continuously by measuring about 2 or 3 times a week until the tumor reaches a predetermined size and / or weight, or until the animal dies if this occurs before the tumor reaches the pre-determined size / weight The animals are then sacrificed and the tissue histology, size and / or proliferation of the tumor assessed

Orthotopic xenograft models are an alternative to subcutaneous models and may more accurately reflect the cancer development process In this model, tumor cells are implanted at the site of the organ of origin and develop internally Daily evaluation of the size of the tumors is thus more difficult than in a subcutaneous model A recently developed technique using green fluorescent protein (GFP) expressing tumors in non-invasive whole-body imaging can help to address this issue (Yang et al, Proc Nat. Aca Sci, (2000), pp 1206-1211). This technique utilises human or murine tumors that stably express very high levels of the *Aqueora vitoria* green fluorescent protein The GFP expressing tumors can be visualised by means of externally placed video detectors, allowing for monitoring of details of tumor growth, angiogenesis and metastatic spread. Angiogenesis can be measured over time by monitoring the blood vessel density within the tumor(s) The use of this model thus allows for simultaneous monitoring of several features associated with tumor progression and has high preclinical and clinical relevance

For the study of the effect of the compositions on leukemias, the animals are grafted with a particular number of cells, and the anti-tumor activity is determined by the increase in the survival time of the treated mice relative to the controls

To study the effect of the MPPTs of the present invention on tumor metastasis, tumor cells are typically treated with the composition *ex vivo* and then injected into a suitable test animal The spread of the tumor cells from the site of injection is then monitored over a suitable period of time

*In vivo* toxic effects of the MPPTs can be evaluated by measuring their effect on animal body weight during treatment and by performing hematological profiles and liver enzyme analysis after the animal has been sacrificed

**Table 5: Examples of xenograft models of human cancer**

| Cancer Model | Cell Type |
|---|---|
| Tumor Growth Assay | Prostate (PC-3, DU145) |
| Human solid tumor xenografts in mice (sub-cutaneous injection) | Breast (MDA-MB-231, MVB-9) |
| | Colon (HT-29) |
| | Lung (NCI-H460, NCI-H209) |
| | Pancreatic (ASPC-1, SU86 86) |
| | Pancreatic: drug resistant (BxPC-3) |
| | Skin (A2058, C8161) |
| | Cervical (SIHA, HeLa-S3) |
| | Cervical: drug resistant (HeLa S3-HU-resistance) |
| | Liver (HepG2) |
| | Brain (U87-MG) |
| | Renal (Caki-1, A498) |
| | Ovary (SK-OV-3) |
| Tumor Growth Assay | Breast: drug resistant (MDA-CDDP-S4, MDA-MB435-To.1) |
| Human solid tumor isografts in mice (fat pad injection) | |
| Survival Assay | Human: Burkitts lymphoma (Non-Hodgkin's) (raji) |
| Experimental model of lymphoma and leukemia in mice | Murine: erythroleukemia (CB7 Friend retrovirus-induced) |
| Experimental model of lung metastasis in mice | Human: melanoma (C8161) |
| | Murine: fibrosarcoma (R3) |

### 3 2.1 General toxicity

The general toxicity of the MPPTs according to the present invention can be tested according to methods known in the art. For example, the overall systemic toxicity of' the MPPTs can be tested by determining the dose that kills 100% of mice (i e LD₁₀₀) following a single intravenous injection

### 3.3 Determination and Reduction of Antigenicity

Therapeutic proteins may elicit some level of antibody response when adminstered to a subject, which in some cases may lead to undesirable side effects Therefore, if necessary, the antigenicity of the MPPTs can be assessed as known in the art and described below In addition, methods to reduce potential antigenicity are described.

The kinetics and magnitude of the antibody response to the MPPTs described herein can be determined, for example, in immunocompetent mice and can be used to facilitate the development of a dosing regimen that can be used in a immunocompetent human. Immunocompetent mice such as the strain C57-BL6 are administered intravenous doses of MPPT. The mice are sacrificed at varying intervals (e.g., following single dose, following multiple doses)

To decrease antigenicity of MPPTs according to the present invention, the native binding domain of the MPPT can be functionally deleted and replaced, for example with an ARD as described above The antigenicity of such MPPTs can be determined following exposure to varying schedules of the MPPT which lack portions of the native binding domain using the methods described above. Another method that can be used to allow continued treatment with MPPTs is to use sequentially administered alternative MPPTs derived from other nPPTs with non-overlapping antigenicity. For example, an MPPT derived from proaerolysin can be used alternately with an MPPT derived from *Clostridium septicum* alpha toxin or *Bacillus thuringiensis* delta-toxin. All of these MPPTs would target cancer cells, but would not be recognized or neutralize by the same antibodies.

Serum samples from these mice can be assessed for the presence of anti-MPPT antibodies as known in the art. As another example, epitope mapping can also be used to determine antigenicity of proteins as described in Marcia M. Stickler, David A. Estell and Fiona A. Harding CD+ T cell epitope prediction using unexposed human donor peripheral blood mononuclear cells. J. Immunotherapy, 23(6):654-660, 2000. Briefly, immune cells known as dendritic cells and CD4+ T cells are isolated from the blood of community donors who have not been exposed to the protein of interest. Small synthetic peptides spanning the length of the protein are then added to the cells in culture Proliferation in response to the presence of a particular peptide suggests that a T cell epitope is encompassed in the sequence This peptide sequence can subsequently be deleted or modified in the MPPT thereby reducing its antigenicity

### 4. Pharmaceutical Compositions

The present invention provides for pharmaceutical compositions comprising one or more MPPTs and one or more non-toxic pharmaceutically acceptable carriers, diluents, excipients and/or adjuvants If desired, other active ingredients may be included in the compositions As indicated above, such compositions are suitable for use in the treatment of cancel The term "pharmaceutically acceptable carrier" refers to a carrier medium which does not interfere with the effectiveness of the biological activity of the active ingredients and which is not toxic to the host or patient Representative examples are provided below

The pharmaceutical compositions may comprise, for example, from about 1% to about 95% of a MPPT of the invention Compositions formulated for administration in a single dose form may comprise, for example, about 20% to about 90% of the MPPTs of the invention, whereas compositions that are not in a single dose form may comprise, for example, from about 5% to about 20% of the MPPTs of the invention. Concentration of the MPPT in the final formulation can be as low as 0.01 µg/mL. For example, the concentration in the final formulation can be between about 0 01 µg/mL and about 1,000 µg/mL In one embodiment, the concentration in the final formulation is between about 0.01 µg/mL and about 100 µg/mL Non-limiting examples of unit dose forms include dragées, tablets, ampoules, vials, suppositories and capsules, Non-limiting examples of unit dose forms include dragées, tablets, ampoules, vials, suppositories and capsules

The composition can be a liquid solution, suspension, emulsion, tablet, pill, capsule, sustained release formulation, or powder. For solid compositions (e.g., powder, pill, tablet, or capsule forms), conventional non-toxic solid carriers can include, for example, pharmaceutical grades of mannitol, lactose, starch, sodium saccharine, cellulose, magnesium carbonate, or magnesium stearate. The composition can be formulated as a suppository, with traditional binders and carriers such as triglycerides

For administration to an animal, the pharmaceutical compositions can be formulated for administration by a variety of routes For example, the compositions can be formulated for oral, topical, rectal or parenteral administration or for administration by inhalation or spray The term parenteral as used herein includes subcutaneous injections, intravenous, intramuscular, intrathecal, intrasternal injection or infusion techniques. Direct injection or infusion into a tumor is also contemplated Convection enhanced delivery, a standard administration technique for protein toxins, is also contemplated by the present invention. The MPPTs can be delivered along with a pharmaceutically acceptable vehicle In one embodiment, the vehicle may enhance the stability and/or delivery properties Thus, the present invention also provides for formulation of the MPPT with a suitable vehicle, such as an artificial membrane vesicle (including a liposome, noisome, nanosome and the like), microparticle or microcapsule, or as a colloidal formulation that comprises a pharmaceutically acceptable polymer The use of' such vehicles/polymers may be beneficial in achieving sustained release of the MPPTs. Alternatively, or in addition, the MPPT formulations can include additives to stabilise the protein *in vivo*, such as human serum albumin, or other stabilisers for protein therapeutics known in the art MPPT formulations can also include one or more viscosity enhancing agents which act to prevent backflow of the formulation when it is administered, for example by injection or via catheter. Such viscosity enhancing agents include, but are not limited to, biocompatible glycols and sucrose.

Pharmaceutical compositions for oral use can be formulated, for example, as tablets, troches, lozenges, aqueous or oily suspensions, dispersible powders or granules, emulsion hard or soft capsules, or syrups or elixirs. Such compositions can be prepared according to standard methods known to the art for the manufacture of' pharmaceutical compositions and may contain one or more agents selected from the group of sweetening agents, flavoring agents, colouring agents and preserving agents in order to provide pharmaceutically elegant and palatable preparations Tablets contain the active ingredient in admixture with suitable non-toxic pharmaceutically acceptable excipients including, for example, inert diluents, such as calcium carbonate, sodium carbonate, lactose, calcium phosphate or sodium phosphate; granulating and disintegrating agents, such as corn starch, or alginic acid; binding agents, such as starch, gelatine or acacia, and lubricating agents, such as magnesium stearate, stearic acid or talc The tablets can be uncoated, or they may be coated by known techniques in order to delay disintegration and absorption in the gastrointestinal tract and thereby provide a sustained action over a longer period For example, a time delay material such as glyceryl monostearate or glyceryl distearate may be employed

Pharmaceutical compositions for oral use can also be presented as hard gelatine capsules wherein the active ingredient is mixed with an inert solid diluent, for example, calcium carbonate, calcium phosphate or kaolin, or as soft gelatine capsules wherein the active ingredient is mixed with water or an oil medium such as peanut oil, liquid paraffin or olive oil.

Pharmaceutical compositions formulated as aqueous suspensions contain the active compound(s) in admixture with one or more suitable excipients, for example, with suspending agents, such as sodium carboxymethylcellulose, methyl cellulose, hydropropylmethylcellulose, sodium alginate, polyvinylpyrrolidone, hydroxypropyl-β-cyclodextrin, gum tragacanth and gum acacia; dispersing or wetting agents such as a naturally-occurring phosphatide, for example, lecithin, or condensation products of an alkylene oxide with fatty acids, for example, polyoxyethyene stearate, or condensation products of ethylene oxide with long chain aliphatic alcohols, for example, hepta-decaethyleneoxycetanol, or condensation products of ethylene oxide with partial esters derived from fatty acids and a hexitol for example, polyoxyethylene sorbitol monooleate, or condensation products of ethylene oxide with partial esters derived from fatty acids and hexitol anhydrides, for example, polyethylene sorbitan monooleate. The aqueous suspensions may also contain one or more preservatives, for example ethyl, or *n*-propyl *p*-hydroxybenzoate, one or more colouring agents, one or more flavoring agents or one of more sweetening agents, such as sucrose or saccharin.

Pharmaceutical compositions can be formulated as oily suspensions by suspending the active compound(s) in a vegetable oil, for example, arachis oil, olive oil, sesame oil or coconut oil, or in a mineral oil such as liquid paraffin. The oily suspensions may contain a thickening agent, for example, beeswax, hard paraffin or cetyl alcohol. Sweetening agents such as those set forth above, and/or flavoring agents may be added to provide palatable oral preparations. These compositions can be preserved by the addition of an anti-oxidant such as ascorbic acid

The pharmaceutical compositions can be formulated as a dispersible powder or granules, which can subsequently be used to prepare an aqueous suspension by the addition of' water Such dispersible powders or granules provide the active ingredient in admixture with one or more dispersing or wetting agents, suspending agents and/or preservatives. Suitable dispersing or wetting agents and suspending agents are exemplified by those already mentioned above. Additional excipients, for example, sweetening, flavoring and coloring agents, can also be included in these compositions

Pharmaceutical compositions of the invention can also be formulated as oil-in-water emulsions. The oil phase can be a vegetable oil, for example, olive oil or arachis oil, or a mineral oil, for example, liquid paraffin, or it may be a mixture of these oils Suitable emulsifying agents for inclusion in these compositions include naturally-occurring gums, for example, gum acacia or gum tragacanth; naturally-occurring phosphatides, for example, soy bean, lecithin; or esters or partial esters derived from fatty acids and hexitol, anhydrides, for example, sorbitan monoleate, and condensation products of the said partial esters with ethylene oxide, for example, polyoxyethylene sorbitan monoleate. The emulsions can also optionally contain sweetening and flavoring agents

Pharmaceutical compositions can be formulated as a syrup or elixir by combining the active ingredient(s) with one or more sweetening agents, for example glycerol, propylene glycol, sorbitol or sucrose. Such formulations can also optionally contain one or more demulcents, preservatives, flavoring agents and/or coloring agents.

The pharmaceutical compositions can be formulated as a sterile injectable aqueous or oleaginous suspension according to methods known in the art and using suitable one or more dispersing or wetting agents and/or suspending agents, such as those mentioned above The sterile injectable preparation can be a sterile injectable solution or suspension in a non-toxic parentally acceptable diluent or solvent, for example, as a solution in 1,3-butanediol. Acceptable vehicles and solvents that can be employed include, but are not limited to, water, Ringer's solution, lactated Ringer's solution and isotonic sodium chloride solution Other examples include, sterile, fixed oils, which are conventionally employed as a solvent or suspending medium, and a variety of bland fixed oils including, for example, synthetic mono- or diglycerides. Fatty acids such as oleic acid can also be used in the preparation of injectables.

In one embodiment, the MPPT is conjugated to a water-soluble polymer, e.g., to increase stability or circulating half life or reduce immunogenicity Clinically acceptable, water-soluble polymers include, but are not limited to, polyethylene glycol (PEG), polyethylene glycol propionaldehyde, carboxymethylcellulose, dextran, polyvinyl alcohol (PVA), polyvinylpyrrolidone (PVP), polypropylene glycol homopolymers (PPG), polyoxyethylated polyols (POG) (e.g., glycerol) and other polyoxyethylated polyols, polyoxyethylated sorbitol, or polyoxyethylated glucose, and other carbohydrate polymers. Methods for conjugating polypeptides to water-soluble polymers such as PEG are described, e.g, in U.S. patent Pub No. 20050106148 and references cited therein

Other pharmaceutical compositions and methods of preparing pharmaceutical compositions are known in the art and are described, for example, in "Remington: The Science and Practice of Pharmacy" (formerly "Remingtons Pharmaceutical Sciences"); Gennaro, A , Lippincott, Williams & Wilkins, Philidelphia, PA (2000)

The pharmaceutical compositions of' the present invention described above include one or more MPPTs of the invention in an amount effective to achieve the intended purpose Thus the term "therapeutically effective dose" refers to the amount of the MPPT that ameliorates the symptoms of cancer Determination of a therapeutically effective dose of' a compound is well within the capability of those skilled in the art. For example, the therapeutically effective dose can be estimated initially either in cell culture assays, or in animal models, such as those described herein Animal models can also be used to determine the appropriate concentration range and route of administration Such information can then be used to determine useful doses and routes for administration in other animals, including humans, using standard methods known in those of ordinary skill in the art

Therapeutic efficacy and toxicity can also be determined by standard pharmaceutical procedures such as, for example, by determination of the median effective dose, or ED₅₀ (*i.e* the dose therapeutically effective in 50% of the population) and the median lethal dose, or *LD₅₀* (*i.e*., the dose lethal to 50% of the population). The dose ratio between therapeutic and toxic effects is known as the "therapeutic index," which can be expressed as the ratio, LD₅₀/ED₅₀. The data obtained from cell culture assays and animal studies can be used to formulate a range of' dosage for human or animal use The dosage contained in such compositions is usually within a range of' concentrations that include the ED₅₀ and demonstrate little or no toxicity The dosage varies within this range depending upon the dosage form employed, sensitivity of the subject, and the route of administration and the like.

The exact dosage to be administered to a subject can be determined by the practitioner, in light of factors related to the subject requiring treatment. Dosage and administration are adjusted to provide sufficient levels of the MPPT and/or to maintain the desired effect. Factors which may be taken into account when determining an appropriate dosage include the severity of the disease state, general health of the subject, age, weight, and gender of the subject, diet, time and frequency of administration, drug combination(s), reaction sensitivities, and tolerance/response to therapy. Dosing regimens can be designed by the practitioner depending on the above factors as well as factors such as the half-life and clearance rate of the particular formulation.

### 5. Use of Modified Pore-forming Toxins

### 5.1 Cancers

The MPPTs of the present invention can be used to treat, stabilize or prevent cancer. In this context, the MPPTs may exert either a cytotoxic or cytostatic effect resulting in, for example, a reduction in the size of a tumor, the slowing or prevention of an increase in the size of a tumor, an increase in the disease-free survival time between the disappearance or removal of a tumor and its reappearance, prevention of an initial or subsequent occurrence of a tumor (*e g*. metastasis), an increase in the time to progression, reduction of one or more adverse symptom associated with a tumor, or an increase in the overall survival time of a subject having cancer

Examples of cancers which may be may be treated or stabilized in accordance with the present invention include, but are not limited to, hematologic neoplasms, including leukemias, myelomas and lymphomas; carcinomas, including adenocarcinomas and squamous cell carcinomas; melanomas and sarcomas. Carcinomas and sarcomas are also frequently referred to as "solid tumors," examples of' commonly occuning solid tumors include, but are not limited to, cancer of the brain, breast, cervix, colon, head and neck, kidney, lung, ovary, pancreas, prostate, stomach and uterus, non-small cell lung cancer and colorectal cancer Various forms of lymphoma also may result in the formation of a solid tumor and, therefore, are also often considered to be solid tumors In one embodiment of the present invention, the MPPTs are used to treat a solid tumor.

The term "leukemia" refers broadly to progressive, malignant diseases of the blood-forming organs. Leukemia is typically characterized by a distorted proliferation and development of leukocytes and their precursors in the blood and bone marrow but can also refer to malignant diseases of other blood cells such as erythroleukemia, which affects immature red blood cells. Leukemia is generally clinically classified on the basis of (1) the duration and character of the disease - acute or chronic; (2) the type of cell involved -- myeloid (myelogenous), lymphoid (lymphogenous) or monocytic, and (3) the increase or non-increase in the number of abnormal cells in the blood - leukaemic or aleukaemic (subleukaemic) Leukemia includes, for example, acute nonlymphocytic leukemia, chronic lymphocytic leukemia, acute granulocytic leukemia, chronic granulocytic leukemia, acute promyelocytic leukemia, adult T-cell leukemia, aleukaemic leukemia, aleukocythemic leukemia, basophylic leukemia, blast cell leukemia, bovine leukemia, chronic myelocytic leukemia, leukemia cutis, embryonal leukemia, eosinophilic leukemia, Gross' leukemia, hairy-cell leukemia, hemoblastic leukemia, hemocytoblastic leukemia, histiocytic leukemia, stem cell leukemia, acute monocytic leukemia, leukopenic leukemia, lymphatic leukemia, lymphoblastic leukemia, lymphocytic leukemia, lymphogenous leukemia, lymphoid leukemia, lymphosarcoma cell leukemia, mast cell leukemia, megakaryocytic leukemia, micromyeloblastic leukemia, monocytic leukemia, myeloblastic leukemia, myelocytic leukemia, myeloid granulocytic leukemia, myelomonocytic leukemia, Naegeli leukemia, plasma cell leukemia, plasmacytic leukemia, promyelocytic leukemia, Rieder cell leukemia, Schilling's leukemia, stem cell leukemia, subleukemic leukemia, and undifferentiated cell leukemia

The term "lymphoma" generally refers to a malignant neoplasm of the lymphatic system, including cancer of the lymphatic system The two main types of lymphoma are Hodgkin's disease (HD or HL) and non-Hodgkin's lymphoma (NHL) Abnormal cells appear as congregations which enlarge the lymph nodes, form solid tumors in the body, or more rarely, like leukemia, circulate in the blood Hodgkin's disease lymphomas, include nodular lymphocyte predominance Hodgkin's lymphoma; classical Hodgkin's lymphoma; nodular sclerosis Hodgkin's lymphoma; lymphocyte-rich classical Hodgkin's lymphoma; mixed cellularity Hodgkin's lymphoma; lymphocyte depletion Hodgkin's lymphoma. Non-Hodgkin's lymphomas include small lymphocytic NHL, follicular NHL; mantle cell NHL; mucosa-associated lymphoid tissue (MALT) NHL; diffuse large cell B-cell NHL; mediastinal large B-cell NHL; precursor T lymphoblastic NHL; cutaneous T-cell NHL; T-cell and natural killer cell NHL; mature (peripheral) T-cell NHL; Burkitt's lymphoma; mycosis fungoides; Sézary Syndrome; precursor B-lymophoblastic lymphoma; B-cell small lymphocytic lymphoma; lymphoplasmacytic lymphoma; spenic marginal zome B-cell lymphoma; nodal marginal zome lymphoma; plasma cell myeloma/plasmacytoma; intravascular large B-cell NHL; primary effusion lymphoma; blastic natural killer cell lymphoma; enteropathy-type T, cell lymphoma; hepatosplenic gamma-delta T-cell lymphoma; subcutaneous panniculitis-like T-cell lymphoma; angioimmunoblastic T-cell lymphoma; and primary systemic anaplastic large T/null cell lymphoma.

The term "sarcoma" generally refers to a tumor which originates in connective tissue, such as muscle, bone, cartilage or fat, and is made up of a substance like embryonic connective tissue and is generally composed of closely packed cells embedded in a fibrillar or homogeneous substance. Sarcomas include soft tissue sarcomas, chondrosarcoma, fibrosarcoma, lymphosarcoma, melanosarcoma, myxosarcoma, osteosarcoma, Abemethy's sarcoma, adipose sarcoma, liposarcoma, alveolar soft part sarcoma, ameloblastic sarcoma, bottyoid sarcoma, chloroma sarcoma, chorio carcinoma, embryonal sarcoma, Wilms' tumor sarcoma, endometrial sarcoma, stromal sarcoma, Ewing's sarcoma, fascial sarcoma, fibroblastic sarcoma, giant cell sarcoma, granulocytic sarcoma, Hodgkin's sarcoma, idiopathic multiple pigmented haemorrhagic sarcoma, immunoblastic sarcoma of B cells, lymphoma, immunoblastic sarcoma of T-cells, Jensen's sarcoma, Kaposi's sarcoma, Kupffer cell sarcoma, angiosarcoma, leukosarcoma, malignant mesenchymoma sarcoma, parosteal sarcoma, reticulocytic sarcoma, Rous sarcoma, serocystic sarcoma, synovial sarcoma, and telangiectaltic sarcoma

The term "melanoma," is taken to mean a tumor arising from the melanocytic system of the skin and other organs, Melanomas include, for example, acral-lentiginous melanoma, amelanotic melanoma, benign juvenile melanoma, Cloudman's melanoma, S91 melanoma, Harding-Passey melanoma, juvenile melanoma, lentigo maligna melanoma, malignant melanoma, nodular melanoma, subungal melanoma, and superficial spreading melanoma,

The term "carcinoma" refers to a malignant new growth made up of' epithelial cells tending to infiltrate the surrounding tissues and give rise to metastases Exemplary carcinomas include, for example, acinar carcinoma, acinous carcinoma, adenocystic carcinoma, adenoid cystic carcinoma, carcinoma adenomatosum, carcinoma of adrenal cortex, alveolar carcinoma, alveolar cell carcinoma, basal cell carcinoma, carcinoma basocellulare, basaloid carcinoma, basosquamous cell carcinoma, bionchioalveolar carcinoma, bronchiolar carcinoma, bronchogenic carcinoma, cerebriform carcinoma, cholangiocellular carcinoma, chorionic carcinoma, colorectal carcinoma, colloid carcinoma, comedo carcinoma, corpus carcinoma, cribriform carcinoma, carcinoma en cuirasse, carcinoma cutaneum, cylindrical carcinoma, cylindrical cell carcinoma, duct carcinoma, carcinoma durum, embryonal carcinoma, encephaloid carcinoma, epiermoid carcinoma, carcinoma epitheliale adenoides, exophytic carcinoma, carcinoma ex ulcere, carcinoma fibrosum, gelatiniform carcinoma, gelatinous carcinoma, giant cell carcinoma, carcinoma gigantocellulare, glandular carcinoma, granulosa cell carcinoma, hair-matrix carcinoma, haematoid carcinoma, hepatocellular carcinoma, Hurthle cell carcinoma, hyaline carcinoma, hypemephroid carcinoma, infantile embryonal carcinoma, carcinoma, in situ, intraepidermal carcinoma, intraepithelial carcinoma, Krompecher's carcinoma, Kulchitzky-cell carcinoma, large-cell carcinoma, lenticular carcinoma, carcinoma lenticulare, lipomatous carcinoma, lymphoepithelial carcinoma, carcinoma medullare, medullary carcinoma, melanotic carcinoma, carcinoma molle, mucinous carcinoma, carcinoma muciparum, carcinoma mucocellulare, mucoepidermoid carcinoma, carcinoma mucosum, mucous carcinoma, carcinoma myxomatodes, naspharyngeal carcinoma, oat cell carcinoma, non-small cell carcinoma, carcinoma ossificans, osteoid carcinoma, papillary carcinoma, periportal carcinoma, preinvasive carcinoma, prickle cell carcinoma, pultaceous carcinoma, renal cell carcinoma of kidney, reserve cell carcinoma, carcinoma sarcomatodes, schneiderian carcinoma, scirrhous carcinoma, carcinoma scroti, signet-ring cell carcinoma, carcinoma simplex, small-cell carcinoma, solanoid carcinoma, spheroidal cell carcinoma, spindle cell carcinoma, carcinoma spongiosum, squamous carcinoma, squamous cell carcinoma, string carcinoma, carcinoma telangiectaticum, carcinoma telangiectodes, transitional cell carcinoma, carcinoma, tuberosum, tuberous carcinoma, verrucous carcinoma, and carcinoma villosum

The term "carcinoma" also encompasses adenocarcinomas Adenocarcinomas are carcinomas that originate in cells that make organs which have glandular (secretory) properties or that originate in cells that line hollow viscera, such as the gastrointestinal tract or bronchial epithelia. Examples include, but are not limited to, adenocarcinomas of the breast, lung, pancreas and prostate

Additional cancers encompassed by the present invention include, for example, multiple myeloma, neuroblastoma, rhabdomyosarcoma, primary thrombocytosis, primary macroglobulinemia, small-cell lung tumors, primary brain tumors, malignant pancreatic insulanoma, malignant carcinoid, urinary bladder cancer, premalignant skin lesions, gliomas, testicular cancer, thyroid cancer, esophageal cancer, genitourinary tract cancer, malignant hypercalcemia, endometrial cancer, adrenal cortical cancer, mesothelioma and medulloblastoma.

In accordance with the present invention, the MPPTs can be used to treat various stages and grades of cancer development and progression The present invention, therefore, contemplates the use of the MPPTs in the treatment of early stage cancers including early neoplasias that may be small, slow growing, localized and/or nonaggressive, for example, with the intent of curing the disease or causing regression of' the cancer, as well as in the treatment of intermediate stage and in the treatment of late stage cancers including advanced and/or metastatic and/or aggressive neoplasias, for example, to slow the progression of the disease, to reduce metastasis or to increase the survival of' the patient Similarly, the MPPTs may be used in the treatment of low grade cancers, intermediate grade cancers and or high grade cancers.

The present invention also contemplates that the MPPTs can be used in the treatment of' indolent cancels, recurrent cancers including locally recurrent, distantly recurrent and/or refractory cancers (*i.e*., cancers that have not responded to treatment), metastatic cancers, locally advanced cancers and aggressive cancers Thus, an "advanced" cancer includes locally advanced cancer and metastatic cancer and refers to overt disease in a patient, wherein such overt disease is not amenable to cure by local modalities of treatment, such as surgery of radiotherapy. The term "metastatic cancer" refers to cancer that has spread from one part of the body to another. Advanced cancers may also be unresectable, that is, they have spread to surrounding tissue and cannot be surgically removed

One skilled in the art will appreciate that many of' these categories may overlap, for example, aggressive cancers are typically also metastatic "Aggressive cancer," as used herein, refers to a rapidly growing cancer One skilled in the art will appreciate that for some cancers, such as breast cancer or prostate cancer the term "aggressive cancer" will refer to an advanced cancer that has relapsed within approximately the earlier two-thirds of the spectrum of relapse times for a given cancer, whereas for other types of cancer, such as small cell lung carcinoma (SCLC) nearly all cases present rapidly growing cancers which are considered to be aggressive The term can thus cover a subsection of a certain cancer type or it may encompass all of other cancer types

The MPPTs may also be used to treat drug resistant cancers, including multidrug resistant tumors As is known in the art, the resistance of cancer cells to chemotherapy is one of the central problems in the management of cancer.

Certain cancers, such as prostate and breast cancer, can be treated by hormone therapy, *i.e.* with hormones or anti-hormone drugs that slow or stop the growth of certain cancers by blocking the body's natural hormones Such cancers may develop resistance, or be intrinsically resistant, to hormone therapy The present invention further contemplates the use of the MPPTs in the treatment of such "hormone-resistant or "hormone-refractory" cancers

The present invention also contemplates the administration to a subject of a therapeutically effective amount of' one or more MPPTs together with one or more anti-cancer therapeutics The compound(s) can be administered before, during or after treatment with the anti-cancer therapeutic. An "anti-cancer therapeutic" is a compound, composition or treatment that prevents or delays the growth and/or metastasis of cancer cells Such anti-cancer therapeutics include, but are not limited to, chemotherapeutic drug treatment, radiation, gene therapy, hormonal manipulation, immunotherapy and antisense oligonucleotide therapy Examples of' useful chemotherapeutic drugs include, but are not limited to, hydroxyurea, busulphan, cisplatin, carboplatin, chlorambucil, melphalan, cyclophosphamide, Ifosphamide, danorubicin, doxorubicin, epirubicin, mitoxantrone, vincristine, vinblastine, Navelbine® (vinorelbine), etoposide, teniposide, paclitaxel, docetaxel, gemcitabine, cytosine, arabinoside, bleomycin, neocarcinostatin, suramin, taxol, mitomycin C and the like. The compounds of the invention are also suitable for use with standard combination therapies employing two or more chemotherapeutic agents It is to be understood that anti-cancer therapeutics for use in the present invention also include novel compounds or treatments developed in the future.

### 5.2 Administration

Typically in the treatment of cancer, MPPTs are administered systemically to patients, for example, by bolus injection or continuous infusion into a patient's bloodstream Alternatively, the MPPTs may be administered locally, at the site of a tumor (intratumorally) When used in conjunction with one or more known chemotherapeutic agents, the compounds can be administered prior to, or after, administration of the chemotherapeutic agents, or they can be administered concomitantly The one or more chemotherapeutics may be administered systemically, for example, by bolus injection or continuous infusion, or they may be administered orally

In one embodiment, the MPPT can be injected into a subject having cancer, using an administration approach similar to the multiple injection approach of brachythenapy. For example, multiple aliquots of the purified MPPT in the form of a pharmaceutical composition or formulation and in the appropriate dosage units, may be injected using a needle Alternative methods of administration of the MPPTs according to the present invention will be evident to one of skill in the art. Such methods include, for example, the use of catheters, or implantable pumps to provide continuous infusion of' the MPPT to the subject in need of therapy.

As is known in the art, software planning programs can be used in combination with brachytherapy treatment and ultrasound, for example, for placement of catheters for infusing MPPTs to treat, for example, brain tumors or other localized tumors. For example, the positioning and placement of the needle can generally be achieved under ultrasound guidance. The total volume, and therefore the number of injections and deposits administered to a patient, can be adjusted, for example, according to the volume or area of the organ to be treated. An example of a suitable software planning program is the brachytherapy treatment planning program Variseed 7.1 (Varian Medical Systems, Palo Alto, California) Such approaches have been successfully implemented in the treatment of prostate cancel, among others

It is also contemplated that the MPPTs of the present invention can be co-administered with tracers in order to measure how the MPPT is distributed within the body after administration

If necessary to reduce a systemic immune response to the MPPTs, immunosuppressive therapies can be administered in combination with the MPPTs. Examples of immunosuppressive therapies include, but are not limited to, systemic or topical corticosteroids (Suga et al., Ann. Thorac Surg 73:1092-7, 2002), cyclosporin A (Fang et al , Hum Gene Ther 6:1039-44, 1995), cyclophosphamide (Smith et al., Gene Ther. 3:496-502, 1996), deoxyspergualin (Kaplan et al., Hum. Gene Ther. 8:1095-1104, 1997) and antibodies to T and/or B cells [e.g., anti-CD40 ligand, anti CD4 antibodies, anti-CD20 antibody (Rituximab)] (Manning et al., Hum Gene Their 9:477-85, 1998), Such agents can be administered before, during, or subsequent to administration of MPPTs according to the present invention).

The MPPTs may be used as part of a neo-adjuvant therapy (to primary therapy), as part of an adjuvant therapy regimen, where the intention is to cure the cancer in a subject The present invention contemplates the use of the MPPTs at various stages in tumor development and progression, including in the treatment of' advanced and/or aggressive neoplasias (*i*.*e*. overt disease in a subject that is not amenable to cure by local modalities of' treatment, such as surgery or radiotherapy), metastatic disease, locally advanced disease and/or refractory tumors (*i.e* a cancer or tumor that has not responded to treatment).

"Primary therapy" refers to a first line of treatment upon the initial diagnosis of cancer in a subject Exemplary primary therapies may involve surgery, a wide range of chemotherapies and radiotherapy "Adjuvant therapy" refers to a therapy that follows a primary therapy and that is administered to subjects at risk of relapsing Adjuvant systemic therapy is begun soon after primary therapy to delay recurrence, prolong survival or cure a subject.

As noted above, it is contemplated that the MPPTs of the invention can be used alone or in combination with one or more other chemotherapeutic agents as part of an adjuvant therapy Combinations of' the MPPTs and standard chemotherapeutics may act to improve the efficacy of the chemotherapeutic and, therefore, can be used to improve standard cancer therapies

This application can be particularly important in the treatment of drug-resistant cancers which are not responsive to standard treatment

The dosage to be administered is not subject to defined limits, but it will usually be an effective amount. The compositions may be formulated in a unit dosage form. The term "unit dosage form" refers to physically discrete units suitable as unitary dosages for human subjects and other mammals, each unit containing a predetermined quantity of active material calculated to produce the desired therapeutic effect, in association with a suitable pharmaceutical excipient The unit dosage forms may be administered once or multiple unit dosages may be administered, for example, throughout an organ, or solid tumor. Examples of ranges for the MPPT(s) in each dosage unit are from about 0 0005 to about 100 mg, or more usually, from about 1 0 to about 1000 µg.

Daily dosages of the compounds of the present invention will typically fall within the range of about 001 to about 100 mg/kg of body weight, in single or divided dose However, it will be understood that the actual amount of the compound(s) to be administered will be determined by a physician, in the light of the relevant circumstances, including the condition to be treated, the chosen route of administration, the actual compound administered, the age, weight, and response of the individual patient, and the severity of the patient's symptoms. The above dosage range is given by way of example only and is not intended to limit the scope of' the invention in any way. In some instances dosage levels below the lower limit of the aforesaid range may be more than adequate, while in other cases still larger doses may be employed without causing harmful side effects, for example, by first dividing the larger dose into several smaller doses for administration throughout the day.

### 6. Gene Therapy

The MPPTs according to the present invention, may also be employed in accordance with the present invention by expression of' such proteins *in vivo*, which is often referred to as "gene therapy "

Thus, for example, cells from a patient may be engineered with a polynucleotide (DNA or RNA) encoding an MPPT *ex vivo,* with the engineered cells then being provided to a patient to be treated with the polypeptide. Such methods are well-known in the art For example, cells may be engineered by procedures known in the art by use of a retroviral particle containing RNA encoding an MPPT or a biologically active fragment thereof

Similarly, cells may be engineered in vivo for expression of' a polypeptide in vivo using procedures known in the art, As known in the art, a producer cell for producing a retroviral particle containing RNA encoding an MPPT, or a biologically active fragment thereof, may be administered to a patient for engineering cells *in vivo* and expression of the polypeptide *in vivo* These and other methods for administering MPPTs by such methods should be apparent to those skilled in the art from the teachings of the present invention For example, the expression vehicle for engineering cells may be other than a retrovirus, for example, an adenovirus which may be used to engineer cells *in vivo* after combination with a suitable delivery vehicle

Retroviruses, from which the retroviral plasmid vectors hereinabove mentioned, may be derived include, but are not limited to, Moloney Murine Leukemia Virus, spleen necrosis virus, retroviruses such as Rous Sarcoma Virus, Harvey Sarcoma Virus, avian leukosis virus, gibbon ape leukemia virus, human immunodeficiency virus, adenovirus, Myeloproliferative Sarcoma Virus, and mammary tumor virus In one embodiment, the retroviral plasmid vector is derived from Moloney Murine Leukemia Virus

The vector includes one or more promoters Suitable promoters which may be employed include, but are not limited to, the retroviral LTR; the SV40 promoter; and the human cytomegalovirus (CMV) promoter described in Miller, et al , Biotechniques, Vol 7, No. 9, 980-990 (1989), or any other promoter (e.g., cellular promoters such as eukaryotic cellular promoters including, but not limited to, the histone, pol III, and β-actin promoters). Other viral promoters which may be employed include, but are not limited to, adenovirus promoters, thymidine kinase (TK) promoters, and B19 parvovirus promoters The selection of a suitable promoter will be apparent to those skilled in the art from the teachings contained herein.

The nucleic acid sequences encoding the MPPTs of the present invention are under the control of suitable promoters Suitable promoters which may be employed include, but are not limited to, adenoviral promoters, such as the adenoviral major late promoter; or heterologous promoters, such as the cytomegalovirus (CMV) promoter; the respiratory syncytial virus (RSV) promoter; inducible promoters, such as the MMT promoter, the metallothionein promoter; heat shock promoters; the albumin promoter; the ApoAI promoter; human globin promoters; viral thymidine kinase promoters, such as the Herpes Simplex thymidine kinase promoter; retroviral LTRs (including the modified retroviral LTRs hereinabove described); the β-acting promoter; and human growth hormone promoters, The promoter also may be the native promoter which controls the genes encoding the MPPTs The retroviral plasmid vector is employed to transduce packaging cell lines to form producer cell lines Examples of packaging cells which may be transfected include, but are not limited to, the PE501, PA317, ψ-2, ψ-AM, PA12, T19-14X, VT-19-17-H2, ψCRE, ψCRIP, GP+E-86, GP+envAm12, and DAN cell lines as described in Miller, Human Gene Therapy, Vol. 1, pgs. 5-14 (1990), which is incorporated wherein by reference in its entirety. The vector may transduce the packaging cells through any means known in the art Such means include, but are not limited to, electroporation, the use of liposomes, and CaPO₄ precipitation In one alternative, the retroviral plasmid vector may be encapsulated into a liposome, or PTH to a lipid, and then administered to a host

The producer cell line generates infectious retroviral vector particles which include the nucleic acid sequence(s) encoding the polypeptides, Such retroviral vector particles then may be employed, to transduce eukaryotic cells, either *in vitro* or *in vivo*, The transduced eukaryotic cells will express the nucleic acid sequence(s) encoding the polypeptide, Eukaryotic cells which may be transduced include, but are not limited to, embryonic stem cells, embryonic carcinoma cells, as well as hematopoietic stem cells, hepatocytes, fibroblasts, myoblasts, keratinocytes, endothelial cells, and bronchial epithelial cells

### 7. Clinical Trial

One skilled in the art will appreciate that, following the demonstrated effectiveness of an MPPT *in vitro* and in animal models, MPPTs should be tested in Clinical Trials in order to further evaluate its efficacy in the treatment of cancer and to obtain regulatory approval for therapeutic use As is known in the art, clinical trials progress through phases of testing, which are identified as Phases I, II, III, and IV.

Initially MPPTs will be evaluated in a Phase I trial Typically Phase I trials are used to determine the best mode of administration (for example, by pill or by injection), the frequency of administration, and the toxicity for the compounds Phase I studies frequently include laboratory tests, such as blood tests and biopsies, to evaluate the effects of a compound in the body of the patient For a Phase I trial, a small group of cancer patients is treated with a specific dose of an MPPT During the trial, the dose is typically increased group by group in order to determine the maximum tolerated dose (MTD) and the dose-limiting toxicities (DLT) associated with the compound This process determines an appropriate dose to use in a subsequent Phase II trial.

A Phase II trial can be conducted to further evaluate the effectiveness and safety of an MPPT, In Phase II trials, an MPPT is administered to groups of patients with either one specific type of cancer or with related cancers, using the dosage found to be effective in Phase I trials.

Phase III trials focus on determining how a compound compares to the standard, or most widely accepted, treatment In Phase III trials, patients are randomly assigned to one of two or more "arms" In a trial with two arms, for example, one arm will receive the standard treatment (control group) and the other arm will receive MPPT treatment (investigational group)

Phase IV trials are used to further evaluate the long-term safety and effectiveness of a compound Phase IV trials are less common than Phase I, II and III trials and will take place after the MPPT has been approved for standard use

### 7.1 Eligibility of patients for- Clinical Trials

Participant eligibility criteria can range from general (for example, age, sex, type of cancer) to specific (for example, type and number of prior treatments, tumor characteristics, blood cell counts, organ function) Eligibility criteria may also vary with trial phase For example, in Phase I and II trials, the criteria often exclude patients who may be at risk from the investigational treatment because of abnormal organ function or other factors. In Phase II and III trials additional criteria are often included regarding disease type and stage, and number and type of prior treatments.

Phase I cancer trials usually comprise 15 to 30 participants for whom other treatment options have not been effective Phase II trials typically comprise up to 100 participants who have already received chemotherapy, surgery, or radiation treatment, but for whom the treatment has not been effective Participation in Phase II trials is often restricted based on the previous treatment received, Phase III trials usually comprise hundreds to thousands of participants. This large number of participants is necessary in order to determine whether there are true differences between the effectiveness of an MPPT and the standard treatment Phase III may comprise patients ranging from those newly diagnosed with cancer to those with extensive disease in order to cover the disease continuum,

One skilled in the art will appreciate that clinical trials should be designed to be as inclusive as possible without making the study population too diverse to determine whether the treatment might be as effective on a more narrowly defined population The more diverse the population included in the trial, the more applicable the results could be to the general population, particularly in Phase III trials. Selection of appropriate participants in each phase of clinical that is considered to be within the ordinary skills of a worker in the art

### 7.2 Assessment of patients prior to treatment

Priors to commencement of the study, several measures known in the art can be used to first classify the patients Patients can first be assessed, for example, using the Eastern Cooperative Oncology Group (ECOG) Performance Status (PS) scale ECOG PS is a widely accepted standard for the assessment of the progression of a patient's disease as measured by functional impairment in the patient, with ECOG PS 0 indicating no functional impairment, ECOG PS 1 and 2 indicating that the patients have progressively greater functional impairment but are still ambulatory and ECOG PS 3 and 4 indicating progressive disablement and lack of mobility.

Patients' overall quality of life can be assessed, for example, using the McGill Quality of Life Questionnaire (MQOL) (Cohen et al (1995) Palliative Medicine 9: 207-219) The MQOL measures physical symptoms; physical, psychological and existential well-being; support; and overall quality of life. To assess symptoms such as nausea, mood, appetite, insomnia, mobility and fatigue the Symptom Distress Scale (SDS) developed by McCorkle and Young ((1978) Cancer Nursing 1: 373-378) can be used

Patients can also be classified according to the type and/or stage of their disease and/or by tumor size

### 7.3Administration of MPPTs in Clinical Trials

MPPTs are typically administered to the trial participants parenterally In one embodiment, an MPPT is administered by intravenous infusion In another embodiment, an MPPT is administered intratumorally. Methods of administering drugs by intravenous infusion are known in the art. Usually intravenous infusion takes place over a certain time period, for example, over the course of 60 minutes.

A range of doses of' an MPPT can be tested An exemplary dose range for MPPT treatment includes dosages in the range 0.2 µg/kg body weight to 20 µg/kg body weight in single or divided doses

### 7.4 Pharmacokinetic monitoring

To fulfill Phase I criteria, distribution of the MPPT is monitored, for example, by chemical analysis of' samples, such as blood or urine, collected at regular intervals For example, samples can be taken at regular intervals up to until about 72 hours after the start of infusion In one embodiment, samples are taken at 0, 0.33, 0.67, 1, 1.25, 1.5, 2, 4, 6, 8, 12, 24, 48 and 72 hours after the start of' each infusion of' the MPPT.

If analysis is not conducted immediately, the samples can be placed on dry ice after collection and subsequently transported to a freezer to be stored at -70 °C until analysis can be conducted Samples can be prepared for analysis using standard techniques known in the art and the amount of the MPPT present can be determined, for example, by high-performance liquid chromatography (HPLC)

Pharmacokinetic data can be generated and analyzed in collaboration with an expert clinical pharmacologist and used to determine, for example, clearance, half-life and maximum plasma concentrations

### 7.5 Monitoring of Patient Outcome

The endpoint of a clinical trial is a measurable outcome that indicates the effectiveness of a compound under evaluation The endpoint is established prior to the commencement of the trial and will vary depending on the type and phase of' the clinical trial. Examples of endpoints include, for example, tumor response rate - the proportion of trial participants whose tumor was reduced in size by a specific amount, usually described as a percentage; disease-free survival - the amount of time a participant survives without cancer occurring or recurring, usually measured in months; overall survival - the amount of time a participant lives, typically measured from the beginning of the clinical trial until the time of death For advanced and/or metastatic cancers, disease stabilization- the proportion of trial participants whose disease has stabilized, for example, whose tumor(s) has ceased to grow and/or metastasize, can be used as an endpoint Other endpoints include toxicity and quality of life.

Tumor response rate is a typical endpoint in Phase II trials However, even if a treatment reduces the size of a participant's tumor and lengthens the period of disease-free survival, it may not lengthen overall survival In such a case, side effects and failure to extend overall survival might outweigh the benefit of longer disease-free survival. Alternatively, the participant's improved quality of life during the tumor-free interval might outweigh other factors Thus, because tumor response rates are often temporary and may not translate into long-term survival benefits for the participant, response rate is a reasonable measure of a treatment's effectiveness in a Phase II trial, whereas participant survival and quality of life are typically used as endpoints in a Phase III trial

### 8. Pharmaceutical Kits

The present invention additionally provides for therapeutic kits or packs containing one or more of the MPPTs or a pharmaceutical composition comprising one or more of the MPPTs for use in the treatment of' cancer. Individual components of the kit can be packaged in separate containers, associated with which, when applicable, can be a notice in the form prescribed by a governmental agency regulating the manufacture, use or sale of pharmaceuticals or biological products, which notice reflects approval by the agency of manufacture, use or sale for human or animal administration. The kit can optionally further contain one or more other therapeutic agents for use in combination with the MPPTs of' the invention The kit may optionally contain instructions or directions outlining the method of use or dosing regimen for the MPPTs and/or additional therapeutic agents.

When the components of the kit are provided in one or more liquid solutions, the liquid solution can be an aqueous solution, for example a sterile aqueous solution In this case the container means may itself be an inhalant, syringe, pipette, eye dropper, or other such like apparatus, from which the composition may be administered to a patient or applied to and mixed with the other components of the kit

The components of the kit may also be provided in dried or lyophilized form and the kit can additionally contain a suitable solvent for reconstitution of the lyophilized components. Irrespective of the number or type of containers, the kits of the invention also may comprise an instrument for assisting with the administration of the composition to a patient Such an instrument may be an inhalant, syringe, pipette, forceps, measured spoon, eye-dropper or similar medically approved delivery vehicle

To gain a better understanding of the invention described herein, the following examples are set forth, It will be understood that these examples are intended to describe illustrative embodiments of the invention and are not intended to limit the scope of' the invention in any way

### EXAMPLES

### EXAMPLE 1: PRODUCTION OF MPPT1

An MPPT according to one embodiment of the invention, MPPT1, which is a naturally occurring proaerolysin polypeptide comprising modification of the activation sequence to add a uPA site, and a histidine tag, was prepared as follows. Proaerolysin is normally activated by cleavage catalyzed by furin within the sequence between amino acids 427 and 432 (KVRRAR). In order to prevent furin activation, and to produce a product that can be activated by uPA, the furin cleavage sequence was replaced with the sequence SGRSAQ, which is known to be a substrate for uPA. This was accomplished by changing 3 codons at a time using the Quikchange II kit from Stratagene. In the first step, 2 complimentary primers were synthesized (5'-GCG GCT GAC AGC AGI GGG CGT CGT GCT C-3' [SEQ ID NO:10] and 5'-AGC ACG ACG CCC ACT GCT GTC AGC CGC G-3' [SEQ ID NO:11]), which replaced the KVR codons in the wild-type sequence with SGR codons in a PCR amplification using plasmid PA-His::pTZ18U as a template, This plasmid was prepared as follows DNA encoding a His tag was added to the end of the aerA gene in plasmid pTZ18pNB5 (Diep, D.B., Lawrence, T.S., Ausio, J., Howard, S.P. and Buckley, J T 1998 Secretion and properties of the large and small lobe of the channel-forming toxin aerolysin Mol Microbiol 30:341-352) using a Stratagene Quikchange II kit

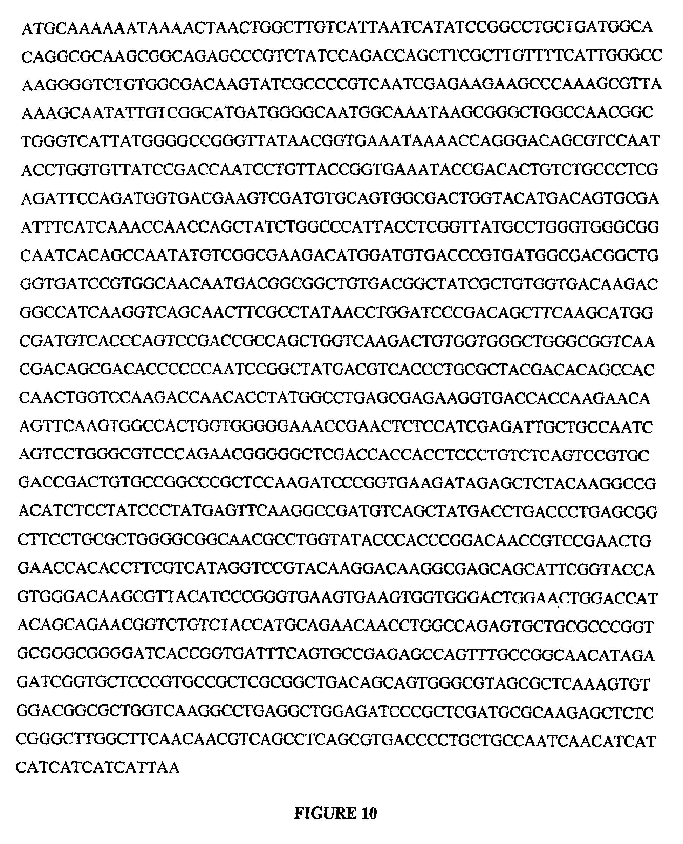

In a second PCR step, two more complimentary primers were synthesized (5'-AGC AGT GGG CGT AGC GCT CAA AGT GTG GAC G-3' [SEQ ID NO:12] and 5'-GTC CAC ACT TTG AGC GCT ACG CCC ACT GCT G-3' [SEQ ID NO:13]) which replace the last three codons of the target sequence, encoding RAR, to the codons for SAQ in a PCR amplification using the product from the first PCR as the template The resulting construct, MPPT1, was excised with EcoRI and HindIII and ligated into the EcoRI and HindIII sites of the vector pMMB66HE. This plasmid, MPPT1::pMMB66, was transconjugated into *Aeromonas salmonicida* strain CB3 using the helper strain MM297-pRK2013 (Figurski and Helinski, 1989) as previously described (Wong et al., 1989). The resulting strain, CB3 MPPTI::pMMB66, was used to express MPPT1 for purification of the protein. The nucleotide sequence of MPPT1 (SEQ ID NO:20) is shown in Figure 10

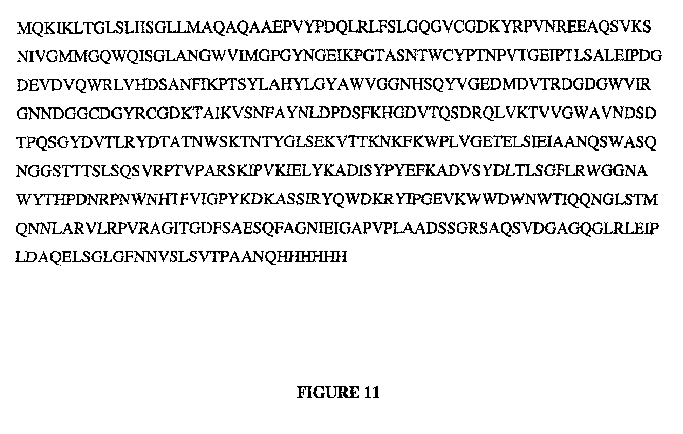

MPPT1 was expressed and purified as follows. CB3 MPPT1::pMMB66 was inoculated into LB Davis media containing 02% glucose, 40 µg/ml rifampicin, 40 µg/ml kanamycin and 100 µg/ml ampicillin. and grown overnight at 27°C/250 rpm It was then subinoculated (1%) into the same medium and incubated at 27°C/250 rpm. When the culture reached an OD₆₀₀ₙₘ of approx 0.7, IPTG was added to a final concentration of 1 mM to induce MPPT1 production and incubation was continued for 17 5 hours The overnight cultures (OD₆₀₀ₙₘ =45-4.8) were centrifuged at 10,000 rpm/15 minutes/4°C in a JA-16.25 rotor (Beckman) and the culture supernatants were collected. The supernatant was concentrated from 2 4 L to ~ 90 ml using a Kvick Lab SCU 10,000 Dalton cutoff membrane (Amersham; polyethersulfone membrane/0 11 m² filtration area) at 4°C The concentrate was centrifuged at 10,000 rpm/4°C using a JA-25.5 (Beckman) rotor for 10 minutes. The supernatant was loaded onto a 5 ml XK16 Ni²⁺ column (Chelating Sepharose Fast Flow, Amersham) equilibrated in 20 mM Na₂HPO₄, 0.5 M NaCl, 10 mM imidazole, pH 7 4 at 1 ml/min After washing the column with 105 ml equilibration buffer, the column was eluted with 60 ml of 20 mM Na₂HPO₄, 0,5 M NaCl, 200 mM imidazole, pH 7.4 at 2.5 ml/min The protein containing fractions were identified by measuring A₂₈₀ values of the fractions, and the peak tubes were loaded in 2.5 ml aliquots onto a PD10 column (Amersham) equilibrated in 10 mM NaH₂PO₄, 0.15 M NaCl, 1 mM EDTA, pH 7 4 Protein was eluted with 3 5 ml of the same buffer and the PD10 fractions were frozen and stored at -80°C The amino acid sequence of MPPT1 (SEQ ID NO:21) is shown in Figure 11

### EXAMPLE 2: PRODUCTION OF MPPT2, AND MPPT3

MPPTs according to additional embodiments of the invention were prepared MPPT2 is a proaerolysin polypeptide with an activation sequence modified to add a uPA cleavage site, an ARD (specifically an AFAI antibody fragmant), and a histidine tag MPPT3 is a proaerolysin polypeptide with an activation sequence modified to add a uPA cleavage site, an ARD (specifically an AFAI antibody fragment) linked to the proaerolysin polypeptide with a linker that is cleavable by uPA, and a histidine tag These MPPTs were prepared as follows:

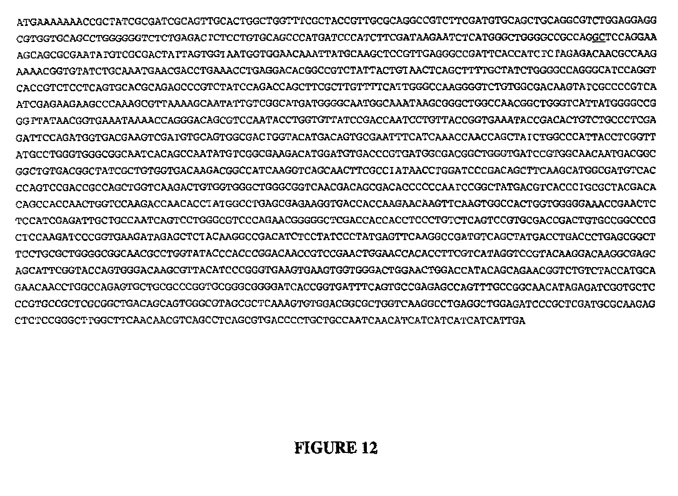

MPPT2 was prepared using the two sets of primers shown in the production of MPhT1 in Example 1 (SEQ ID NO:10-13) in a two-step Quikchange mutagenesis procedure (as described in Example 1) using the plasmid AFA-PA-His::pTZ18U as a template (see below for construction of this plasmid). The resulting plasmid was named MPPT2::pMMB67EH. The nucleotide sequence of MPPT2 (SEQ ID NO:22) is shown in Figure 12.

AFA-PA-His::pIZ18U was prepared as follows The starting point for production of the plasmid AFA-PA-His::pTZ18U was the construct encoding the AFA-large lobe of proaerolysin fusion described in International Patent Application No PCI/CA2004/000309 (WO 2004/078097). It encodes 116 amino acids from AFA, preceded by an *E. coli ompA* signal sequence, and followed by amino acids 80-470 of proaerolysin This construct was inserted into the vector pSJF2 and named AFA-PALLa::pSJF2 Six histidines codons were placed at the C-terminus of AFA-PALLa by a two-step Quikchange (Stratagene) procedure, adding 3 codons at each step. The resulting plasmid was called AFA-PALLa-His6::pSJF2.

In order to make a full length proaerolysin molecule with the AFA fragment fused to its N-terminus, AFA-PALLa-His6::pSJF2 was used as a template in a PCR mutagenesis procedure. One primer was synthesized (5'-ATA GAC GGG CTC TGC GTG CAC TGA GGA GAC G - 3' SEQ ID NO:53) and used with the pUC reverse primer and AFA-PALLa::pSJF2 plasmid to amplify a fragment that contained the *ompA* signal sequence and AFA portions of the construct Wild-type proaerolysin was used as the template in a second PCR step A second primer was synthesized (5'-GTC TCC TCA GTG CAC GCA GAG CCC GTC TAT C-3' SEQ ID NO:54) and used with the pUC reverse primer to amplify the entire proaerolysin gene from the plasmid PA::pTZ18U (originally named pTZ18pNB5 in Diep, D.B., Lawrence, T S , Ausio, J., Howard, S.P. and Buckley, J.T. 1998. Secretion and properties of the large and small lobe of the channel-forming toxin aerolysin Mol. Microbiol 30:341-352) When the two resulting PCR products were mixed together with the pUC reverse primer in a third PCR reaction, a product that contained the *ompA* signal sequence and AFA molecule fused to the entire proaerolysin molecule was formed The *ompA* signal sequence/AFA/small lobe portion of this product was cut out with the restriction enzymes PstI and BamHI and ligated into a PstI/BamHI digested fragment of AFA-PALLa-His6::pSJF2 The resulting plasmid was called AFA-PA-H6::pSJF2 In order to use AFA-PA-His in subsequent cloning steps, the AFA-PA-His insert was cut out of the pSJF2 plasmid with the restriction enzymes EcoRI and HindIII and ligated into the EcoRI and HindIII sites of the cloning plasmid pTZ18U to produce AFA-PA-His::pTZ18U. The PCR product resulting from mutagenesis of AFA-PA-His::pTZ18U was cut with EcoRI and HindIII and ligated into the EcoRI and HindIII sites of the vector pMMB67EH.

MPPT3 was prepared as follows. An initial construct was prepared in which a thrombin cut-site (LVPRGS) had been engineered between AFA and the small lobe of' proaerolysin (called AFA-t-PA::pTZ18U). This thrombin construct was engineered by adding codons for the first 3 amino acids of the cut-site (LVP) using the Quikchange Mutagenesis kit (Stratagene). Two primers were synthesized to insert these 3 amino acids:
5'-GTCTCCTCAGTGCACCTAGTCCCTGCAGAGCCCGTCTATC-3' (fwd) [SEQ ID NO:14]
5'-ATAGACGGGCTCTGCAGGGACTAGGTGCACTGAGGAGACG-3' (rev) [SEQ ID NO:15]

The product of this mutagenesis reaction was then used as the template for a second Quickchange mutagenesis step, in which 2 new primers were synthesized in order to insert the codons for the final three amino acids (RGS):
5'-GTGCACCTAGTCCCTCGTGGTTCCGCAGAGCCCGTCTATC-3' (fwd) [SEQ ID NO:16]
5'-ATAGACGGGCTCTGCGGAACCACGAGGGACTAGGTGCACTG-3' (rev) [SEQ ID NO:17]

This Quickchange mutagenesis product contained the thrombin cut-site between the AFA and proaerolysin potions of the protein This product was digested with HindIII and EcoRI and ligated into the vector pTZ18U to form the plasmid AFA-t-PA::pTZ18U. This plasmid was used as the template for a one step mutagenesis protocol using the Quikchange mutagenesis (Stratagene) kit. The primers used in this mutagenesis were:
5'- GTC TCC TCA GTG CAC TCA GGC CGT AGT GCT CAA GCA GAGC - 3' (fwd) [SEQ ID NO:18]

5' – C TGG ATA GAC GGG CTC TGC TTG AGC ACT ACG GCC TGA GTG CAC TGA
GGA GAC -3 (rev) [SEQ ID NO:19]

These primers changed the LVPRGS thrombin cut site to a SGRSAQ uPA cut-site in a single Quikchange (Stratagene) PCR step. The resulting PCR product was cut with EcoRI and HindIII and ligated into the EcoRI and HindIII sites of vector pMMB67EH. This plasmid was named AFA-uPA-PA-His::pMMB67EH

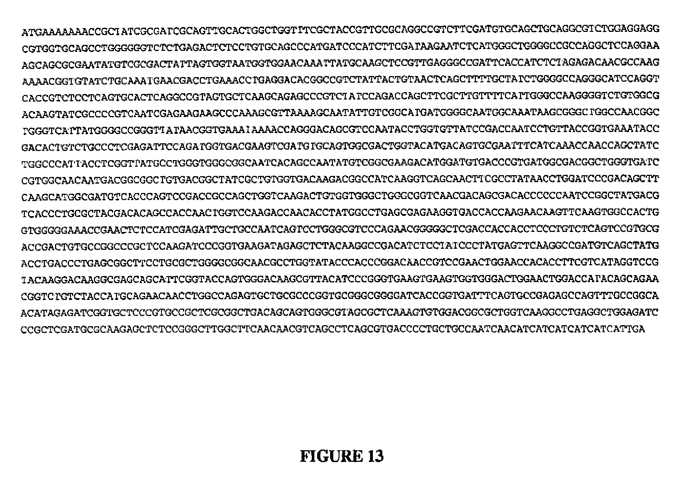

In order to prepare the MPPT3 construct, both AFA-uPA-PA-His::pTZ18U and MPPT2::pTZ18U plasmids were digested with KpnI and PstI restriction enzymes A band of approximately 1.4 kb was purified from the AFA-uPA-PA-His::pTZ18U digest, while a band of approximately 3.3 kb was purified from the MPPT2::pTZ18U digest. These two fragments were ligated together to form the construct MPPT3::pTZ18U. This plasmid was digested with EcoRI and HindIII, and the MPPT3 insert was ligated into the EcoRI and HindIII restriction sites of vector pMMB67EH. The resulting plasmid was called MPPT3::pMMB67EH The nucleotide sequence of MPPT3 (SEQ ID NO:24) is shown in Figure 13

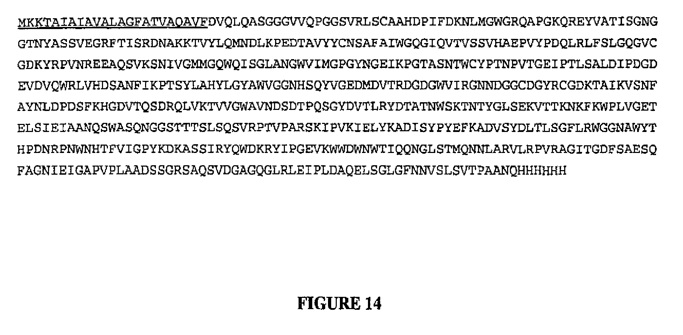

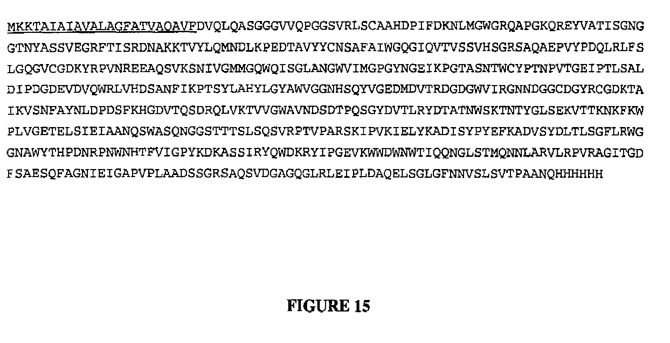

MPPT2::pMMB67EH and MPPT3::pMMB67EH were transconjugated into CB3 as described above. Strains CB3 MPPT2::pMMB67EH, and CB3 MPPT3::pMMB67EH were used to express and purify MPPT2 and MPPT3 These stains were each inoculated into 30 ml of LB Davis media containing 0.2% glucose, 40 µg/ml rifampicin, 40 µg/ml kanamycin and 100 µg/ml ampicillin The cultures were grown overnight at 27°C/250 rpm. The overnight cultures were subinoculated and the purifications were carried out as described as described for MPPT1 in Example 1. The amino acid sequences of MPPT2 (SEQ ID NO:23) and MPPT3 (SEQ ID NO:25) are shown in Figures 14 and 15.

### EXAMPLE 3: ACTIVATION OF MPPT1 BY uPA - I

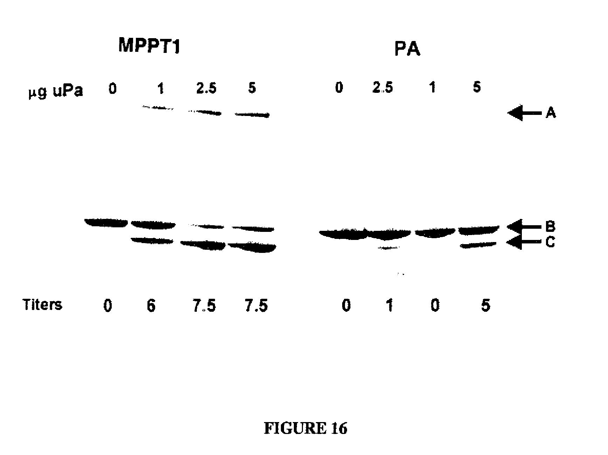

To test whether the MPPT1 could be activated by uPA, both MPPT1 and a control wild-type proaerolysin (PA) were incubated with varying concentrations of uPA (Sigma) and tested for their ability to lyse horse red blood cells Protease digestion was carried out by incubating 4 µg of wild type proaerolysin or MPPT1 with 0, 1, 2.5, or 5 µg of uPA in a volume of 62 5 µL HBS (20 mM HEPES, 0.15 M NaCl, pH 7.4) for 60 min. at room temperature The protease was inhibited by adding PMSF to 1 mM After incubation, samples (2 µg) were also run on SDS polyacrylamide gels (10% NuPAGE Bis-Tris gels run in 1 x MOPS buffer, Invitrogen) and stained with Coomasie Blue (Figure 16)

The hemolysis assay was carried out as follows. Washed erythrocytes were prepared by diluting whole horse blood in phosphate buffered saline (PBS; 10 mM NaH₂PO₄, 0.15 M NaCl, pH 7 4) and centrifuging to pellet cells. The supernatant and white blood cells were removed and the erythrocytes were resuspended in PBS and pelleted again. This washing procedure was repeated until the supernatant was colorless The packed cells were then suspended in HBS to 0 8% (v:v) To carry out the titer assay, 6 µg of test sample was added to the first row of 96 well titer plates and the volume was adjusted to 90 µl with HBS The test samples were activated by adding 10 µl of protease, to obtain the indicated final protease concentration, and then incubating at room temperature for the required time. After incubation, 100 µl of HBS was added to the first well, and the samples were serially diluted 1:2 with HBS After dilution, 100 µl of the 0 8% erythrocytes was added to each well and the plate was incubated at 37°C The titer values were recorded at 5 minutes, 10 minutes, 15 minutes, 30 minutes and 60 minutes by visually assessing the number of wells showing lysis or clearing in each row

The results are shown in Figure 16, where A indicates oligomer; B indicates MPPT1; C indicates activated aerolysin. The results indicate that MPPT1 was much more readily activated by uPA than native PA was. Thus, the MPPT1 sample digested with 1 µg of uPA showed an activated aerolysin band (∼48 kDa; lowest band), and a high molecular weight oligomer band No activated aerolysin band, and only a very faint high molecular weight oligomer band, was seen for the PA sample digested with 1 µg of uPA

### EXAMPLE 4: ACTIVATION OF MPPT2 AND MPPT3

To test whether MPPT2 and MPPT3 could be activated upon digestion with uPA, 7,5 µg of each protein was incubated with 4 µg of uPA in a total of 100 µl of HBS (20 mM HEPES, 0.15 M NaCl, pH 74) for 4 hours at 37°C The protease inhibitor phenylmethylsulfonylfluoride was then added to a final concentration of 1 mM to stop digestion Samples (6 µg) were transferred to 96-well titer plates, and then assayed for hemolytic activity against horse red blood cells (0 4%) as described in Example 3

**Table 6: Hemolytic titers of proteins treated with uPA.**

| Protein | Hemolytic activity after 60 minutes |
|---|---|
| MPPT2 | 4 5 |
| MPPT3 | 8 |

The results show that MPPT3 was most active after treatment with uPA, which can remove both the AFA portion and the C-teiminus of the molecule, producing aerolysin. This also indicated that the presence of the AFA fusion molecule reduced the hemolytic activity of aerolysin (Table 6)

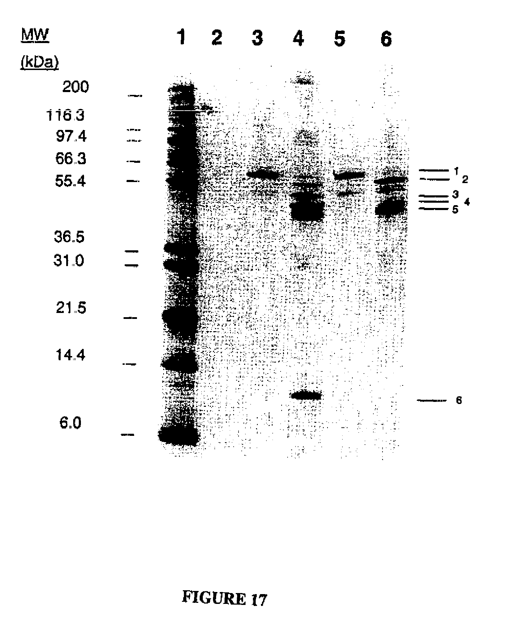

Each of the MPPTs was also examined by SDS-PAGE after digestion with uPA (see Figure 17) Samples (7 5 µg) were incubated with (Lanes 4, 6) or without (Lanes 3, 5) 0 04 mg/ml urokinase (Sigma) at 37°C for 4 hr before loading 100 ng of each sample onto a 10% NuPAGE Bis-Tris gel (Invitrogen) and running under non-reducing conditions Lane 1, Molecular weight standards; Lanes 3 and 4, MPPT3; Lanes 9 and 10, MPPT2. Lane 2 is blank. Bands that are observed are the full length AFA-PA-His (1), AFA-PA, AFA-PA constructs with C-terminal peptide removed (2), AFA-PA with AFA portion removed (3), aerolysin (4), urokinase (5), and AFA that has been digested from the molecule (6) The results shown in Figure 17 are consistent with the presence or absence of the uPA activation site in the variants. MPPT3 is the only polypeptide that produced a band corresponding to aerolysin after digestion, accounting for its activity in the titer assay

### EXAMPLE 5: ABILITY OF MPPT1, MPPT2 AND MPPT3 TO KILL A549 HUMAN LUNG CARCINOMA CELLS

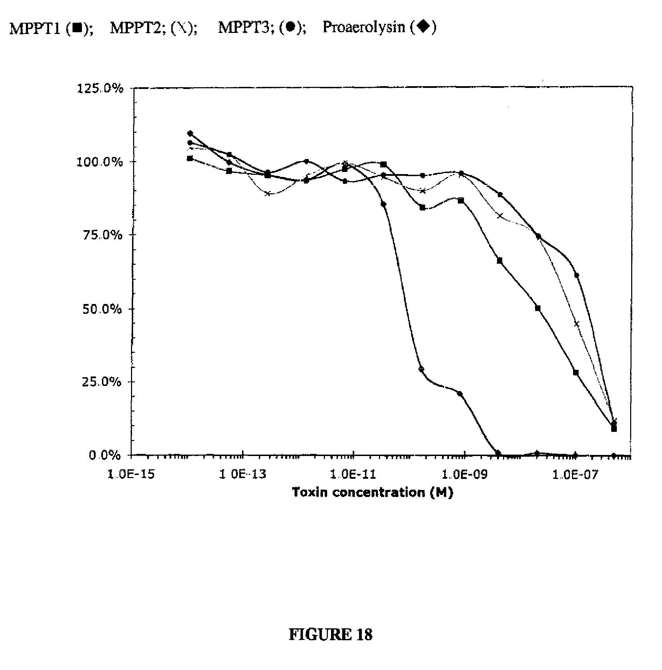

The toxicities of MPPT1, MPPT2, and MPPT3 were tested in a cell killing assay with the A549 cell line Cell killing assays were performed with A549 cells that were grown in DMEM media (Gibco) containing 5% fetal bovine serum (FBS; Gibco) at 37 °C/5% CO₂/48 hrs. Media was pipetted from the cell culture plate and the cells were rinsed 3 times with 5 ml of PBS (Gibco). The A549 cells were trypsinized with 3 ml of 0 05% trypsin in 0 53 mM EDTA for 2 min at 37°C/5%CO2, and for 3 more minutes without trypsin, before being resuspended in fresh DMEM/5% FBS to give a cell concentration of 1.67 x 10⁵ cells/ml Each well of a 96 well titer plate had 40 µl of DMEM/5% FBS media added to it Sample was added (10 µl) to the first well in each of the first 7 rows of the plate, mixed by gently pipetting the contents of the well up and down, and 10 µl was transferred to the next well This serial 1:5 dilution was continued along the entire row, and then 60 µl of A549 was added to each well of 7 rows of the plate The 8^{th} row was a no-cell negative control. The plate was incubated at 37°C/5% CO₂ for 1 h 20 µl of Cell Titer 96 Aqueous One Solution Reagent (Promega) was added to each well, and incubation continued for 3 more hours at 37°C/5%CO₂. The absorbance at 490 nm was read for each well, and the cell viability was calculated by subtracting the A₄₉₀nm value of the negative control. The results in Figure 18 show that these MPPT1, MPPT2, and MPPT3 were much less active than native PA, but still able to kill A549 cells.

### EXAMPLE 6: IN VIVO EFFICACY OF MPPT1 AND MPPT2 IN A549 HUMAN LUNG CARCINOMA XENOGRAFT TUMORS

The ability of MPPTs according to the present invention to reduce tumor growth in mice was determined using an A549 human lung carcinoma xenograft model as follows On study day 0, 44 female Rag2m mice (T & B cell deficient) were inoculated subcutaneously on the back with 2 X 10⁶ A549 human lung carcinoma cells When average tumor size was 200 mm³, mice were treated intratumorally with test compound at a dose of 5 µg/mouse in 25 µL of phosphate-buffered saline, 1 mM EDTA, pH 7 4. Mice were monitored for tumor size and four tumor or treatment-related morbidity/mortality (body weight changes; altered gait, lethargy, gross magnifications of stress) over the course of the study Mice with ulcerated tumors, or tumors in excess of 1000 mm³ were terminated by CO₂ asphyxiation

### Test compound coding

| | |
|---|---|
| MPPT1 | Proaerolysin containing a uPA selective activation site |
| MPPT2 | Proaerolysin containing a uPA selective activation site and with AFA antibody attached |
| Control 1 | Native proaerolysin |
| Control 2 | PBS/EDTA buffer |

All of the test compounds have a His tag at the C-terminus of proaerolysin

### Method of Group Assignment

Mice were randomized to groups on the basis of tumor volume.

### Results

### Test Compound Administration

One mouse in group two had 35 µL injected rather than the required 25 µL due to some leakage of test compound that occurred during the injection. All other mice had test compounds administered as per the study protocol No adverse reactions were noted for five of the test compounds, however 3 of the 5 mice injected with native proaerolysin were found dead within two days

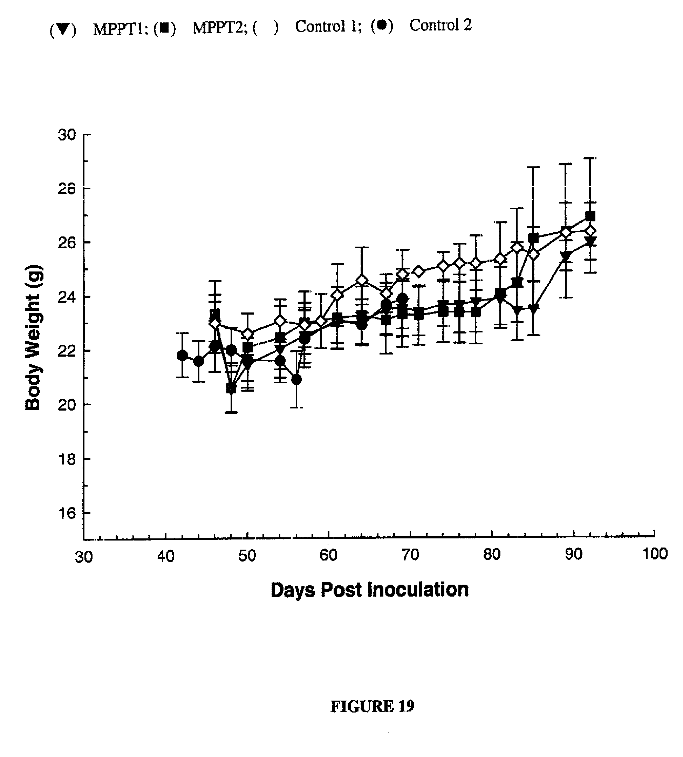

Figure 19 shows the effect on body weight of mice treated with MPPTs Although there were no significant differences in body weights between groups, a decrease in average body weight was noted in mice receiving MPPT1 and MPPT2 two days following test compound administration (12% loss) Average body weight in these groups had recovered to within 5% of initial by day 8 (MPPT1) or day 4 (MPPT2) post administration and no further loss was noted

### Change in Tumor Volumes

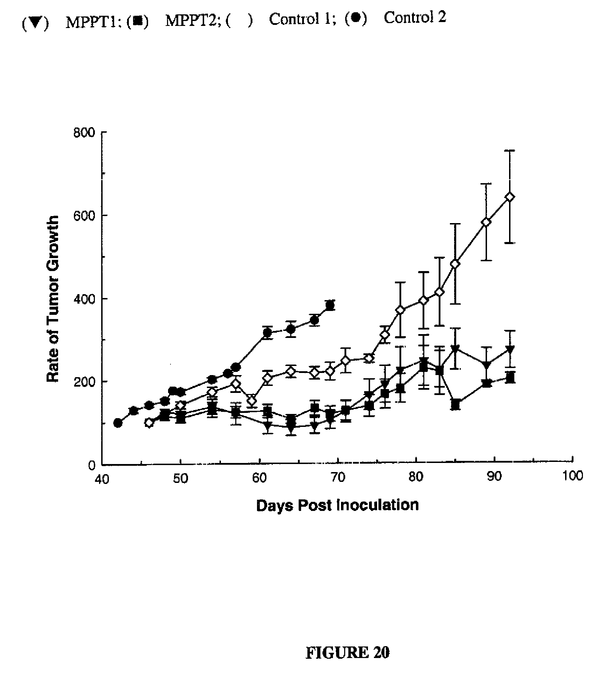

Administration of both MPPT1 and MPPT2 resulted in significant reduction in growth rate of A549 tumors compared to both the rate of growth of the PBS-EDTA injected control group and to the growth of tumors injected with proaerolysin (Control 1), as shown in Figure 20 Growth rate was expressed as tumor volume divided by initial tumor volume, multiplied by 100

### Observations

### Necropsy results

Small spleens were noted on termination in two mice from the group receiving MPPT2.

### Conclusions

Except for proaerolysin itself, test compounds were well tolerated when administered intratumorally. Proaerolysin with the native furin activation site will be toxic to most cells, because the cells display furin on their surfaces. MPPTs containing the uPA or proaerolysin polypeptide containing a PSA activation sequence are not activated by furin and would be expected to be less toxic than native proaerolysin in the absence of uPA or PSA

Intratumoral administration of MPPT1 or MPPT2 resulted in a significant reduction in the rate of subcutaneous A549 xenograft tumor growth in female Rag2m mice. This is consistent with the evidence that both uPA and the uPA receptor are upregulated in A549 tumors (1).

### References

1. Marshall B.C, Xu, Q P, Rao, N V, Brown, B R, and Hoidal, J. R. (1992 Pulmonary epithelial cell urokinase-type plasminogen activator. Induction by interleukin-1 beta and tumor necrosis factor-alpha. J Biol Chem 267:11462-9.

### EXAMPLE 7: ACTIVATION OF MPPT1 BY uPA - II

The ability of MPPT1 to be activated by uPA was determined again by methods similar to those described in Example 3. Both PA and MPPT1 were incubated at 0 4 mg/mL with 0, 0.016 mg/mL, 0.04 mg/mL or 0 08 mg/mL uPA in HBS, pH 7.4 for 120 minutes at room temperature., in a total volume of 62.5 µL After the incubation, 2 µg of each sample was removed and immediately loaded in 1x SDS-PAGE sample buffer onto 10% Bis-Tris NuPAGE gels in 1x MOPS-SDS running buffer under non-reducing conditions and run at 200 V constant voltage for 50 minutes followed by staining in Coomassie Brilliant Blue stain. Also, 4 µg of each sample was removed and inhibited in a final volume of 100 µl HBS with 1 mM PMSF for a hemolytic titer assay The hemolytic titer assay was carried out using a protocol similar to that described in Example 3. After serially diluting 1:2 in HBS across a 96 well plate, the samples were titered with 0 4% horse red blood cells (final concentration) incubated at 37°C for 1 hour. Titer, or number of wells containing lysed cells, was estimated visually

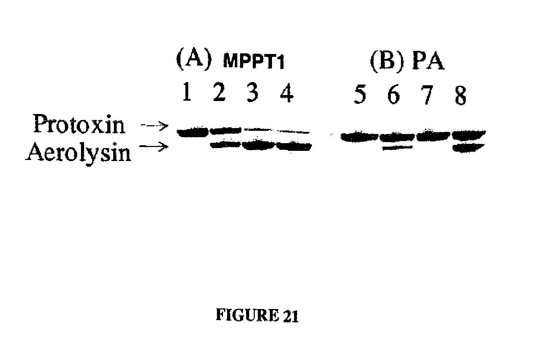

The results indicated that both PA and MPPT1 are activated by uPA, but MPPT1 is significantly more sensitive as seen in Figure 21 Figure 21 shows a Coomassie stained SDS-PAGE gel of urokinase digest of PA and MPPT1 after 120 minutes incubation at room temperature Each lane contains 2 µg of toxin Lanes: 1, MPPT1 undigested; 2, MPPT1 with 0 016 mg/mL uPA; 3, MPPT1 with 0 04 mg/mL uPA; 4, MPPT1 with 0 08 mg/mL uPA; 5, PA undigested; 6, PA with 0 04 mg/mL uPA; 7, PA with 0 016 mg/mL uPA; 8, PA with 0 08 mg/mL uPA After 120 minutes, the MPPT1 is almost completely activated by 0 04 mg/mL uPA, whereas much less PA was activated No PA was converted to aerolysin when treated with 0 016 mg/mL uPA under these conditions, whereas MPPT1 was nearly 50% converted.

The results of the corresponding hemolytic titer assay after 60 minutes of incubation are displayed in Table 7 below. As noted above, the hemolytic titers of PA and MPPT1 digested with a range of uPA concentrations for 120 minutes at room temperature were measured Titers were read after 60 minutes incubation at 37°C These data confirm the data shown in Figure 21 No activation of PA by treatment with 0016 mg/ml uPA was detected, yet MPPT1 was nearly 50% activated

**Table 7: Horse red blood cells Hemolytic titers of PA and MPPT1 after 120 minutes of incubation**

| | Hemolytic titer | | | |
|---|---|---|---|---|
| | Unactivated | 0.016 mg/mL uPA | 0.04 mg/mL uPA | 0.08 mg/mL uPA |
| PA | 0 | 0 | 2 5 | 6 |
| MPPT1 | 0 | 6 | 7.5 | 7.5 |

### EXAMPLE 8: ABILITY OF MPPT1 TO KILL CELLS EXPRESSING UROKINASE-TYPE PLASMINOGEN ACTIVATOR-RECEPTOR (uPAR)

The toxicity of MPPT1 towards uPAR expressing cells was determined by testing the ability of MPPT1 to kill HeLa cells or A2058 cells. Two 96 well Costar titer plates containing either HeLa or A2058 cells at an estimated 50% confluency were prepared for each adherent cell line. Since A2058 cells grow faster than HeLa cells, it was likely that there were more cells in the A2058 plates

The plates were washed twice in warmed plain DMEM media by pipetting off old media and replacing with 100 µl new media The cells were preincubated with or without 0.1 µg/mL pro-urokinase and 1 µg/mL glu-plasminogen in a final volume of 240 µl media for 30 minutes at 37°C, 5% CO₂ with humidity Cells that express the urokinase receptor will activate pro-urokinase under these conditions. Either PA or MPPT1 (1 mg/mL and 0.82 mg/mL, respectively) was added to the first well in each row, excluding those used for negative/negative and positive/negative controls, which received media to bring the volumes to 250 µL. The wells were serially diluted 1:5 in media with or without pro-urokinase and glu-plasminogen across the titer plate (200 µL remaining in each well). The plates were incubated for 1 hour at 37°C, 5% CO₂ with humidity.

To develop the plates, the old media was removed and 200 µL fresh media containing FBS was added back in addition to 50 µL of 2.5 mg/mL MTT. The plates were incubated as above for 80 minutes until purple precipitate became visible at the bottom of the wells.

The media was carefully removed without disturbing the precipitate and 100 µL of solubilization buffer (0 5% (w/v) SDS and 25 mM HCl in 90% (v/v) isopropanol) was added to each well. The openings of the wells were sealed with aluminum sealing foil and the plates were gently agitated at room temperature to solubilize the precipitate. The plates were measured at 560nm using a 96 well plate reader and the raw data was used to generate cell killing curves of the toxin concentration vs cell viability.

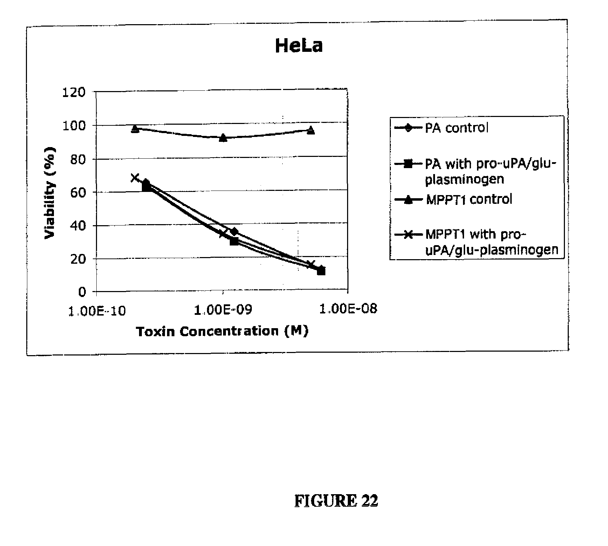

The results of the HeLa cell killing assay are shown in Figure 22. The cell killing curve, as seen in Figure 22, shows that the preincubation of uPAR-expressing HeLa cells with pro-uPA and glu-plasminogen (which results in the production of active uPA) enables MPPT1 to kill the cells as effectively as PA Without the preincubation step, the MPPT1 shows little cell killing activity. This is because the normal furin activation site has been removed

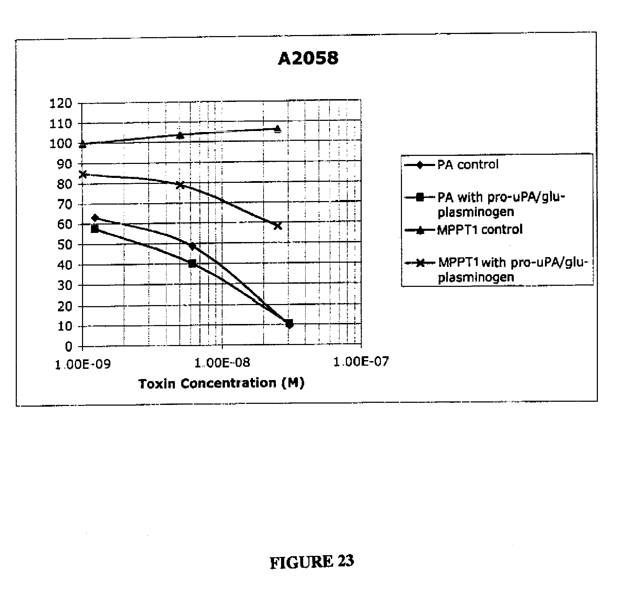

Similarly, as shown in Figure 23, the cell killing curve for A2058 cells indicates that the preincubation of A2058 cells with pro-uPA and glu-plasminogen increases the toxicity of MPPT1, although the uPA-sensitive toxin was not able to kill as efficiently as PA

### EXAMPLE 9: ABILITY OF MPPT1 TO KILL EL4 MOUSE LYMPHOMA CELLS

The toxicity of MPPT1 towards non-uPAR expressing cells was determined by testing its ability to kill EL4 cells as follows EL4 cells were prepared to 1 x10⁶ cells/mL suspended in media with or without 0.1 µg/mL pro-urokinase and 1 µg/mL glu-plasminogen and incubated at 37°C, 5% CO₂ with humidity for 30 minutes The PA and MPPT1 (1 mg/mL and 0 82 mg/mL, respectively) were serially diluted 1/5 in triplicate across two 96 well plates in 20 µL media. The pre-incubated cells were added to each well (80 µL), excluding negative control and the plates were further incubated for 1 hour as above To develop the assay, 20 µL Promega cell killing media (CellTiter 96AQueous One Solution Cell Proliferation Assay) was added to each well and incubated further for 4 hours as above The bubbles were popped in each well by the addition of 7 µL 70% isopropanol and the plates were measured at 490nm using a Bio-Tek plate reader. The raw absorbance data was used to produce cell killing curves of toxin concentration vs. the percentage of viable cells.

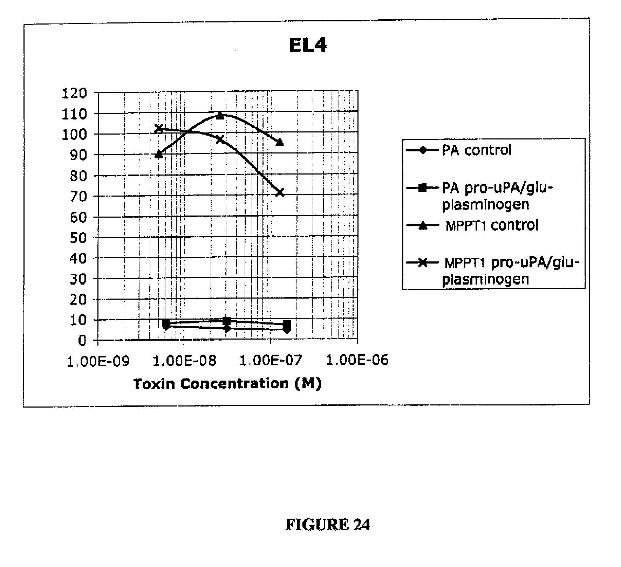

Figure 24 depicts the cell killing curve for EL4 cells, and indicates that preincubation of the cells with pro-uPA and glu-plasminogen does not lead to activation of MPPT1. This is a necessary control, since EL4 cells do not express the uPA receptor needed to generate active uPA under these conditions

The data shown in Examples 7 to 9 indicate that replacing the native furin activation site of PA with one that is specific for urokinase creates a variant, MPPT1, that is toxic to cells expressing urokinase receptors when pro-urokinase and glu-plasminogen are present.

### EXAMPLE 10: PRODUCTION OF AN MPPT (MPPT4) THAT CAN BE ACTIVATED BY MATRIX METALLOPROTEINASE 2 (MMP2)

MPPT4, derived from proaerolysin and containing an activation sequence modified to include a single cleavage site cleavable by a general activating agent was prepared by replacing the native furin activation site with a sequence cleavable by MMP2 The MPPT4 was made by 3-step PCR and purified using nickel affinity and anion exchange chromatography as described below.

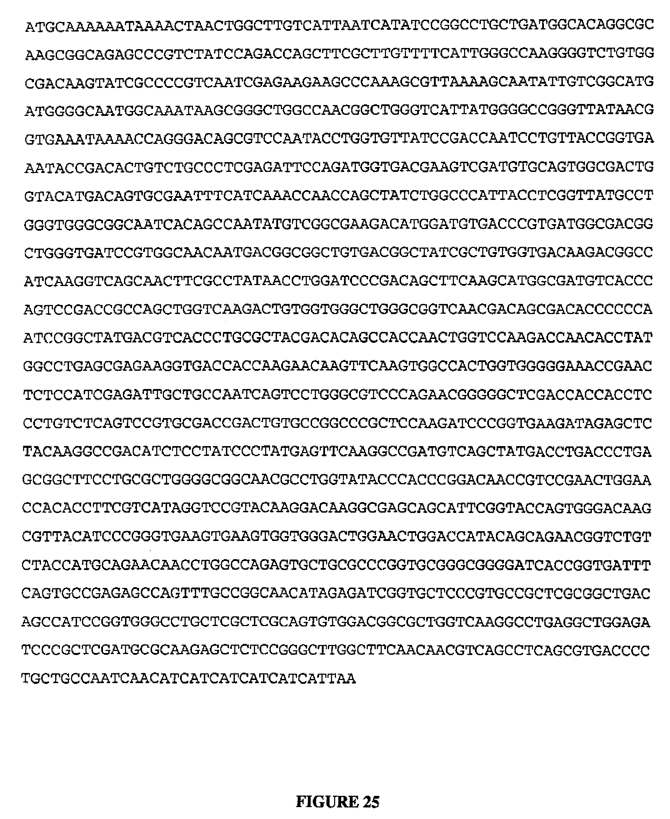

Cloning of MPPT4, derived from proaerolysin and having the activation sequence (KVRRAR) changed to that of MMP2 (HPVGLLAR) was prepared by three rounds of Site Directed Mutagenesis (SDM) using the Quick Change Kit (Stratagene) according to the manufacturer's instructions. In the first SDM, plasmid pTZ18U containing the gene coding for aerolysin with a 6xHis tag at its C-terminus was used as a template, with the pair of primers MMP2-1fwd and MMP2-1rev (Table 8) The second SDM was peifoimed using the product of the first SDM as a template and the pair of primers - MMP2-2fwd and MMP2-2rev (Table 8). The third SDM was performed using the product of the second SDM as a template and the pair of primers MMP2-3fwd and MMP2-3rev (Table 8) After the desired changes in the final product were confirmed by DNA sequencing, the mutated *aerA* gene was cloned (as *Hin*dIII - *Eco*RI fragment) into the broad host-range expression vector pMMB66HE Recombinant clones were then transferred into *Aeromonas salmonicida* CB3 cells by conjugation using the filter-mating technique The nucleotide sequence of MPPT4 (SEQ ID NO:38) is shown in Figure 25

**Table 8. Primers used in Quick Change reactions**

| Primer | Sequence | SEQ ID NO |
|---|---|---|
| MMP2-1fwd | CTCGCGGCTGACAGCCATCCGGTGCGTGCTCGCAG | 26 |
| MMP2-1rev | CTGCGAGCACGCACCGGATGGCTGTCAGCCGCGAG | 27 |
| MMP2-2fwd | GACAGCCATCCGGTGGGCCTGCTCAGTGTGGACGGC | 28 |
| MMP2-2rev | GCCGTCCACACTGAGCAGGCCCACCGGATGGCTGTC | 29 |
| MMP2-3fwd | CCGGTGGGCCTGGTCGCTCGCAGTGTGGACGGC | 30 |
| MMP2-3rev | GCCGTCCACACTGCGAGCGAGCAGGCCCACCGG | 31 |

MPPT4 was expressed and purified as follows A single colony of CB3 MPPT4::pMMB66 (CB3 strain containing a plasmid expressing MPPT4) was inoculated into 30 mL of LB media (10 % Bacto Tryptone, 5% Bacto Yeast Extract, 10% NaCl, pH 7 5) containing Davis minimal media, 0 2% glucose, 100 µg/mL ampicillin and 40 µg/mL of both kanamycin and rifampicin The culture was grown at 27°C with shaking at 250 rpm overnight to an OD₆₀₀ₙₘ of 3 76 The culture was inoculated (1%) into 5x 2 L flasks containing a final volume of 500 mL of the above media excluding rifampicin. Cultures were grown as above for 5 25 hours to OD₆₀₀ₙₘ from 0 59-0 68 and induced with a final concentration of 1 mM IPTG The induced cultures were incubated as above for 17 5 hours The culture supernatant was harvested by pelleting the cells at 10,000 rpm in a JA16 25 rotor for 15 minutes at 4°C. A few drops of polypropylene glycol were added to the supernatant to prevent frothing during concentration with a KVICK SCU concentrator (Amersham, 10,000 kDa cutoff) The supernatant was concentrated from 2.5 L to 80 mL and aliquoted in 20 mL fractions for storage at-20°C.

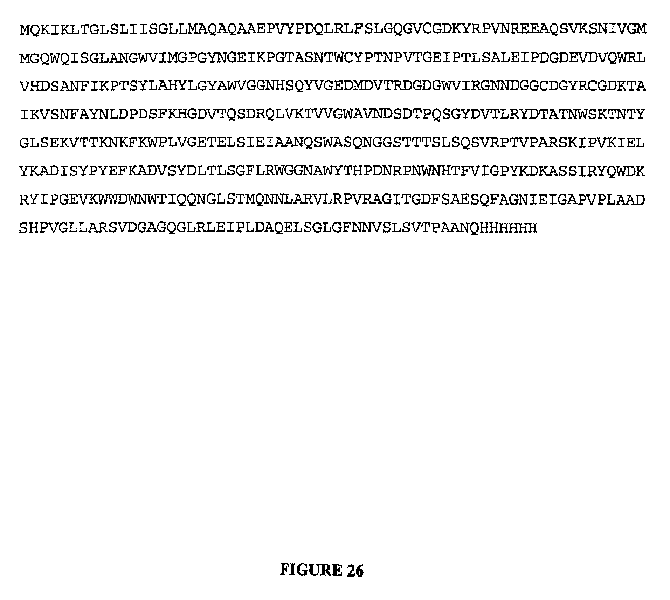

An aliquot of MPPT4 supernatant was thawed and clarified by centrifugation in a JA25.5 rotor at 10,000 rpm for 10 minutes at 4°C. The clarified supernatant was loaded at 1 mL/min onto a 10 mL FPLC nickel affinity column (Amersham Chelating Sepharose Fast Flow resin) equilibrated in Buffer A (20 mM Na₂HPO₄, 0.5M NaCl, 10 mM imidazole, pH 74). The column was washed at 5 mL/min with 50 mL of Buffer A and eluted with 60% Buffer B (20 mM Na₂HPO₄, 0 5M NaCl, 0 5M imidazole, pH 7.4) at approximately 300 mM imidazole. The toxin containing fractions were desalted into 20 mM Tris, 1 mM EDTA, 150 mM NaCl, pH 7.4 using PD-10 exchange columns (Amersham) The desalted material was loaded at 1 mL/min onto an FPLC HiPrep 16/10 Q FF anion exchange column (Amersham) equilibrated in the same buffer as used for desalting The column was washed in equilibration buffer and eluted with a linear gradient of 0 15-1 M NaCl in 20 mM Tris, 1 mM EDTA, pH 7.4 Concentrations were determined by measuring absorbance at 280nm. The purification yielded pure protein as seen on Coomassie stained SDS-PAGE (not shown). The total yield of' protein was 8.6 mg from 625 mL culture supernatant. The amino acid sequence of MPPT4 (SEQ ID NO:39) is shown in Figure 26.

### EXAMPLE 11: SENSITIVITY OF MPPT4 TO CLEAVAGE BY MMP2

The sensitivity of MPPT4 to MMP2 was demonstrated using silver stained SDS-PAGE and Western blotting as follows. Both wild type PA and MPPT4 were incubated at a concentration of 0 02 mg/mL with 0, 0.5 µg, 0.25 µg, 0.125 µg and 0.0625 µg of active MMP2 protease (EMD/Calbiochem) in HBS, 10 mM CaCl₂, pH 7 4 for 3 hours, 24 hours and 48 hours at 37°C. Digestion was stopped with a final concentration of 1 µM 1,10-o-phenanthroline followed by chilling on ice

Samples were prepared for electrophoresis by removing 10 µL inhibited material into 5 µL dH₂O and 5 µL 4x SDS-PAGE sample buffer (SB) and 10 µL of this mixture were loaded onto 10% Bis-Tris NuPAGE gels in 1x MOPS-SDS non-reduced running buffer. Gels were run at 200 V constant voltage for 50 minutes. The 3 hour and 24 hour sample gels were silver stained using a SilverXpress silver staining kit (Invitrogen) as per manufacturer's instructions.

After electrophoresis, the 48 hour samples were transferred to a nitrocellulose membrane by electroblotting at 30 V constant for 1 hour in 1x transfer buffer (NuPAGE) with 10% methanol. The membrane was probed with polyclonal antibody anti-AerA at 1/4000 dilution followed by goat anti-rabbit polyclonal antibody conjugated with alkaline phosphatase (Calbiochem) at 1/4000 dilution and developed with NBT/BCIP.

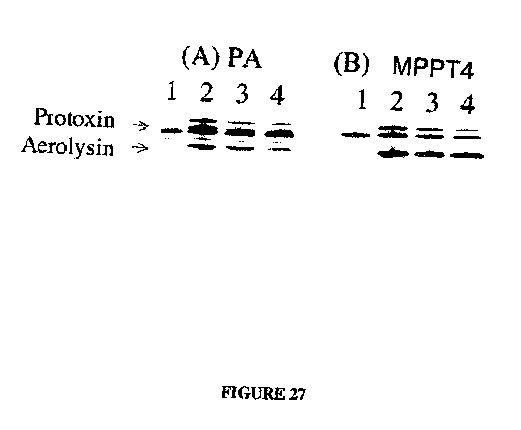

The results indicated that the silver stained gel of' the 3 hour incubation samples showed little digestion for PA and complete digestion for MPPT4 (not shown) Figure 27 shows a silver stained SDS-PAGE gel of' PA and MPPT4 digested for 24 hours with MMP2 at 37°C showing 100 ng of each sample ranging from 0-0.5 µg MMP2. The samples in each lane are: lane 1, no protease control; lane 2, 0.5 µg MMP2; lane 3, 0.25 µg MMP2; lane 4, 0.125 µg MMP2 Note that several of the bands in this figure are contributed by the MMP2 that was used. They do not represent contaminants or breakdown products in the PA and MPPT4 preparations. The silver stained gel for 24 hour incubation samples seen in Figure 27 showed a small amount of digestion for PA and complete digestion for MPPT4 for all concentrations shown.

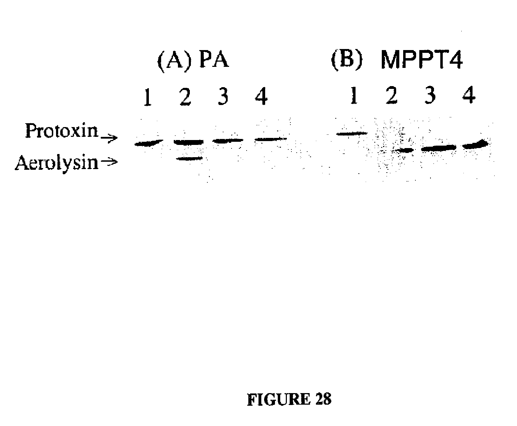

Figure 28 shows the Western blot of 100 ng of PA and MPPT4 digested for 24 hours with 0-0.5 µg MMP2 The blot was probed with polyclonal anti-aerA antibody followed by goat anti-rabbit polyclonal antibody/alkaline phosphatase conjugate both at 1/4000 dilution and developed with NBT/BCIP Samples in the lanes of the gel are identified as follows: lane 1, no protease control; lane 2, 0.5 µg MMP2; lane 3, 0.25 µg MMP2; lane 4, 0.125 µg MMP2. The Western blot for the 48 hour incubation samples seen in Figure 28 shows that there is less than 50% digestion for PA and complete digestion for MPPT4.

### EXAMPLE 12: ABILITY OF MPPT4 TO KILL HT 1080 HUMAN FIBROSARCOMA CELLS

A cell killing assay was performed with the MMP2 expressing cell line HT 1080 Both wild type PA and MPPT4 (toxins) were incubated with HI 1080 cells in a cell killing assay.

The HT 1080 cells were prepared to a concentration of 1x10⁶ cells/mL in fresh media. The toxins were serially diluted in 96 well plates in triplicate starting with 5 µl toxin (PA at 1 mg/mL; MPPT4 at 1.288 mg/mL) in 20 µL media. The toxins were serially diluted 1/5 across the plate Both a negative/negative and a negative/positive control were also prepared for each plate The cells were added to a final volume of 100 µL and incubated for 1 hour at 37°C, 5% CO₂ with humidity.

To develop the assay, 20 µL Promega cell killing media (CellTiter 96AQueous One Solution Cell Proliferation Assay) was added to each well and incubated further for 2 hours The bubbles were popped in each well by the addition of 7 µL 70% isopropanol and the plates were measured at 490nm using a Bio-Tek plate reader The raw absorbance data was used to produce cell killing curves of toxin concentration vs the percentage of viable cells

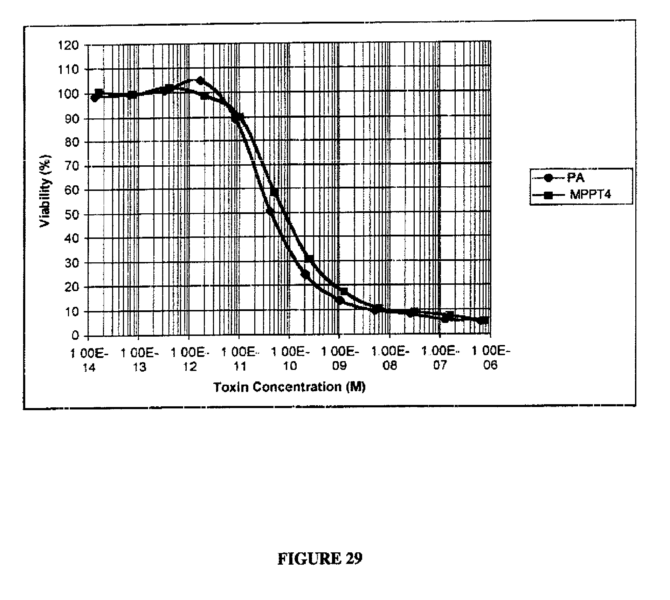

The results in Figure 29 show that HT 1080 cells are nearly equally sensitive to PA and MPPT4 (LC₅₀s of 4x10⁻¹¹ M and 8x10⁻¹¹ M, respectively). These cells can activate PA with furin and they can activate MPPT4 with MMP2.

### EXAMPLE 13: ABILITY OF MPPT4 TO KILL EL4 MOUSE LYMPHOMA CELLS

Both wild type PA and MPPT4 (toxins) were incubated with EL4 cells (non-MMP2 expressing cells) in a cell killing assay.

The EL4 cells were prepared to 0.82 x 10⁶ cells/mL in fresh media The toxins were serially diluted in 96 well plates in triplicate starting with 5 µl toxin (PA at 1 mg/mL; MPPT4 at 1 288 mg/mL) in 20 µL media. The toxins were serially diluted 1/5 across the plate Both a negative/negative and a negative/positive control were also prepared for each plate. The cells were added to a final volume of 100 µL and incubated for 1 hour at 37°C, 5% CO₂ with humidity.

To develop the assay, 20 µL Promega cell killing media (CellTiter 96AQueous One Solution Cell Proliferation Assay) was added to each well and incubated further for 4 hours as above. The bubbles were popped in each well by the addition of 7 µL 70% isopropanol and the plates were measured at 490nm using a Bio-Tek plate reader. The raw absorbance data was used to produce cell killing curves of toxin concentration vs the percentage of viable cells.

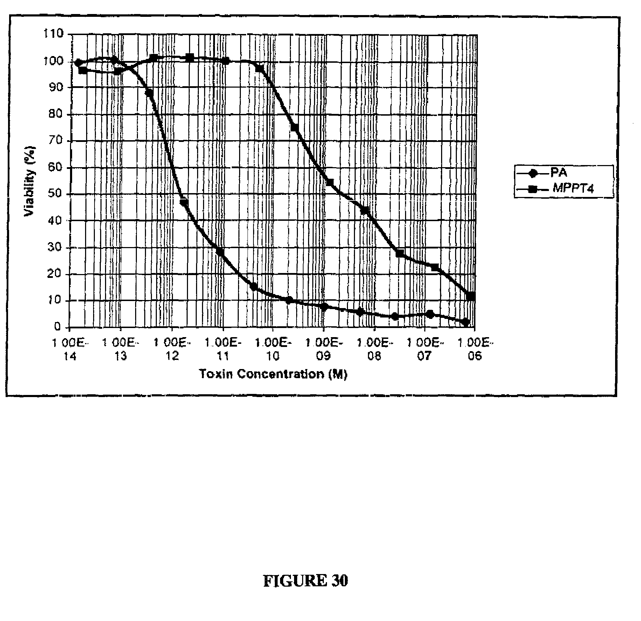

The cell killing curves for EL4 cells, which do not produce MMP2, indicated that MPPT4 is much less toxic than PA (Figure 30) This is indicated by the difference in concentration required for the toxin to kill 50% of the cells PA requires 1.5x10⁻¹² M, whereas MPPT4 requires 2x10⁻⁹ M under the same conditions. The EL4 cells can convert PA to aerolysin using furin, however MPPT4 lacks a furin activation site

The results depicted in Examples 11 TO 13 indicate that MPPT4 is activated by MMP2. Because the furin activation site has been removed, MPPT4 is much less toxic to normal cells MPPT4 is as toxic as native PA to cells that express MMP2

### EXAMPLE 14: PRODUCTION OF AN MPPT (MPPT5) THAT CAN BE ACTIVATED BY EITHER uPA OR MMP2

An MPPT derived from proaerolysin was designed that can be activated by either of two different proteases, matrix metalloproteinase 2 (MMP2) and urokinase plasminogen activator (uPA), but that cannot be activated by furin The MPPT5 was cloned by multi-step PCR and it was purified using nickel affinity and anion exchange chromatography as described below

Cloning of MPPT5 was carried out in two stages The first stage was the cloning of PA-MMP2 This variant of proaerolysin with the native activation sequence (K⁴²⁷VRRAR) changed to that of MMP2 (HPVGLLAR) was prepared by three consecutive rounds of' Site Directed Mutagenesis (SDM) using the Quick Change Kit (Stratagene) according to the manufacturer's instructions In the first SDM, the plasmid pTZ18U containing the gene coding for proaerolysin with a 6xHis tag at its C-terminus was used as a template, with the pair of primers MMP2-1fwd and MMP2-1rev (Table 9). The second SDM was performed using the product of the first SDM as a template and the pair of primers - MMP2-2fwd and MMP2-2rev (Table 9). The third SDM was performed using the product of' the second SDM as a template and the pair of primers MMP2-3fwd and MMP2-3rev (Table 9) After the desired changes in the final product were confirmed by DNA sequencing, the mutated *aerA* gene was cloned (as *Hind*III - *Eco*RI fragment) into the broad host-range expression vector pMMB66HE. Recombinant clones were then transferred into *Aeromonas salmonicida* CB3 cells by conjugation using the filter-mating technique

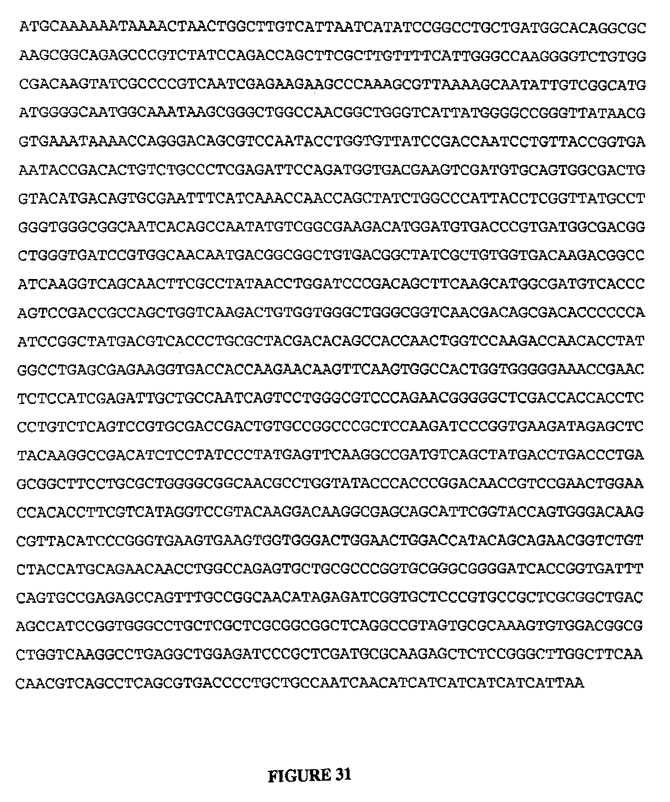

The second stage was the cloning of PA-MMP2-uPA (MPPT5). This variant of proaerolysin with the activation sequence MMP2 (HPVGLLAR) connected to that of' uPA (SGRSAQ) by a GG linker was prepared by three subsequent rounds of SDM. In the first SDM plasmid pTZ18U containing the gene coding for aerolysin with the MMP2 activation sequence and a 6xHis tag at its C-terminus was used as a template, with the pair of primers MMPuPA1fwd and MMPuPA1rev (Table 9) The second SDM was performed using the product of the first SDM as a template and the pair of primers - MMPuPA2fwd and MMPuPA2rev (Table 9) The third SDM was performed using the product of the second SDM as a template and the pair of primers MMPuPA3fwd and MMPuPA3rev (Table 9). After the desired changes in the final product were confirmed by DNA sequencing, the mutated *aerA* gene was cloned (as *Hin*dIII *-Eco*RI fragments) into broad host-range expression vector pMMB66HE Recombinant clones were then transferred into *Aeromonas salmonicida* CB3 cells by conjugation using the filter-mating technique The nucleotide sequence of MPPT5 (SEQ ID NO:40) is shown in Figure 31

**Table 9. Primers used in Quick Change reactions**

| Primer | Sequence | SEQ ID NO |
|---|---|---|
| MMP2-1fwd | CTCGCGGCTGACAGCCATCCGGTGCGTGCTCGCAG | 26 |
| MMP2-1rev | CTGCGAGCACGCACCGGATGGCTGTCAGCCGCGAG | 27 |
| MMP2-2fwd | GACAGCCATCCGGTGGGCCTGCTCAGTGTGGACGGC | 28 |
| MMP2-2rev | GCCGTCCACACTGAGCAGGCCCACCGGATGGCTGTC | 29 |
| MMP2-3fwd | CCGGTGGGCCTGGTCGCTCGCAGTGTGGACGGC | 30 |
| MMP2-3rev | GCCGTCCACACTGCGAGCGAGCAGGCCCACCGG | 31 |
| MMPuPA1fwd | GGCCTGCTCGCTCGCGGCGGCTCAAGTGTGGACGGC | 32 |
| MMPuPA1rev | GCCGTCCACACTTGAGCCGCCGCGAGCGAGCAGGCC | 33 |
| MMPuPA2fwd | GCTCGCGGCGGCTCAGGCCGTAGTGTGGAGGGC | 34 |
| MMPuPA2rev | GCCGTCCACACTACGGCCTGAGCCGCCGCGAGC | 35 |
| MMPuPA3fwd | GGCTCAGGCCGTAGTGCGCAAAGTGTGGACGGC | 36 |
| MMPuPA3rev | GCCGTCCACACTTTGCGCACTACGGCCTGAGCC | 37 |

### Expression and purification of MPPT5

A single colony of CB3 MPPT5::pMMB66 (CB3 strain containing a plasmid expressing MPPT5)was inoculated into 30 mL of LB media (10 % Bacto Tryptone, 5% Bacto Yeast Extract, 10% NaCl, pH 7.5) containing Davis minimal media, 0.2% glucose, 100 µg/mL ampicillin and 40 µg/mL of both kanamycin and rifampicin The culture was grown at 27°C with shaking at 250 rpm overnight to an OD₆₀₀ₙₘ of 4 62 The culture was subinoculated (1%) into 5x 2 L flasks containing a final volume of 500 mL of the above media excluding rifampicin Cultures were grown as above for 5.5 hours to OD₆₀₀ₙₘ from 0.95-0.99 and induced with a final concentration of 1 mM IPTG The induced cultures were incubated as above for 18 hours. The culture supernatant was harvested by pelleting the cells at 10,000 rpm in a JA1625 rotor for 15 minutes at 4°C. A few drops of polypropylene glycol were added to the supernatant to prevent frothing during concentration with a KVICK SCU concentrator (Amersham, 10,000 kDa cutoff). The supernatant was concentrated from 2.5 L to 100 mL and aliquoted in 25 mL fractions for storage at -20°C

An aliquot of MPPT5 supernatant was thawed and clarified by centrifugation in a JA25.5 rotor at 10,000 rpm for 10 minutes at 4°C. The clarified supernatant was loaded at 1 mL/min onto a 10 mL FPLC nickel affinity column (Amersham Chelating Sepharose Fast Flow resin) equilibrated in Buffer A (20 mM Na₂HPO₄, 0.5M NaCl, 10 mM imidazole, pH 7 4). The column was washed at 5 mL/min with 50 mL of Buffer A and eluted with 60% Buffer B (20 mM Na₂HPO₄, 0 5M NaCl, 0.5M imidazole, pH 7 4) at approximately 300 mM imidazole. The toxin containing fractions were desalted into 20 mM Tris, 1 mM EDTA, 150 mM NaCl, pH 7.4 using PD-10 exchange columns (Amersham) The desalted material was loaded at 1 mL/min onto an FPLC HiPrep 16/10 Q FF anion exchange column (Amersham) equilibrated in the same buffer as used for desalting The column was washed in equilibration buffer and eluted with a linear gradient of 0 15-1 M NaCl in 20 mM Tris, 1 mM EDTA, pH 74 Concentrations were determined by measuring absorbance at 280nm.

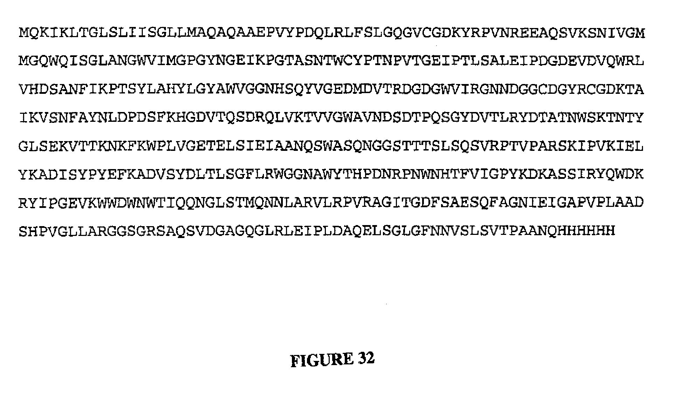

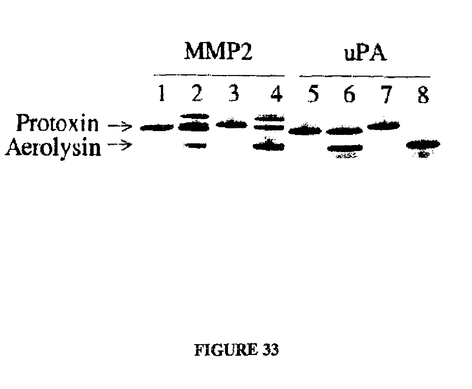

This purification procedure yielded pure protein as determined by silver stained SDS-PAGE (Figure 33) The amino acid sequence of MPPT5 (SEQ ID NO:41) is depicted in Figure 32

### EXAMPLE 15: ACTIVATION OF MPPT5 BY MMP2 AND uPA

The sensitivity of MPPT5 to MMP2 and uPA was demonstrated using silver stained SDS-PAGE and a hemolytic titer assay

*Digestion of MPPT5 with MMP2.* PA and MPPT5 were prepared in HBS, 10 mM CaCl₂, pH 7.4 to a concentration of 0.06 mg/mL with or without the addition of 0.5 µg of MMP2 in a total of 60 µL The reaction mixtures were incubated for 2 hours at 37°C and then inhibited with a final concentration of 1 µM 1,10-o-phenanthroline followed by chilling on ice.

*Hemolytic titer*. This assay was carried out as described in Example 3 The titer value, number of wells containing lysed cells, was estimated visually after incubation at 37°C for 1 hour

*Silver stained SDS*-*PAGE* For each reaction mixture, 100 ng of material was prepared in 1x sample buffer and run on a 10% Bis-Tris NuPAGE gel in 1x MOPS-SDS running buffer under non-reduced conditions for 50 minutes at 200 V constant voltage The gel was silver stained using a SilverXpress silver staining kit (Invitrogen) following the manufacturer's instructions

Figure 33 depicts a silver stained gel of PA and MPPT5 digested with MMP2 or uPA for 2 hours at 37°C Each lane contains 100 ng of material identified as follows: lane 1, PA control; lane 2, PA with MMP2; lane 3, MPPT5 control; lane 4, MPPT5 with MMP2; lane 5, PA control; lane 6, PA with uPA; lane 7, MPPT5 control; lane 8, MPPT5 with uPA

The results shown in Figure 33 indicate that MPPT5 is sensitive to digestion by both MMP2 and uPA PA is not measurably affected by MMP2, but it is somewhat sensitive to uPA, though not to the degree seen for MPPT5 The aerolysin product of the uPA digestion is slightly larger than that of the MMP2 product This indicates that the uPA cuts only at the uPA site, which follows the MMP2 site after a 2 amino acid linker, and that uPA does not cut at the MMP2 site.

The corresponding hemolytic titer data is displayed in Table 10. Titers were read after 60 minutes incubation at 37°C. The data confirm that PA was not activated by MMP2, and only partially activated by uPA, whereas MPPT5 was activated by both proteases PA and MPPT5 were digested with MMP2 and uPA for 120 minutes at 37°C,

**Table 10. Hemolytic titers of proaerolysin and MPPT5**

| | Control in HBS, 10 mM CaCl₂ | Control in HBS | MMP2 | uPA |
|---|---|---|---|---|
| PA | 0 | 0 | 0 | 5 |
| MPPT5 | 0 | 0 | 6.5 | 7 |

### EXAMPLE 16: ABILITY OF MPPT5 TO KILL HT 1080 HUMAN FIBROSARCOMA CELLS

The ability of MPPT5 to kill MMP2 expressing cells was tested as follows Both wild type PA and MPPT5 were incubated with HT 1080 cells (MMP2 expressing) in a cell killing assay The HT 1080 cells were prepared to a concentration of 1x10⁶ cells/mL in fresh media The proteins were serially diluted in 96 well plates in triplicate starting with 5 µl toxin (PA at 1 mg/mL; MPPT5 at 146 mg/mL) in 20 µL media The toxins were serially diluted 1/5 across the plate. Both a negative/negative and a negative/positive control were also prepared The cells were added to a final volume of 100 µL and incubated for 1 hour at 37°C, 5% CO₂ with humidity To develop the assay, 20 µL Promega cell killing media (CellTiter 96AQueous One Solution Cell Proliferation Assay) was added to each well and incubated further for 2 hours as above The bubbles were popped in each well by the addition of 7 µL 10% isopropanol and the plates were measured at 490nm using a Bio-Tek plate reader The raw absorbance data was used to produce cell killing curves of toxin concentration vs the percentage of viable cells.

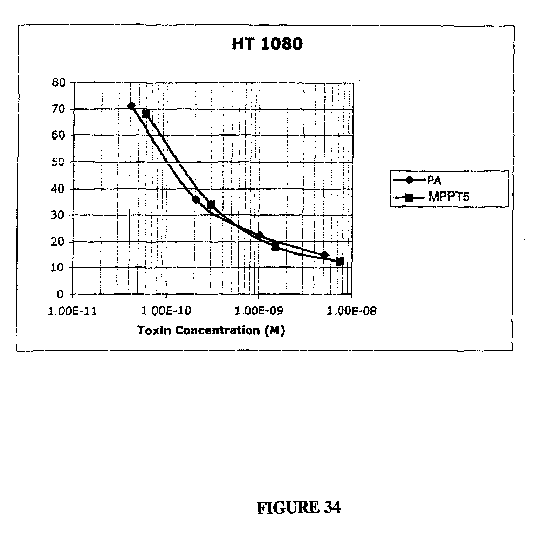

The cell killing data for HT 1080 (MMP2 expressing) cells, as shown in Figure 34, indicates that HT 1080 cells were nearly equally sensitive to PA and PA-MMP2 (LC50's of 1x10⁻¹⁰ M and 1 5x10⁻¹⁰ M, respectively) These cells were able to activate PA with furin and they could activate MPPT5 with MMP2

### EXAMPLE 17: ABILITY OF MPPT5 TO KILL HELA HUMAN CERVICAL CANCER CELLS OR A2058 HUMAN MELANOMA CELLS

The ability of MPPT5 to kill urokinase receptor expressing cells, with and without the addition of pro-uPA and glu-plasminogen was tested as follows. The urokinase receptor expressing cell lines used were HeLa cells and A2058 cells. Two 96 well Costar titer plates for each adherent cell line were prepared containing either HeLa or A2058 cells at an estimated 50% confluency Since A2058 cells grow faster than HeLa cells, it was likely that there were more cells in the A2058 plates. The plates were washed twice in warmed plain DMEM media by pipetting off old media and replacing with 100 µl new media, The cells were preincubated with or without 0.1 µg/mL pro-urokinase and 1 µg/mL glu-plasminogen in a final volume of 240 µl media for 30 minutes at 37°C, 5% CO₂ with humidity. Either PA or MPPT5 (1 mg/mL and 146 mg/mL, respectively) was added to the first well in each row excluding those used for negative/negative and positive/negative controls, which received media to bring the volumes to 250 µL The wells were serially diluted 1:5 in media with or without pro-urokinase and glu-plasminogen across the titer plate (200 µL, remaining in each well). The plates were incubated for 1 hour at 37°C, 5% CO₂ with humidity

To develop the plates, the old media was removed and 200 µL of fresh media containing FBS was added back in addition to 50 µL of 2.5 mg/mL MTI The plates were incubated as above for 80 minutes until purple precipitate became visible at the bottom of the wells.

The media was carefully removed without disturbing the precipitate and 100 µL of solubilization buffer (0.5% (w/v) SDS and 25 mM HCl in 90% (v/v) isopropanol) was added to each well The openings of the wells were sealed with aluminum sealing foil and the plates were gently agitated at room temperature to solubilize the precipitate Plates were stored overnight at 4°C.

The plates were measured at 560nm using a 96 well plate reader and the raw data was used to produce cell killing curves of protein concentration vs cell viability.

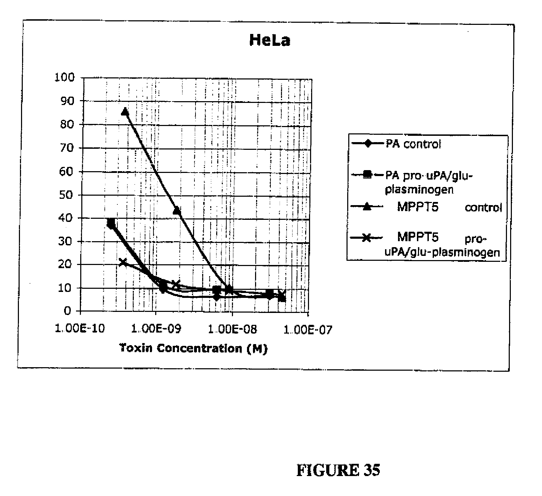

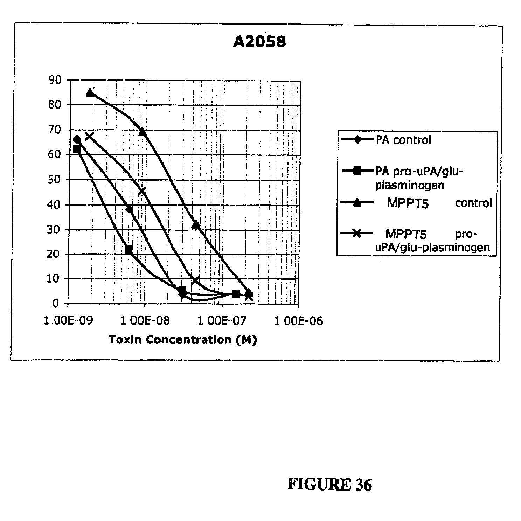

The cell killing curves for HeLa and A2058 (uPAR expressing) cells, as shown in Figures 35 and 36, respectively, indicate that both cell lines are sensitive to PA and MPPT5, although cellular toxicity of the MPPT5 toxin is significantly increased by the addition of both pro-uPA and glu-plasminogen This indicates that both HeLa and A2058 cells are capable of facilitating the production of active uPA via interactions at uPAR expressed on the cell surface The toxicity of PA was not affected by the addition of pro-uPA and glu-plasminogen

### EXAMPLE 18: ABILITY OF MPPT5 TO KILL MOUSE LYMPHOMA EL4 CELLS

The ability of MPPT5 to kill cells that do not express urokinase receptor, with and without the addition of pro-uPA and glu-plasminogen was determined as follows. Both wild type PA and MPPT5 (toxins) were incubated with EL4 cells in a cell killing assay with or without the addition of pro-uPA/glu-plasminogen Two batches of EL4 cells were prepared to a concentration of 1x10⁶ cells/mL in fresh media To one batch, pro-uPA and glu-plasminogen were added to 0.1 µg/mL and 1 µg/mL, respectively Both preparations were incubated for 30 minutes at 37°C, 5% CO₂ with humidity

The toxins were serially diluted in 96 well plates in triplicate starting with 5 µl toxin (PA at 1 mg/mL; MPPT5 at 1.46 mg/mL) in 20 µL media The toxins were serially diluted 1/5 across the plate. Both a negative/negative and a negative/positive control were also prepared The cells were added to a final volume of 100 µL and incubated for 1 hour at 37°C, 5% CO₂ with humidity.

To develop the assay, 20 µL Promega cell killing media (CellTiter 96AQueous One Solution Cell Proliferation Assay) was added to each well and incubated further for 4 hours as above. The bubbles were popped in each well by the addition of 7 µL 70% isopropanol and the plates were measured at 490nm using a Bio-Tek plate reader The raw absorbance data was used to produce cell killing curves of toxin concentration vs the percentage of viable cells.

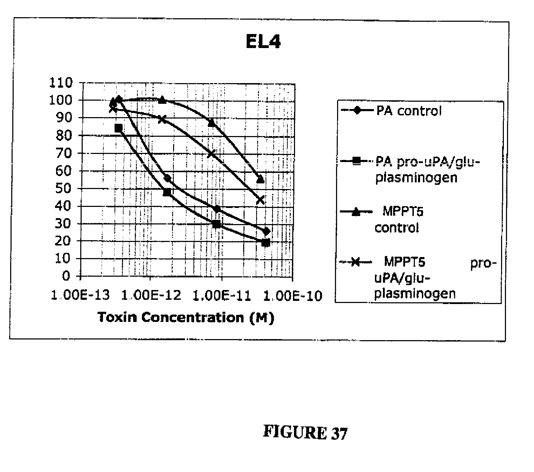

The cell killing curves for EL4 (non-MMP2 expressing and non-uPAR expressing) cells, shown in Figure 37, indicate that EL4 cells are less sensitive to MPPT5 than to PA The EL4 cells can convert native PA to aerolysin using furin, however MPPT5 lacks a furin activation site The addition of pro-uPA and glu-plasminogen did not significantly increase the toxicity of either protein, as EL4 cells do not express uPAR, which is required for the production of active uPA

The data shown in Examples 15 to 18 indicate that MPPT5 can be activated by either MMP2 or uPA. Because the furin activation site has been removed, MPPT5 is much less toxic to normal cells that do not express either MMP2 or uPAR MPPT5 is as toxic as native PA to cells that express MMP2, and is toxic to uPAR expressing cells when pro-uPA and glu-plasminogen are present.

Examples 19 to 24 presented below relate to modified forms of proaerolysin (PA) that contain an ARD and/or a large binding domain mutation. The activation sequence of any one of these modified PA proteins could be modified to provide a MPPT in accordance with the present invention using standard techniques such as those described herein.

### EXAMPLE 19: PRODUCTION OF PROAEROLYSIN WITH AFAI AS A TARGETING UNIT AND AN R336A MUTATION (AFA-PA-R336A)

A modified PA protein comprising an AFAI as a targeting unit and a mutation in a native binding domain (AFA-PA-R336A) was prepared as follows. This protein also included a histidine tag. The AFA-PA-R336A protein was prepared using AFA-PA-his::pTZ18U (or AFA-PA-H6::pTZ18U preparation described in Example 2) as a starting point In order to change amino acid R336 to an alanine (R.336A), two primers were synthesized:
R336A fwd: 5'-CAC CCG GAC AAC GCA CCG AAC TGG AAC-3'(SEQ ID NO:55)
R336A rev: 5'-GTT CCA GTT CGG TGC GTT GTC CGG GTG-3' (SEQ ID NO:56)

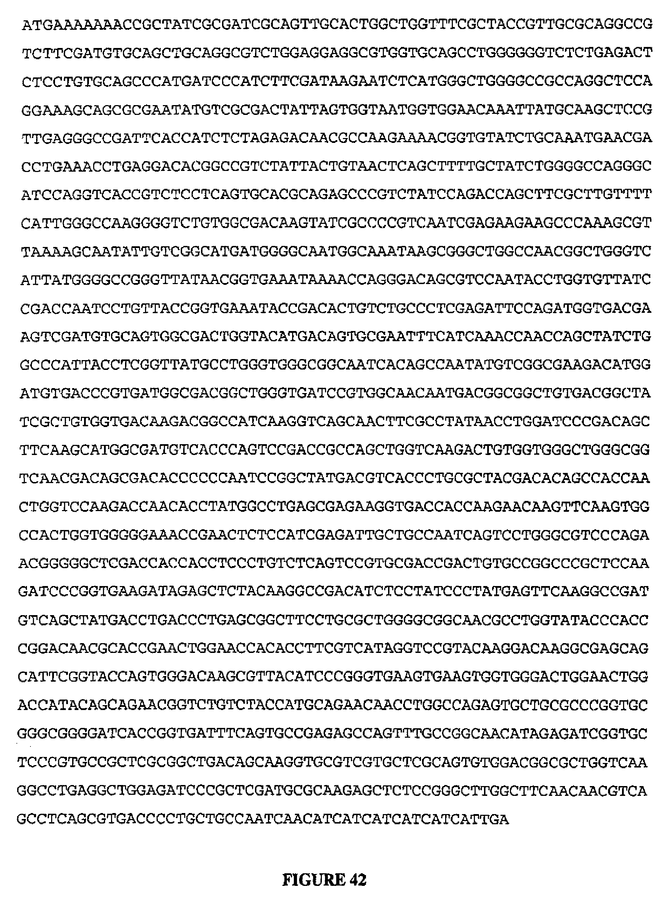

These two primers, along with plasmid AFA-PA-H6::pTZ18U as the template, were used to create the R336A mutation using the Quikchange II Site-Directed Mutagenesis kit (Stratagene) as per the manufacturer's instructions. The resulting clones were sequenced to ensure the correct mutation was made. The resulting plasmid was called AFA-PA(R336A) - H6::pTZ18U and the nucleotide sequence of AFA-PA-R336A (SEQ ID NO:44) is shown in Figure 42.

In order to express AFA-PA(R336A)-H6 proteins, the coding region was cut out of AFA(R336A)-H6::pTZ18U with EcoRI and HindIII, and cloned into the EcoRI and HindIII sites of the wide-host expression vector pMMB67EH. The resulting plasmid AFA-PA(R336A)-H6::pMMB67EH was conjugated into CB3 using the filter mating technique to produce the strain CB3 AFA-PA(R336A)-H6::pMMB67EH

A single colony of CB3 AFA1-PA R336A-His6::pMMB67EH was picked and inoculated into 30 ml of LB Davis media (1% Bacto Tryptone [BBL], 0.5% Yeast Extract [BBL], 1% NaCl) containing 0.2% glucose, 40 µg/ml rifampicin, 40 µg/ml kanamycin and 100 µg/ml ampicillin The culture was grown overnight at 27°C with shaking at 250 rpm. Five 2 L flasks, each containing 500 ml of media as above, were subinoculated (1%) with overnight culture (A₆₀₀ₙₘ 3 70) and incubated at 27°C with shaking at 250 rpm When the A₆₀ₙₘ of the cultures reached 064-072, IPTG was added to a final concentration of 1 mM, to induce AFA-PA R336A-His production. The flasks were incubated at 27°C with shaking at 250 rpm for 17.25 hours When the overnight A₆₀₀ₙₘ of the cultures reached 2.6-3.2, the contents of the flasks were pooled and the culture was centrifuged at 10,000 rpm in a JA-16.25 rotor (Beckman) for 15 minutes at 4°C. The culture supernatant was collected and 6 drops of polypropylene glycol were added to prevent frothing during concentration. The protease inhibitor 1,10-o-phenanthroline was added to a final concentration of 1mM

The culture supernatant was concentrated from 2.4 L to ∼ 50 ml using a Kvick Lab SCU 10,000 Dalton cutoff membrane (Amersham; polyethersulfone membrane with polypropylene screen and a 0 11 m² filtration area) on ice. PMSF was added to a final concentration of I mM.

The concentrate was centrifuged at 10,000 rpm using a JA-25.5 (Beckman) rotor for 10 minutes at 4° C to clarify. At 4°C, the supernatant was pooled (50 ml) and loaded at 1 ml/min onto a 5 ml XK16 Ni²⁺ column (Chelating Sepharose Fast Flow, Amersham) equilibrated in 20 mM Na₂HPO₄, 0.5 M NaCl, 10 mM imidazole, pH 7.4 The column was washed at 2.5 ml/min with 100 ml equilibration buffer (above) to remove any unbound material. The column was eluted for 50 ml with 20 mM Na₂HPO₄, 0.5 M NaCl, 200 mM imidazole, pH 7 4 at 2.5 ml/min. The protein containing fractions were loaded onto a PD10 column (Amersham) equilibrated in 20 mM HEPES, 0.1 M NaCl, 1 mM EDTA, pH 7.4 in 2.5 ml aliquots and eluted with 3.5 ml of the same buffer The PD10 fractions were pooled and loaded at 0.25 ml/min onto a DEAE column (75 ml XK16, CL-6B DEAE, Amersham) equilibrated in the same buffer The column was eluted with a 0.1-0.4 M NaCl linear gradient in 20 mM HEPES, 1 mM EDTA, pH 7.4 at 0 25 ml/min. The protein containing fractions were left in the elution buffer as the final storage buffer. The concentrations were determined by measuring A₂₈₀ₙₘ values

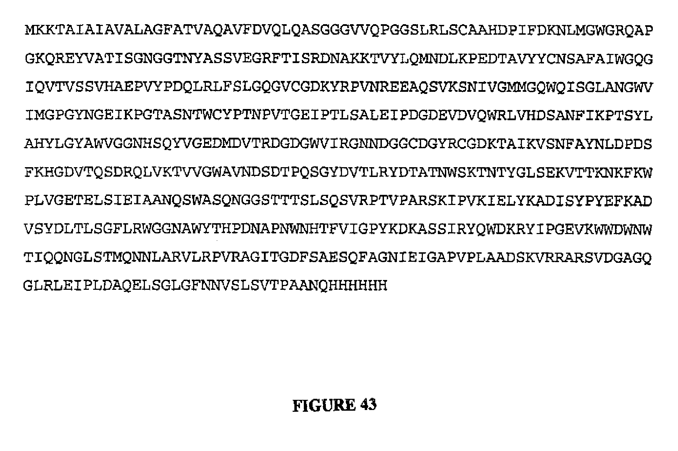

The amino acid sequence of AFA-PA-R336A (SEQ ID NO:45) is shown in Figure 43.

### EXAMPLE 20: PRODUCTION OF PROAEROLYSIN WITH AN R336A MUTATION (PA- R336A)

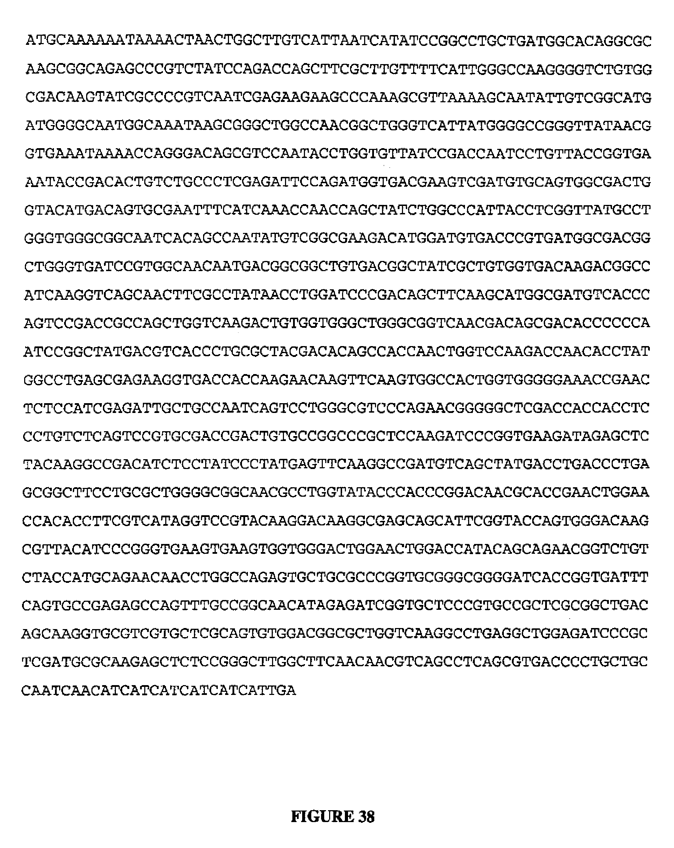

The plasmid AFA-PA(R336A)-H6::pTZ18U, prepared as described in Example 19, was then used to create a proaerolysin construct containing the R336A mutation AFA-PA(R336A)-H6::pTZ18U was digested with XhoI and StuI to give a 1046 nt fragment that contains the portion of *aerA* with the R336A change This was cloned into the XhoI and StuI restriction sites of PA-H6::pTZ18U to give a complete *aerA* gene containing the R336A change The resulting clone was checked by sequencing and the plasmid was called PA(R336A)-H6::pTZ18U. The nucleotide sequence of PA-R336A (SEQ ID NO:42) is shown in Figure 38

In order to express PA-R336A protein, the coding region was cut out of PA-H6::pTZ18U with EcoRI and HindIII, and cloned into the EcoRI and HindIII sites of the wide-host expression vector pMMB66HE The resulting plasmid PA(R336A)-H6::pMMB66HE was conjugated into CB:3 using the filter mating technique to produce the strain CB3 PA(R336A)-H6::pMMB66HE.

A single colony of CB3-PA-R336A-His6::pMMB66 was grown overnight at 27°C with shaking at 250 rpm in 30 ml Phytone Davis media (1% Phytone Peptone [BBL], 0 5% Yeast Extract [BBL], 1% NaCl) containing 0.2% glucose, 100 µg/ml ampicillin, 40 µg/ml kanamycin and 40 µg/ml rifampicin. Five 2 L flasks, each containing Phytone Davis media as above with ampicillin and kanamycin only, were subinoculated (1%) with overnight culture (A₆₀₀ₙₘ 3.75) and incubated at 27°C with shaking at 250 rpm When the A₆₀ₙₘ of the cultures reached 11-12, IPTG was added to a final concentration of 1 mM, to induce toxin production. The cultures were incubated at 27°C with shaking at 250 rpm for 16 hours. When the A₆₀₀ₙₘ of the cultures reached 4.6-5.2, the contents of' the flasks were pooled and the culture was centrifuged at 10,000 rpm using a JA-16.25 (Beckman) rotor for 15 minutes at 4°C. The culture supernatant was saved and 6 drops of polypropylene glycol were added to prevent frothing The protease inhibitor 1,10,-o-phenanthroline was added to a final concentration of 1 mM.

The material was concentrated from 25 L to 45 ml using a Kvick Lab SCU 10,000 Dalton cutoff membrane (Amersham; polyethersulfone membrane with polypropylene screen and a 0.11 m² filtration area) on ice PMSF was added to a final concentration of 1 mM The material was aliquoted and frozen at -20°C for future use

An aliquot of the concentrated material was thawed after 1 day of storage at -20°C and centrifuged at 10,000 rpm using a JA-255 (Beckman) rotor for 10 minutes at 4°C to clarify. The clarified material was loaded onto a 10 ml XK16 FPLC Ni²⁺ (Amersham Chelating Sepharose Fast Flow Resin) column equilibriated in 20 mM Na₂HPO₄, 0.5 M NaCl, 10 mM imidazole, pH 7.4 at 1 ml/min The column was washed for 50 ml at 5 ml/min with equilibration buffer to remove unbound material and eluted at a 59 2% step gradient of 500 mM imidazole in 20 mM Na₂HPO₄, 0.5 M NaCl, 10 mM imidazole pH 7 4 for 40 ml giving a final imidazole concentration of 300 mM. The elution fractions were collected in 4 ml aliquots The peak fractions were pooled and loaded onto a 150 ml XK26 FPLC Sepharose G25 (Sigma) column equilibrated in 20 mM Tris, 1 mM EDTA, 0.15 M NaCl, pH 7.4 at 1 ml/min and eluted with equilibration buffer at 2 ml/min Peak fractions were collected in 4 ml aliquots and pooled yielding 28 ml of material. The desalted material was loaded onto an FPLC HiPrep 16/10 Q FF (Amersham) column equilibrated in desalting buffer at 1 ml/min The column was eluted for 60 ml with a 0.15-1 M NaCl gradient in 20 mM Tris, 1 mM EDIA, pH 74 at 2 ml/min The peak fractions were collected in 4 ml aliquots and the concentrations determined by measuring A₂₈₀ₙₘ values.

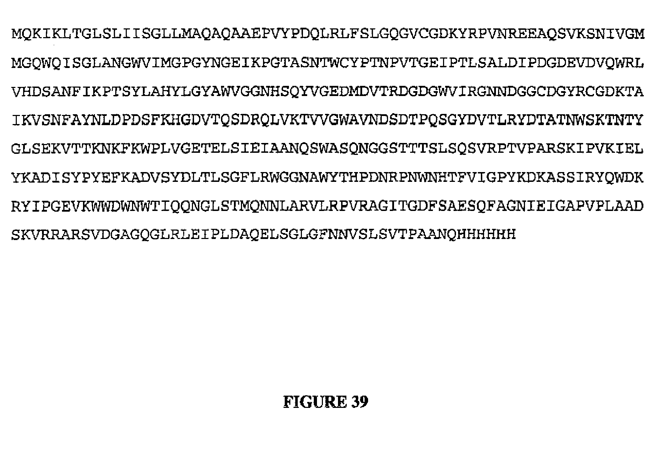

The amino acid sequence of PA-R336A (SEQ ID NO:43) is shown in Figure 39

### EXAMPLE 21: PRODUCTION OF PROAEROLYSIN WITH AN R336C MUTATION (PA- R336C)

The codon for arginine 336 was changed to a cysteine codon using the Quikchange mutagenesis kit (Stratagene) Two primers were synthesized as follows:
Forward 5'-CACCCGGACAACTGTCCGAACTGGAACCACAC-3' (SEQ ID NO:60)
Reverse 5'-GGTTCCAGTTCGGACAGTTGTCCGGGTGGGTAAA-3' (SEQ ID NO:61)

The plasmid PA::pTZ18U was used as the template in the mutagenesis reaction, and the PCR product was digested with EcoRI and HindIII and ligated into the EcoRI and HindIII sites of the vector pMMB66HE This plasmid (PA-R336C::pMMB66HE) was transconjugated into CB3 as described above, and the strain CB3 PA-R336C::pMMB66HE was used to purify the protein PA-R336C

### Purification of PA-R336C

CB3 PA-R336C::pMMB66EH was inoculated into 25 ml of LB media (1% Bacto Tryptone [BBL], 0.5% Yeast Extract [BBL], 1% NaCl) containing 1 x Davis buffer (Miller, 1972; LB-Davis media), 0.2% glucose, 40 µg/ml rifampicin, 40 µg/ml kanamycin and 100 µg/ml ampicillin and grown overnight at 27°C/250 rpm. It was then subinoculated (1%) into the same medium and incubated at 27°C/250 rpm. When the culture reached an OD₆₀₀ₙₘ of approx. 0.7, IPTG was added to a final concentration of 1 mM to induce PA-R336C production and incubation was continued for 17.5 hours. The overnight cultures (OD₆₀₀ₙₘ = 5.8 - 6.0) were centrifuged in a JA-16.25 rotor (Beckman) at 10,000 rpm/15 minutes /4°C. The culture supernatant was collected and concentrated from 2.4 L to - 70 ml using a Sartorius Sartocon Mini-concentrator with a 20,000 Dalton cutoff membrane (cellulose triacetate membrane/0.7 m² filtration area) at 4°C

The concentrate was centrifuged at 10,000 rpm using a JA-25.5 (Beckman) rotor for 10 minutes The supernatant was loaded onto a desalting column (150 ml XK26 Sephadex G25 coarse, Sigma) equilibrated in 20 mM NaH₂PO₄, 0.3 M NaCl, pH 6.0, and eluted with the same buffet at 1 ml/min The protein-containing fractions were identified by measuring A₂₈₀ values of the fractions, and the peak tubes were loaded onto a hydroxyapatite column (40 ml XK16, BioRad) equilibrated in the same buffer. The column was eluted with a 20-150 mM NaH₂PO₄ linear gradient in 0 3 M NaCl, pH 6 0 at 0 25 ml/min Protein containing fractions were pooled and precipitated with 60% ammonium sulfate for 2 hours on ice The precipitated material was pelleted by centrifugation at 15,000 rpm for 10 minutes at 4°C using a JA-25.5 rotor (Beckman) The pellet was resuspended in 15 ml of 20 mM HEPES, 1 mM EDTA, 5 mM β-mercaptoethanol, pH 7 4, and then loaded onto a DEAE column (75 ml XK16 CL-6B DEAE, Amersham) equilibrated in the same buffer, The column was eluted with a 0 1-0 4 M NaCl gradient in 20 mM HEPES, 1 mM EDTA, 5 mM β-mercaptoethanol, pH 7 4, collecting fractions of approximately 5.3 ml.

### EXAMPLE 22: ABILITY OF THE MODIFIED PROAEROLYSIN PA-R336A TO BIND TO MOUSE LYMPHOMA EL4 CELLS

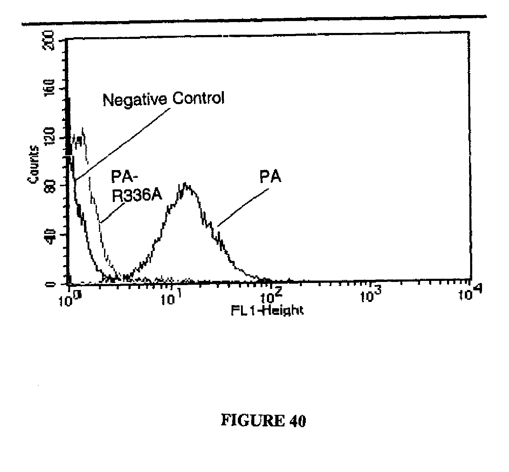

The ability of the modified proaerolysin with a mutation in the large lobe binding domain (PA-R336A) to bind to EL4 mouse lymphoma cells was determined by flow cytometry using a FacsCalibur Flow Cytometer (Pearson). EL4 cells were prepared in unsupplemented cell culture media to a concentration of' 2 x 10⁶ cells/mL by resuspending the cell pellet after centrifugation at 1100 rpm for 5 minutes at room temperature in an IEC Centra CL2 centrifuge PA or PA-R336A was added to cells at 2 x 10⁻⁸ M and incubated on ice for 30 minutes. Cells were centrifuged for 7 seconds in an IEC Microlite microfuge, washed 3x in unsupplemented media and resuspended to the original volume Rabbit anti-aerolysin polyclonal antiserum was added to the cells at a 1/500 dilution and the mixture was incubated on ice for 30 minutes Cells were washed and resuspended as above. The secondary antibody, a polyclonal goat α-rabbit IgG H+L chain specific Fluorscein conjugate (CalBiochem), was added at a 1/5000 dilution and incubated on ice for 30 minutes Cells were washed and resuspended as above A histogram was prepared using CellQuest software and is shown in Figure 40 The results as shown in Figure 40 demonstrates that, as expected, the R336A mutation in the native binding domain of proaerolysin decreased the ability of PAR336A to bind to EL4 cells

### EXAMPLE 23: ABILITY OF THE MODIFIED PROAEROLYSIN PA-R336A TO KILL MOUSE LYMPHOMA EL4 CELLS

The ability of PA-R336A to kill cells was determined as follows. Cell killing assays were performed using EL4 (mouse) cells prepared at a concentration of 1 x 10⁶ cells/mL by resuspending cell pellets in cell culture media (DMEM with 10% FBS) after centrifugation at 1100 rpm for 5 minutes at room temperature in an IEC Centra CL2 centrifuge. Each assay was carried out in triplicate. Using 96 well titer plates, the toxins PA and PA-R336A were serially diluted 1:5 in supplemented cell media using a final volume of' 20 µL After serial dilution, cells were added to appropriate rows to a volume of 100 µL. Two control rows were used: the first containing no toxin and no cells and the second containing no toxin with cells. The cells were then preincubated with the toxins for 1 hour at 37°C, 5% CO₂ with humidity before adding 20 µL of cell killing reagent (Promega CellTiter 96 AQueous One Solution Cell Proliferation Assay) with further incubation for 4 hours under the same conditions After incubation, 7 µL of 70% isopropyl alcohol was added to each well in order to pop air bubbles The plates were read using a BioTec plate reader at 490 nm. Cell killing curves were generated by plotting the calculated average percent cell viability for each well against the respective toxin concentration.

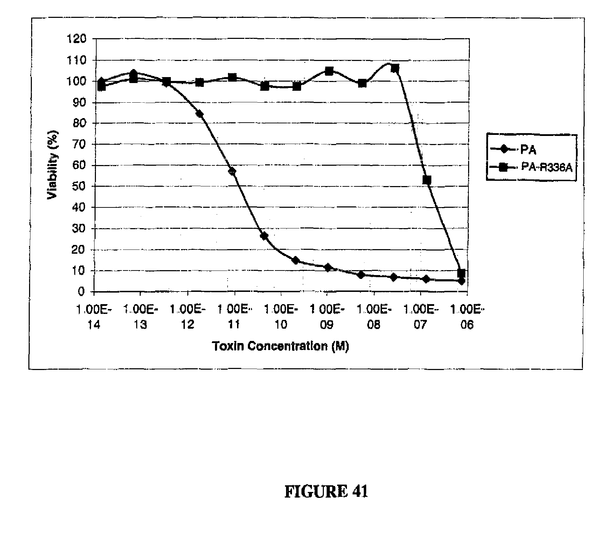

The results of this assay are shown in Figure 41, and indicate that, as expected from the decreased binding ability of PA-R336A shown in Example 22, the ability of this protein to kill cells was decreased compared to that of PA

### EXAMPLE 24: ABILITY OF THE MODIFIED PROAEROLYSINS AFA-PA R336A AND PA-R336C TO KILL MOUSE LYMPHOMA EL4 AND HUMAN LUNG CANCER A549 CELLS

The effect of' the R336A mutation on the ability of AFA-PA-R336A to kill mouse lymphoma EL4 and human lung cancer A549 cells was tested and compared to that of toxins with the R336C mutation (PA-R336C), and to a control toxin containing AFAI as an artificial regulatory domain but no binding domain mutation (AFA-PA) Cell killing assays in both types of cells were carried out in a manner similar to that described in Examples 13 and 21

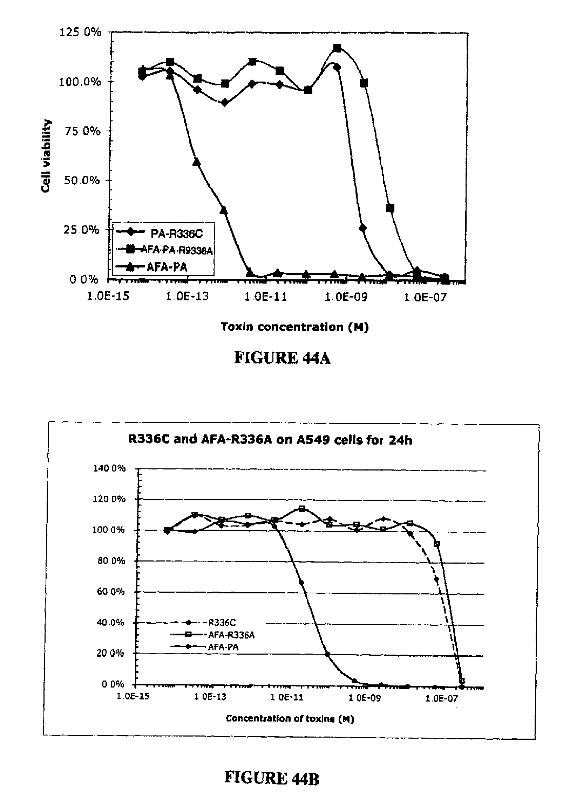

The results are shown in Figure 44A (EL4 cells) and B (A549 cells) and indicate that the toxicity of AFA-PA-R336A and PA-R336C to both types of cells was reduced by the substitution of' arginine in the binding domain. The results also indicated that the AFAI moiety acts as an inhibitor of the modified proaerolysin in that the AFA-PA-R336A protein did not show an improvement in activity over the PA-R336C protein in lung cancer cells, which carry the target for the AFAI moiety This suggests that the addition of the AFAI moiety with a linker that could be cleaved, for example, by an enzyme associated with cancer cells would be a valuable approach for targeting and activating the modified proaerolysin to cancer cells, while decreasing the toxicity of the modified proaerolysin molecule to normal cells.

Although the invention has been described with reference to certain specific embodiments, various modifications thereof will be apparent to those skilled in the art without departing from the spirit and scope of the invention All such modifications as would be apparent to one skilled in the art are intended to be included within the scope of the following claims.

The present invention will now be described by way of reference to the following clauses:
1 A broad-spectrum anti-cancer agent comprising a modified pore forming protein toxin, said modified pore forming protein toxin derived from a naturally occurring aerolysin-related pore forming protein and comprising a modified activation sequence in which a native protease cleavage site has been functionally deleted and replaced with:
   one or more general cleavage sites, each cleavable by an enzyme associated with a plurality of cancers, or
   two or more specific cleavage sites, each cleavable by an enzyme associated
   with the presence of a specific cancer,
   wherein cleavage of said modified activation sequence provides an activated pore forming protein toxin capable of killing cancer cells
2 The broad-spectrum anti-cancer agent according to clause 1, wherein said aerolysin-related pore forming protein toxin is proaerolysin or *Clostridium septicum* alpha toxin
3 The broad-spectrum anti-cancer agent according to clause 1, wherein said aerolysin-related pore forming protein toxin is proaerolysin
4 The broad-spectrum anti-cancer agent according to any one of clauses 1 to 3, wherein said enzyme associated with a plurality of cancers is a protease that is associated with cancer invasion and metastasis, a protease that is up-regulated or secreted by cancer cells, a protease that is activated by enzymes or receptors expressed by cancer cells, or a protease that is associated with angiogenesis.
5 The broad-spectrum anti-cancer agent according to any one of clauses 1 to 4, wherein said enzyme associated with a plurality of cancers is urokinase-type plasminogen activator
6 The broad-spectrum anti-cancer agent according to any one of clauses 1 to 4, wherein said enzyme associated with a plurality of cancers is matrix metalloprotease 2
7 The broad-spectrum anti-cancer agent according to any one of clauses 1 to 6, wherein said modified activation sequence comprises one general cleavage site
8 The broad-spectrum anti-cancer agent according to any one of clauses 1, 2, 3, 4, or 5, wherein said modified activation sequence comprises a general cleavage site that is cleavable by urokinase-type plasminogen activator
9 The broad-spectrum anti-cancer agent according to clause 8, wherein said modified pore forming protein toxin comprises an amino acid sequence substantially identical to the sequence as set forth in SEQ ID NO:21
10 The broad-spectrum anti-cancer agent according to any one of clauses 1, 2, 3, 4, or 6, wherein said modified activation sequence comprises a general cleavage site that is cleavable by matrix metalloprotease 2.
11 The broad-spectrum anti-cancer agent according to clause 10, wherein said modified pore forming protein toxin comprises an amino acid sequence substantially identical to the sequence as set forth in SEQ ID NO:39
12 The broad-spectrum anti-cancer agent according to any one of clauses 1 to 6, wherein said modified activation sequence comprises two general cleavage sites.
13 The broad-spectrum anti-cancer agent according to clause 12, wherein one of said general cleavage sites is cleavable by urokinase-type plasmmogen activator, and the other of said general cleavage sites is cleavable by matrix metalloprotease 2.
14 The broad-spectrum anti-cancer agent according to clause 8 or clause 13, wherein said general cleavage site cleavable by urokinase-type plasminogen activator comprises the amino acid sequence SGRSAQ (SEQ ID NO:51)
15 The broad-spectrum anti-cancer agent according to clause 10 or clause 13, wherein said general cleavage site cleavable by matrix metalloprotease 2 comprises the amino acid sequence HPVGLLAR (SEQ ID NO:52).
16 The broad-spectrum anti-cancer agent according to clause 13, wherein said modified pore forming protein toxin comprises an amino acid sequence substantially identical to the sequence as set forth in SEQ ID NO:41.
17 The broad-spectrum anti-cancer agent according to any one of clauses 1 to 16, wherein said modified pore forming protein toxin further comprises a) an artificial regulatory domain capable of targeting said modified pore forming protein toxin to a cell and/or of inhibiting the activity of said modified pore forming protein toxin, b) one or more mutations in a native binding domain, or c) a combination of a) and b)
18 The broad-spectrum anti-cancer agent according to any one of clauses 1 to 16, wherein said modified pore forming protein toxin further comprises a) an artificial regulatory domain attached to said modified pore forming protein toxin via a linker wherein said artificial regulatory domain is capable of targeting said modified pore forming protein toxin to a cell and/or of inhibiting the activity of said modified pore forming protein, b) one or more mutations in a native binding domain, or c) a combination of a) and b).
19 The broad-spectrum anti-cancer agent according to clause 18, wherein said linker comprises an enzyme cleavage site.
20 The broad-spectrum anti-cancer agent according to clause 19, wherein said enzyme cleavage site is cleavable by urokinase-type plasminogen activator, matrix metalloprotease 2, Factor Xa, enterokinase, or thrombin
21 The broad-spectrum anti-cancer agent according to clause 20, wherein said enzyme cleavage site is cleavable by urokinase-type plasminogen activator
22 The broad-spectrum anti-cancer agent according to clause 20, wherein said enzyme cleavage site is cleavable by thrombin.
23 The broad-spectrum anti-cancer agent according to clause 17, wherein said artificial regulatory domain is capable of targeting said modified pore forming protein toxin to a cell
24 The broad-spectrum anti-cancer agent according to clause 17, wherein said artificial regulatory domain is capable of targeting said modified pore forming protein to a cell and inhibiting the activity of said modified pore forming protein toxin.
25 The broad-spectrum anti-cancer agent according to clause 23, wherein the targeting unit is an antibody, an antibody fragment, a steroid hormone, a peptide hormone, a neuroactive substance, insulin, a growth factor, a cytokine, melanocyte stimulating hormone, a soluble fragment of CD4, a lectin, an adhesion molecule, a selectin, an integrin, or a receptor for an adhesion molecule, or a recognition motif for an adhesion molecule
26 The broad-spectrum anti-cancer agent according to clause 17 or 18, wherein the artificial regulatory domain is an antibody, an antibody fragment, or an enzyme
27 The broad-spectrum anti-cancer agent according to clause 17 or 18, wherein the artificial regulatory domain is an antibody or antibody fragment.
28 The broad-spectrum anti-cancer agent according to clause 17 or 18, wherein the artificial regulatory domain is an AFAI antibody fragment
29 The broad-spectrum anti-cancer agent according to clause 28, wherein said modified pore forming protein toxin comprises an amino acid sequence substantially identical to the sequence as set forth in SEQ ID NO:23.
30 The broad-spectrum anti-cancer agent according to clause 21, wherein said modified pore forming protein toxin comprises an amino acid sequence substantially identical to the sequence as set forth in SEQ ID NO:25
31 The broad-spectrum anti-cancer agent according to any one of clauses 17 to 30, wherein said one or more mutations in a native binding domain are a mutation at position Y61, a mutation at position Y162, a mutation at position W324, a mutation at position R323, a mutation at position R336, a mutation at position W127, or a combination thereof
32 The broad-spectrum anti-cancer agent according to clause 31, wherein at least one mutation is R336A or R336C.
33 An isolated polynucleotide encoding the broad-spectrum anti-cancer agent according to any one of clauses 1 to 32.
34 A vector comprising the polynucleotide according to clause 33, wherein the polynucleotide is operatively linked to one or more expression control sequences
35 A host cell comprising the vector according to clause 34
36 A modified pore forming protein toxin derived from proaerolysin and comprising a modified activation sequence in which a native protease cleavage site has been functionally deleted and replaced with one or more general cleavage sites cleavable by urokinase-type plasminogen activator or matrix metalloprotease 2, said modified pore forming protein toxin comprising an amino acid sequence substantially identical to the sequence as set forth in any one of SEQ ID NOs: 21, 23, 25, 39, or 41.
37 A pharmaceutical composition comprising the broad-spectrum anti-cancer agent according to any one of clauses 1 to 32
38 A pharmaceutical composition comprising the isolated polynucleotide according to clause 33
39 A pharmaceutical composition comprising the modified pore forming protein toxin according to clause 36.
40 A broad-spectrum anti-cancer agent according to any one of clauses 1 to 32, for use in decreasing the size of a tumor in a subject
41 A broad-spectrum anti-cancer agent according to any one of clauses 1 to 32, for use in the treatment of cancer in a subject
42 Use of a modified pore forming protein toxin in the preparation of a medicament for decreasing the size of a tumor in a subject, said modified pore forming protein toxin derived from a naturally occurring aerolysin-related pore forming protein and comprising a modified activation sequence in which a native protease cleavage site has been functionally deleted and replaced with:
   - one or more general cleavage sites, each cleavable by an enzyme associated with a plurality of cancers, or
   - two or more specific cleavage sites each cleavable by an enzyme associated with the presence of a specific cancer,
      wherein cleavage of' said modified activation sequence provides an activated pore forming protein toxin capable of killing cancer cells.
43 Use of a modified pore forming protein toxin in the preparation of a medicament for the treatment of cancer in a subject, said modified pore forming protein toxin derived from a naturally occurring aerolysin-related pore forming protein and comprising a modified activation sequence in which a native protease cleavage site has been functionally deleted and replaced with:
   - one or more general cleavage sites each cleavable by an enzyme associated with a plurality of cancers, or
   - two or more specific cleavage sites each cleavable by an enzyme associated
      with the presence of a specific cancer,
      wherein cleavage of said modified activation sequence provides an activated pore forming protein toxin capable of killing cancer cells
44 The use according to clause 42 or clause 43, wherein said aerolysin-related pore forming protein toxin is proaerolysin or *Clostridium septicum* alpha toxin
45 The use according to clause 42 or 43, wherein said aerolysin-related pore forming protein toxin is proaerolysin.
46 The use according to any one of clauses 42 to 45, wherein said enzyme associated with a plurality of cancers is a protease that is associated with cancer invasion and metastasis, a protease that is up-regulated or secreted by cancer cells, a protease that is activated by enzymes or receptors expressed by cancer cells, or a protease that is associated with angiogenesis
47 The use according to any one of clauses 42 to 46, wherein said enzyme associated with a plurality of cancers is urokinase-type plasminogen activator
48 The use according to any one of clauses 42 to 46, wherein said enzyme associated with a plurality of cancers is matrix metalloprotease 2
49 The use according to any one of clauses 42 to 48, wherein said modified activation sequence comprises one general cleavage site.
50 The use according to any one of clauses 42 to 49, wherein said modified activation sequence comprises a general cleavage site that is cleavable by urokinase-type plasminogen activator
51 The use according to clause 50, wherein said modified pore forming protein toxin comprises an amino acid sequence substantially identical to the sequence as set forth in SEQ ID NO:21
52 The use according to any one of clauses 42, 43, 44, 45, 46, or 48 wherein said modified activation sequence comprises a general cleavage site that is cleavable by matrix metalloprotease 2
53 The use according to clause 52, wherein said modified pore forming protein toxin comprises an amino acid sequence substantially identical to the sequence as set forth in SEQ ID NO:39
54 The use according to any one of clauses 42 to 48, wherein said modified activation sequence comprises two general cleavage sites
55 The use according to clause 54, wherein one of said general cleavage sites is cleavable by urokinase-type plasminogen activator, and the other of said general cleavage sites is cleavable by matrix metalloprotease 2
56 The use according to clause 50 or clause 55, wherein said general cleavage site cleavable by urokinase-type plasminogen activator comprises the amino acid sequence SGRSAQ (SEQ ID NO:51).
57 The use according to clause 52 or clause 55, wherein said general cleavage site cleavable by matrix metalloprotease 2 comprises the amino acid sequence HPVGLLAR (SEQ ID NO:52)
58 The use according to clause 55, wherein said modified pore forming protein toxin comprises an amino acid sequence substantially identical to the sequence as set forth in SEQ ID NO:41.
59 The use according to any one of clauses 42 to 58, wherein said modified pore forming protein toxin further comprises a) an artificial regulatory domain capable of targeting said modified pore forming protein toxin to a cell and/or of inhibiting the activity of said modified pore forming protein toxin, b) one or more mutations in a native binding domain, or c) a combination of a) and b)
60 The use according to any one of clauses 42 to 58, wherein said modified pore forming protein toxin further comprises a) an artificial regulatory domain attached to said modified pore forming protein toxin via a linker, wherein said artificial regulatory domain is capable of targeting said modified pore forming protein toxin to a cell and/or of inhibiting the activity of said modified pore forming protein, b) one or more mutations in a native binding domain, or c) a combination of a) and b)
61 The use according to clause 60, wherein said linker comprises an enzyme cleavage site
62 The use according to clause 61, wherein said enzyme cleavage site is cleavable by urokinase-type plasminogen activator, matrix metalloprotease 2, Factor Xa, enterokinase, or thrombin
63 The use according to clause 62, wherein said enzyme cleavage site is cleavable by urokinase-type plasminogen activator.
64 The use according to clause 62, wherein said enzyme cleavage site is cleavable by thrombin
65 The use according to clause 59, wherein said artificial regulatory domain is capable of targeting said modified pore forming protein toxin to a cell
66 The use according to clause 59, wherein said artificial regulatory domain is capable of targeting said modified pore forming protein to a cell and inhibiting the activity of said modified pore forming protein toxin.
67 The use according to clause 65, wherein the targeting unit is an antibody, an antibody fragment, a steroid hormone, a peptide hormone, a neuroactive substance, insulin, a growth factor, a cytokine, melanocyte stimulating hormone, a soluble fragment of CD4, a lectin, an adhesion molecule, a selectin, an integrin, or a receptor for an adhesion molecule, or recognition motif for an adhesion molecule
68 The use according to clause 59 or 60, wherein the artificial regulatory domain is an antibody, an antibody fragment, or an enzyme.
69 The use according to clause 59 or 60, wherein the artificial regulatory domain is an antibody or antibody fragment.
70 The use according to clause 59 or 60, wherein the artificial regulatory domain is an AFAI antibody fragment
71 The use according to clause 70, wherein said modified pore forming protein toxin comprises an amino acid sequence substantially identical to the sequence as set forth in SEQ ID NO:23.
72 The use according to clause 63, wherein said modified pore forming protein toxin comprises an amino acid sequence substantially identical to the sequence as set forth in SEQ NO:25.
73 The use according to any one of clauses 59 to '72, wherein said one or more mutations in a native binding domain are a mutation at position Y61, a mutation at position Y162, a mutation at position W324, a mutation at position R32.3, a mutation at position R336, a mutation at position W127, or a combination thereof.
74 The use according to clause 73, wherein at least one mutation is R336A or R336C.
75 A method of decreasing the size of a tumor comprising administering to a subject having cancer an effective amount of the broad-spectrum anti-cancer agent according to any one of clauses 1 to 32.
76 A method of treating cancer comprising administering to a subject having cancer an effective amount of the broad-spectrum anti-cancer agent according to any one of clauses 1 to 32
77 A method of preparing a broad-spectrum anti-cancer agent, said method comprising:
   - providing a native pore forming protein toxin wherein said native pore forming protein toxin is proaerolysin or *Clostridium septicum* alpha toxin; and
   - modifying the activation sequence of said native pore forming protein toxin such that a native protease cleavage site is functionally deleted and replaced by one or more general cleavage sites each cleavable by an enzyme associated
   with a plurality of cancers, or by two or more specific cleavage sites each cleavable by an enzyme associated with the presence of a specific cancer, wherein cleavage of said modified activation sequence provides an activated pore forming protein toxin capable of killing cancer cells

## Claims

1. An anti-cancer agent comprising a modified pore forming protein toxin, said modified pore forming toxin comprising a naturally occurring aerolysin-related pore forming protein toxin comprising a modified activation sequence in which a native protease cleavage site has been functionally deleted and replaced with one or more enzyme cleavage sites, each cleavable by a matrix metalloproteinase, caspase, elastase, cathepsin, or plasminogen activator and further comprising an artificial regulatory domain selected from the group consisting of a growth factor, an antibody, and an antibody fragment, wherein cleavage of said modified activation sequence provides an activated pore forming protein toxin capable of killing cancer cells.

2. The anti-cancer agent of claim 1, wherein said artificial regulatory domain is attached to said pore forming protein toxin via a linker.

3. The anti-cancer agent of claim 2, wherein said linker comprises an enzyme cleavage site, preferably wherein said enzyme cleavage site is cleavable by urokinase-type plasminogen activator, matrix metalloprotease 2, Factor Xa, enterokinase, or thrombin.

4. The anti-cancer agent of any one of claims 1 to 3, wherein said growth factor is epidermal growth factor, insulin-like growth factor, fibroblast growth factor, platelet derived growth factor, or tumor necrosis factor.

5. The anti-cancer agent of any one of claims 1 to 3, wherein said antibody or antibody fragment selectively binds a cancer cell, preferably wherein said antibody or antibody fragment recognizes carcinoembryonic antigen, CA-15-3, thyroid transcription factor 1, cytokeratin 7, p97 melanotransferrin antigen, L6 antigen, CD20, CD52, Her2/neu receptor, epidermal growth factor receptor, or vascular endothelial growth factor.

6. The anti-cancer agent of any one of claims 1 to 5, wherein said aerolysin-related pore forming protein toxin is proaerolysin, preproaerolysin, or *Clostridium septicum* alpha toxin.

7. The anti-cancer agent of any one of claims 1 to 6, further comprising one or more mutations in a native binding domain, wherein the mutation results in functional deletion of the large lobe binding domain.

8. The anti-cancer agent of claim 7, wherein the one or more mutations are selected from a mutation at position Y61 of SEQ ID NO: 2, a mutation at position Y162 of SEQ ID NO: 2, a mutation at position W324 of SEQ ID NO: 2, a mutation at position R323 of SEQ ID NO: 2, a mutation at position R336 of SEQ ID NO: 2, a mutation at position W127 of SEQ ID NO: 2, or a combination of two or more thereof, preferably wherein at least one mutation is R336A of SEQ ID NO: 2 or R336C of SEQ ID NO: 2.

9. An isolated polynucleotide encoding the anti-cancer agent of any one of claims 1 to 8.

10. A vector comprising the polynucleotide of claim 9, wherein the polynucleotide is operatively linked to one or more expression control sequences.

11. A host cell comprising the vector of claim 10.

12. A pharmaceutical composition comprising the anti-cancer agent of any one of claims 1 to 8 or the isolated polynucleotide of claim 9.

13. The anti-cancer agent of any one of claims 1 to 8 or the composition of claim 12, for use in the treatment of cancer or for use in decreasing the size of a tumor in a subject.

14. The anti-cancer agent or composition of claim 13, wherein the use further comprises administering to the subject a therapeutically effective amount of one or more anti-cancer therapeutics.
